# NEW EUROPEAN PATENT SPECIFICATION

(11) **EP 0 353 188 B2**
(45) Date of publication and mention of the opposition decision: **06.03.2002**
(45) Mention of the grant of the patent: 16.03.1994
(21) Application number: 89810545.7
(22) Date of filing: 19.07.1989
(51) Int. Cl.: C12N 15/60, C12N 15/80, C12N 15/88

(54) **Novel expression system**
Expressionssystem
Système d'expression

(30) Priority: 28.07.1988 GB 8818046; 26.06.1989 GB 8914666
(43) Date of publication of application: 31.01.1990
(73) Proprietor: Novartis AG, 4056 Basel (CH)
(72) Inventor: Heim, Jutta, Dr., CH-4433 Ramlinsburg (CH); Meyhack, Bernd, Dr., CH-4312 Magden (CH); Visser, Jacob, Prof., NL-6703 CK Wageningen (NL)

(56) References cited:
- EP-A- 0 278 355
- WO-A-86/06097
- GB-A- 88 180 468
- GB-A- 89 146 666
- MEDEDELINGEN LANDBOUWHOGESCHOOL WAGENINGEN, vol. 75, no. 13, 1975, pp.1-96, Wageningen, NL; F. Van Houdenhoven, p. 9, 18, 19
- PATENT ABSTRACTS OF JAPAN, vol. 12. no. 105 (C-539)(3252), 26 Oct. 1988
- BIOTECHNOLOGY, vol. 5, no. 7, 1987, pp. 713-719, New York, US; D.I. Gwynne et al.
- Collmer et al., Palacios and Verma, Molecular Genetics of Plant Microbe Interactions, 1988, pp. 356-361
- Maniatis et al., Molecular Cloning, A Laboratory Manual, Cold Spring Harbor Laboratory, 1982
- Edmann and Begg, A Protein Sequenator, Eur.J.Biochem 1, 80-90
- Kusters-Van Someren, Characterization of an Aspergillus Niger Pectin Lyase Gene Family; PhD thesis, State University Utrecht, the Netherlands, 17 January 1991

## Description

### Field of the invention

The invention relates to the field of genetic engineering and provides new DNA molecules comprising DNA sequences coding for several pectin lyases of Aspergillus niger and/or the promoter, signal and terminator sequences thereof. The novel DNA molecules are useful for the overproduction of the pectin lyases in Aspergillus and/or the construction of hybrid vectors expressing foreign genes in filamentous and other fungi. It is now possible to produce a single pectin lyase which is uncontaminated with other pectin lyases.

### Background of the invention

Although in genetic engineering techniques numerous polypeptide expression systems for prokaryotic and eukaryotic hosts are already known, there is a continuous need for novel systems which may have advantages over the known systems.

Very widely used are the prokaryotic Escherichia coli host and the eukaryotic yeast host, e.g. Saccharomyces cerevisiae, for which a high number of different expression hybrid vectors, mostly plasmids, have been developed. The drawbacks of E. coli hosts are that they cannot glycosylate the formed polypeptide and that for lack of secretion the foreign peptide may accumulate within the host cell and prevent further growth. The yeast hosts do glycosylate, however, like E. coli, they do not secrete the polypeptides, except very small ones, into the nutrient medium. Yeasts secrete only into the periplasmic space. Higher eukaryotic hosts are mammalian cancer cells which are able to glycosylate and secrete into the nutrient medium, however, cultivation thereof is very slow and expensive and the danger exists that oncogenic nucleic acids are isolated together with the desired peptide of which the latter may not be freed.

In the need for other hosts also filamentous fungi, such as Neurospora crassa, Aspergillus nidulans and Aspergillus niger, have been investigated. Such fungi are already widely used for industrial purposes, however, the application thereof in genetic engineering techniques has lagged behind, mainly for lack of an appropriate transformation system. In contrast to Saccharomyces cerevisiae, filamentous fungi do not contain plasmids which could be used for the introduction of foreign genes and phenotype selection. It is, however, possible to transform filamentous fungi with foreign plasmids containing a selectable marker gene. All vectors described so far for filamentous fungi do not autonomously replicate, as those of yeasts do, but are integrated into the fungal chromosome. This event occurs only at a very low frequency. Advantageously, on the other hand, integrative transformation renders the transformants mitotically very stable, even under non-selective conditions. Stable integration of more than one hundred copies has been reported.

The first vector for filamentous fungi described contained the qa-2 gene of Neurospora crassa as selectable marker. This gene encodes the enzyme catabolic dehydroquinase and can be used for functional complementation of aro mutants of N. crassa [Case, M.E., Schweizer, M., Kushner, S.R. and Giles, N.H. (1979) Proc. Natl. Acad. Sci. USA 76, 5259-5263]. Aro mutants are unable to grow on minimal medium without an aromatic amino acid supplement. Transformation of N. crassa by the qa-2 vector occurred by integration of a single copy of the plasmid into the chromosome. 30 % of stable Aro⁺ integrants retained the integrated qa-2 gene still linked to the bacterial plasmid sequences [Case, M.E. (1982) in Genetic Engineering of Microorganisms for Chemicals (Hollander, A., DeMoss, D., Kaplan, S., Konisky, J., Savage, D. and Wolfe, R.S., eds), pp. 87-100, Plenum]. This observation made cotransformation of non-selective DNA-sequences together with selective ones a feasible task.

In Aspergillus nidulans, which has a sexual cycle and is therefore amenable to classical genetic manipulations, both negative and positive selection systems have been identified. Either using heterologous DNA from N. crassa or homologous DNA, functional complementation of A. nidulans pyrG-mutants by transformation with plasmids containing the pyrG gene was obtained (Ballance et al. BBRC 112, 284 1983; Tilburn et al. Gene 26, 205, 1983). In other systems mutations at the trpC or argB locus were functionally complemented by transformation with the appropriate plasmids [Yelton et al. PNAS 81, 1470, 1984; Melton et Timberlake J. Cell. Biochem. Suppl. 9C 173, 1985; Johnstone et al. EMBO J. 4, 1307, 1983].

A dominant positive selection system has also been developed making use of the amdS gene isolated from A. nidulans which enables A. niger transformed therewith to grow on acetamide as sole nitrogen source (Tilburn et al., Gene 26, 205, 1983; Wernars et al., Curr.Genet. 9, 361, 1985; Kelly, J.M. et al., EMBO J. 4, 475, 1985).

Compared to N. crassa or A. nidulans, A. niger is by far the more important organism. It is used widely in the industrial production of enzymes, e.g. for use in the food industry. A. niger differs from A. nidulans by its secretory capacity, in that it secretes a variety of hydrolytic enzymes, e.g. glucoamylase, α-amylase, pectinase, cellulase, β-glucanase, β-galactosidase, naringinase, pentosanase, acid protease and lignase, the glucoamylase and pectinase complex being the most important ones.

A. niger has no known sexual cycle. Mutations can therefore not be introduced via meiotic recombinations. By classical mutation and selection procedures, extensive strain improvements in the secretion of hydrolytic enzymes have however been achieved.

Of the genes of A. niger enzymes only those of glucoamylase (Boel et al. EMBO J. 3, 1581, 1984) and alcohol and aldehyde dehydrogenase (WO 86/06097) together with their promoter and signal sequences have been characterised and used in transformation experiments with A. nidulans and A. niger, respectively.

As selection markers for A. niger have been used the heterologous amds gene (Kelly and Hynes, EMBO 3. 4, 475, 1985), and the argB gene (Buxton et al., Gene 37, 207, 1985; EP 184 438; WO 86/06097), both obtained from A. nidulans.

A. niger is the most important organism for the industrial production of pectin degrading enzymes. Pectins are polygalacturonides of high molecular weight (20000-40000 D) consisting of α-1,4-glycosidic bounded D-galacturonic acid polymers and occur in nature as constituents of higher plant cells, where they are attached to cellulose molecules and where they are mainly found in the primary cell wall and the middle lamella. Amongst the richest sources of pectin are lemon and orange rind, which contain about 30 % of this polysaccharide. Pectic enzymes are degrading the carbohydrate polymer substrate either by hydrolysis of the α-1,4-glycosidic bond (polygalacturonase) or by transelemination of the α-4,5 unsaturated galacturonic residue from the pectin molecule (different pectin lyases). The systematic name of pectin lyase is pectin transeliminase (EC 4.2.2.10).

In A. niger the proteins of the pectic complex are not expressed constitutively. Under inducing conditions using pectin or breakdown products thereof A. niger expresses the above mentioned enzymes, including PLI, when other carbon sources, such as glucose or sucrose, are limiting. In surface cultures the pectic enzymes tend to remain associated with the outer cell wall. Increasing pectin and Ca²⁺ concentration in the medium leads to complete secretion.

Pectinases, such as PLI, are used by the food stuff industry mainly for fruit juice clarification.

From A. niger two different pectin lyases, PLI and PLII, have been purified and partially characterized by F.E.A. Van Houdenhoven (22). PLI contains four residues of mannose, whereas PLII has two residues of mannose and glucose each. The enzymes have different molecular weights (PLI: 37.5 kD, PLII: 36 kD). The amino acid sequence of PLI has been determined and is disclosed in EP 88 101 397.3. This invention was based on a partial structure determination of pectin lyase I (PLI) which allowed the synthesis of DNA probes coding for relevant parts of the protein. By means of the DNA probes it was possible to screen for and isolate DNA coding for PLI, eventually together with pre- and post-sequences thereof, from a gene library of A. niger.

By hybridization of parts of the PLI gene to a genomic library of A. niger further PL genes have been detected which are subject of the present invention. The PLI structural gene with the N-terminus flanking region obtained from A. niger N756 is at its N-terminus identical to the PLD gene obtained from A. niger N400. Accordingly the latter is not part of the present invention. The present PLA seems to be part of the previously purified PL mixture named PLII.

Hereinafter, PLI is also named PLD and the PLI structural gene is named pelD.

### Objects of the invention

Objects of the invention are recombinant DNA molecules comprising DNA sequences coding for novel pectin lyase expression systems and derivatives thereof, such as the structural genes of said pectin lyases and corresponding regulatory sequences, e.g. promoter, signal and terminator sequences, and hybrid vectors comprising corresponding DNAs, including hybrid vectors with DNA coding for homologous or heterologous polypeptides, hosts, especially filamentous fungi, e.g. Aspergillus hosts, transformed by said vectors, methods for the preparation of said recombinant DNA molecules and said hosts and the use of the recombinant DNA molecules for the preparation of new expression systems. A further objective is the preparation of polypeptides by means of said DNAs and said hosts.

The novel pectin lyase expression systems comprise DNA sequences coding for the promoters, the signal sequences, the structural genes and the terminators of said novel pectin lyase genes. The novel pectin lyases are named PLA, PLB, PLC, PLE and PLF.

More particularly, one object of the present invention is the construction of hybrid vectors comprising DNA sequences coding for the promoters of PLA, PLB, PLC, PLE and PLF, optionally for the signal sequence of these proteins, and optionally for the terminators thereof. These hybrid vectors are used for the incorporation of genes coding for particular proteins and for the transformation of filamentous fungi, such as Aspergillus, Penicillium and Cephalosporium. Cotransformation of A. niger with 2 different plasmids can be carried out, whereby one of the plasmids is selected from the group of the novel hybrid vectors of the invention, and the other one is a specially constructed selection plasmid which works in connection with a mutated strain of a filamentous fungus.

The present invention also concerns the overproduction of said novel pectin lyases in Aspergillus species, and the production of the single PLs which are uncontaminated with the other PLs, or of predetermined artificial mixtures thereof.

The present invention further concerns the production of any protein the structural gene of which can be expressed in the presence or under the control of the present recombinant PL DNAs.

The various subjects of the invention will become more evident from the following detailed description of the invention.

### Detailed description of the invention

### The Recombinant DNA Molecules

More particularly, the present invention concerns a recombinant DNA molecule comprising a DNA sequence coding for the expression systems of the pectin lyases PLA, PLB, PLC, PLE or PLF, or a derivative thereof.

The term "expression system" means a DNA sequence capable of expressing a polypeptide and comprises the promoter, the signal sequence, the structural gene and the terminator.

The term "derivative" when used in connection with the novel DNA sequences is intended to include larger derivatives with flanking sequences, fragments of said DNA sequence, mutants, especially naturally occurring mutants, and DNA sequences which are degenerated in accordance with the genetic code.

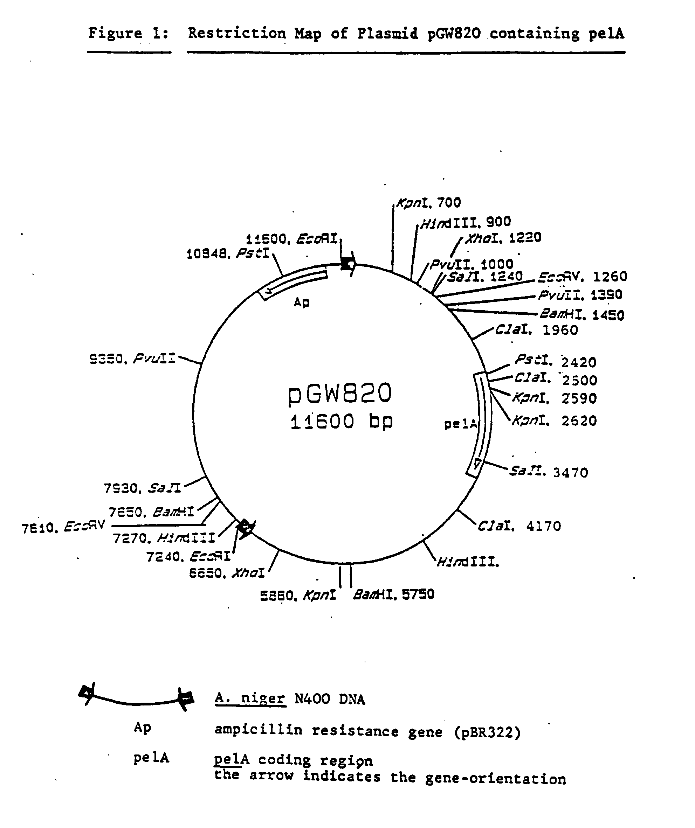

Larger derivatives of the novel recombinant DNA molecules are those excisable from the A. niger genome and comprising the DNA sequences provided in Figures 1 to 3, 5 and 6, such as can be found in a genomic library of A. niger N400 obtained by fragmentation of the nucleic acids, treatment of the fragments with a suitable restriction enzyme, e.g. EcoRI, BamHI or HindIII, ligating into a suitable vector, e.g. the lambda phage and the plasmid pBR322, cloning, e.g. in E. coli, and excising again, with the same restriction enzyme. Such derivatives are e.g. the inserts of plasmids pGW820, pGW830, pGW840, pGW850, pGW860 and pGW880 shown in Figures 1, 2, 3, 4, 5 and 6.

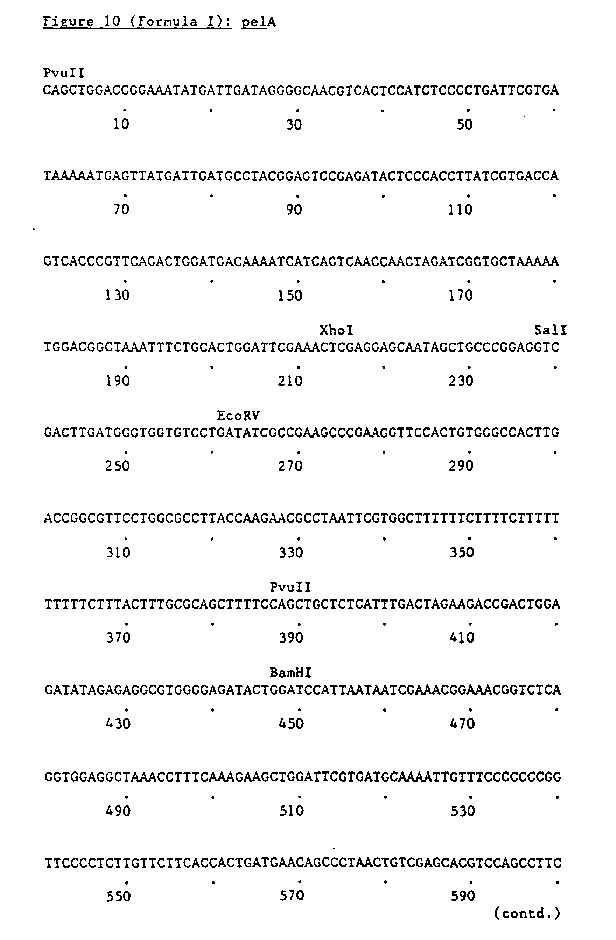

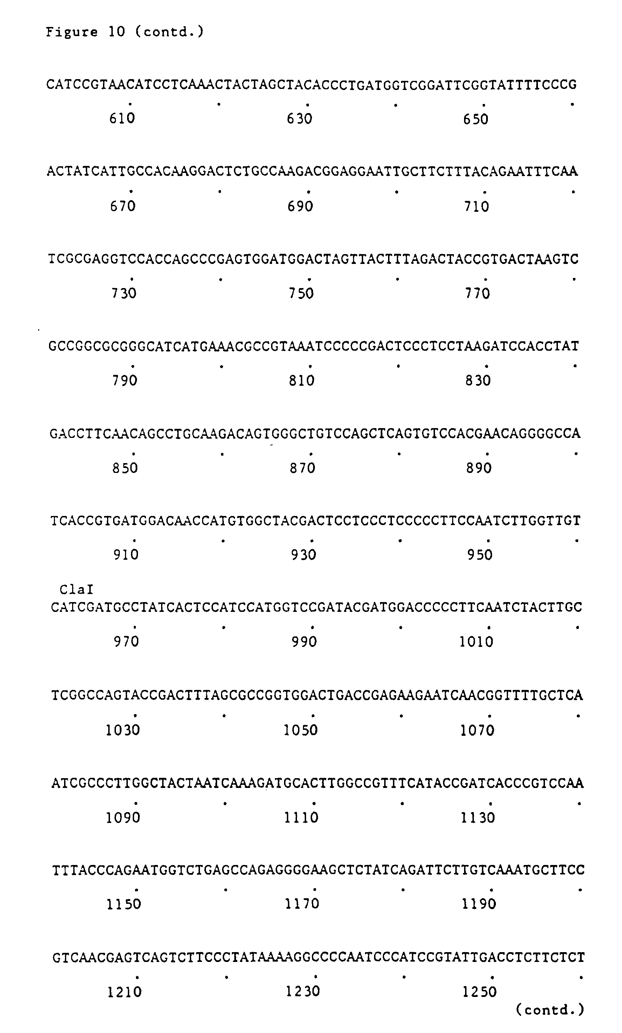

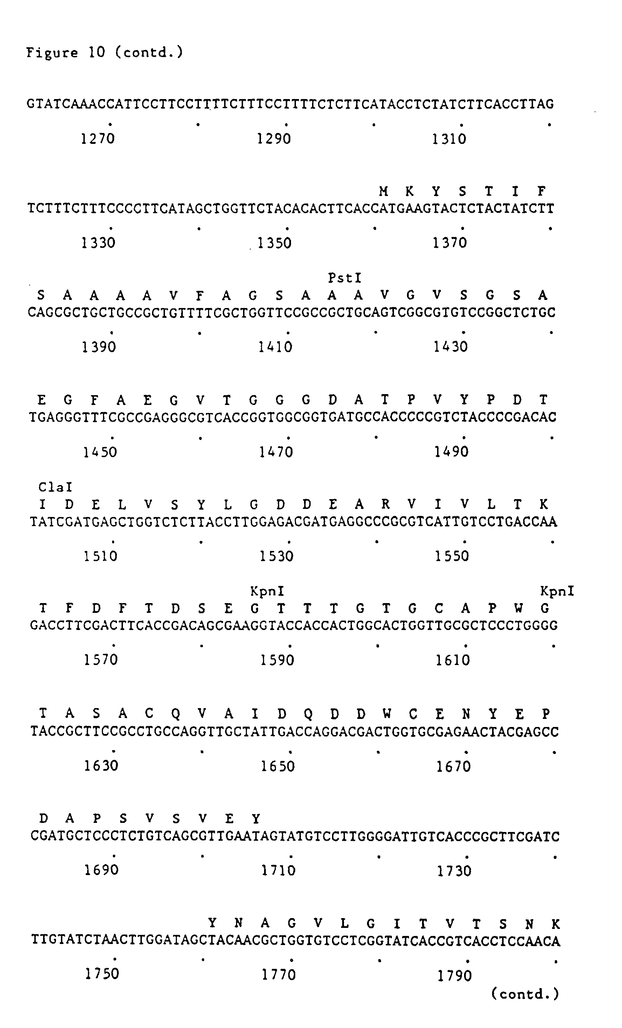

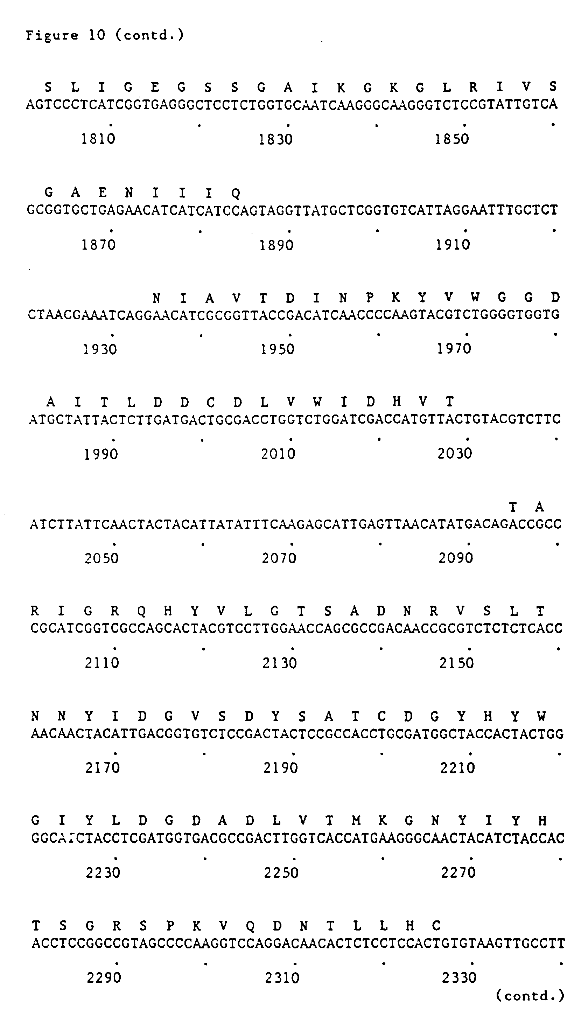

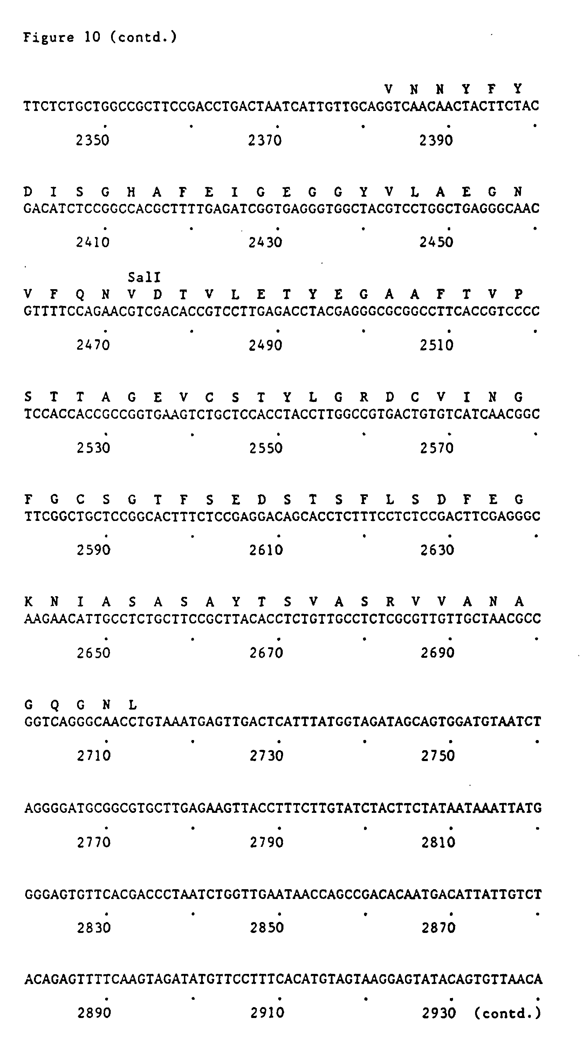

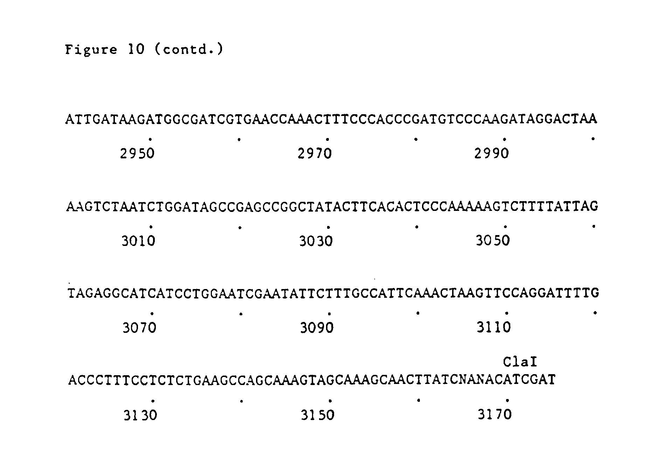

Fragments of the DNA sequence of the Formula I (Fig. 10) are those extending between two restriction sites of these plasmids and retaining promoter, signal, structural or terminator functions.

Preferred fragments are those containing the promoter sequence, the signal sequence, the structural gene of a novel PL, or the terminator sequence, or any combination thereof.

The fragments of the DNA sequence may contain linkers which provide for successful linkage to other DNA molecules.

The PL promoter sequence is contained in the DNA region before the structural gene and comprises up to about 2000, preferably up to about 1000 to 1400 nucleotides. In pGW820 the promoter is located on the sequence between the Sall (1240) and the Pstl (2420) restriction site. For the promoter sequence the TATAA boxes are important as the RNA-polymerase recognition sites. On pelA the TATAA box is located at position 1221 (Fig.10), on pelB at position 951 (Fig.11) and on pelC at position 1261 (Fig.12). The short promoters from around the TATAA boxes up to the start of the signal sequences are of particular interest for the non-induced expression of polypeptides. If pectin induced expression is required the sequences upstream of the TATAA boxes are necessary for regulating the strength of the promoter. Suitable linkers may be attached to these fragments.

The promoter of PLI of A. niger is inducible, i.e., the expression of the structural gene attached thereto, e.g. the structural gene coding for a PL or any foreign gene, is induced by addition of pectin or pectin degradation products to the medium. In the absence of pectin and presence of sufficient glucose the promoter is not operating. If the upstream regulatory sequences are absent the promoter becomes constitutive.

The DNA coding for the signal sequence extends between the end of the promoter and the beginning of the sequence coding for the mature protein. In PLA and PLB the signal sequence contains about 20 amino acids, in pelC about 18 amino acids. The corresponding coding region extends in pelA from the ATG codon at position 1361 down to the Pstl cleaving site at position 1420, in pelB from position 1134 to at least position 1191, and in pelC from position 1368 to position 1422.

As is evident from formulae I, II and III the structural genes of PLA, PLB and PLC contain several introns. Also the structural genes may contain suitable linkers.

The terminators start with the stop codons, e.g. TAA, and may extend up to one of the restriction sites within said sequences. The terminator fragment contains at least 300 bp and may contain suitable linkers.

Suitable linkers to above fragments have a DNA sequence which fits into the restriction site of the DNA to which the fragment is to be linked. They may contain a predetermined restriction site.

Fragments of the DNA sequence of the invention are also such which are composed of smaller fragments, e.g. those containing the promoter, the promoter and the signal or structural sequence, the signal and the structural sequence, or the structural gene without the introns, and the like.

Mutants of the DNA sequence of the invention are e.g. naturally occurring mutants. The invention comprises also natural or synthetic mutants of the signal sequence with a similar or identical hydrophobicity profile, e.g. wherein the codons for the polar amino acids lysine (K^{δ+}), tyrosine (Y^{δ-}) and arginine (R^{δ+}) are exchanged by codons for other amino acids having similar charges, and the hydrophobic amino acids alanine (A), leucine (L) and threonine (T), are replaced by codons for other hydrophobic amino acids. For example, the codon for the positively charged lysine may be replaced by a codon for arginine and vice versa, the codon for the negatively charged tyrosine by a codon for glutamate or aspartate, and/or the codon for the non-polar, hydrophobic alanine by any one of the codons for threonine, proline, valine, isoleucine, leucine, methionine or phenylalanine, and the like. Other mutations are "silent mutations" wherein one or a few other nucleotides, e.g. up to about 30, are replaced by one or more other nucleotides, whereby the new codons code for the same amino acid(s).

DNA sequences of the invention which are degenerated are, like silent mutations, such which are degenerated within the meaning of the genetic code in that an unlimited number of nucleotides are replaced by other nucleotides without changing the amino acid sequence for which they code. Such degenerate DNA sequences may be useful because of their different restriction sites.

Recombinant DNA molecules comprising a DNA sequence of the invention or a derivative thereof are especially recombinant vectors, alternatively called hybrid vectors, containing such DNA sequences as inserts. They are usable for cloning in hosts, such as bacteria, fungi or animal cells. Such hybrid vectors are derived from any vector useful in the art of genetic engineering, such as from phages, cosmids, plasmids or chromosomal DNA, such as derivatives of phage λ, e.g. NM 989, or of phage M13, e.g. M13mp8 phage DNA (ref. 15) linearized by BamHI digestion, bacterial plasmids, e.g. pBR 322, pUN121, pUC18, or yeast plasmids, e.g. yeast 2µ plasmid, or also chromosomal DNA, derived e.g. from Aspergillus, e.g. A. niger, for example those provided by EP 184 438, or defective phages or defective plasmids in the presence of a helper phage or a helper plasmid allowing replication of said defective phages or plasmids, e.g. M13(+)KS vector in presence of e.g. M13K07 helper phage.

A hybrid vector of the invention contains, in addition to the DNA sequences of the invention or a derivative thereof, a replication site and optionally, depending on the type of the DNA derivative, an expression control sequence, such as an enhancer sequence, upstream activation site, a promoter and signal sequence, and/or a structural gene different from the corresponding present A. niger derived sequences. Such enhancer sequence may be derived from the extrachromosomal ribosomal DNA of Physarum polycephalum (PCT/EP 8500278), or it may be the upstream activation site from the acid phosphatase PHO5 gene (EP Appl. No. 86 111 820.6), or the PHO5, trp, PHO5-GAPDH hybrid (EP Appl. No. 86 111 820.6), or the like promoter.

Structural genes ligated to the present promoter, signal and/or terminator sequences are, besides those coding for pectin lyases PLA, PLB, PLC, PLE and PLF with or without introns, also homologous other pectin lyase genes, e.g. the PLD (or PLI) gene, or other Aspergillus genes and heterologous structural genes which originate from viruses, procaryotic cells or eucaryotic cells and which may be derived from genomic DNA or from cDNA prepared via the mRNA route or may be synthesized chemically, coding for a wide variety of useful polypeptides, including glycosylated polypeptides, in particular of higher eukaryotic, especially mammalian, such as animal or especially human origin, such as enzymes which can be used, for example, for the production of nutrients and for performing enzymatic reactions in chemistry, or polypeptides, which are useful and valuable for the treatment of human and animal diseases or for the prevention thereof, for example hormones, polypeptides with immunomodulatory, anti-viral and anti-tumor properties, antibodies, viral antigens, vaccines, clotting factors, foodstuffs and the like.

Examples of such heterologous structural genes are e.g. those coding for hormones such as secretin, thymosin, relaxin, calcitonin, luteinizing hormone, parathyroid hormone, adrenocorticotropin, melanocytestimulating hormone, β-lipotropin, urogastrone or insulin, growth factors, such as epidermal growth factor, insulin-like growth factor (IGF), e.g. IGF-I and IGF-II, mast cell growth factor, nerve growth factor, glia derived nerve cell growth factor, or transforming growth factor (TGF), such as TGFβ, growth hormones, such as human or bovine growth hormones, interleukin, such as interleukin-1 or -2, human macrophage migration inhibitory factor (MIF), interferons, such as human α-interferon, for example interferon-αA, αB, αD or αF, β-interferon, γ-interferon or a hybrid interferon, for example an αA-αD- or an αB-αD-hybrid interferon, especially the hybrid interferon BDBB, proteinase inhibitors such as α ₁-antitrypsin, SLPI and the like, hepatitis virus antigens, such as hepatitis B virus surface or core antigen or hepatitis A virus antigen, or hepatitis nonA-nonB antigen, plasminogen activators, such as tissue plasminogen activator or urokinase, tumour necrosis factor, somatostatin, renin, β-endorphin, immunoglobulins, such as the light and/or heavy chains of immunoglobulin D, E or G, or human-mouse hybrid immunoglobulins, immunoglobulin binding factors, such as immunoglobulin E binding factor, calcitonin, human calcitonin-related peptide, blood clotting factors, such as factor IX or VIIIc, erythropoietin, eglin, such as eglin C, hirudin, desulfatohirudin, such as desulfatohirudin variant HV1, HV2 or PA, human superoxide dismutase, viral thymidin kinase, β-lactamase, glucose isomerase. Preferred genes are those coding for a human α-interferon or hybrid interferon, human tissue plasminogen activator (t-PA), hepatitis B virus surface antigen (HBVsAg), insulin-like growth factor I and II, eglin C and desulfatohirudin, e.g. variant HV1. In the hybrid vectors of the present invention, the present promoter and/or signal sequence is operably linked to the polypeptide coding region so as to ensure effective expression of the polypeptide.

The DNA molecules of the present invention may contain selective markers depending on the host which is to be transformed, selected and cloned. Any marker gene can be used which facilitates the selection of transformants due to the phenotypic expression of the marker. Suitable markers are particularly those expressing antibiotic resistance, e.g. against tetracycline or ampicillin, or, in the case of auxotrophic yeast mutants, genes which complement host lesions. Corresponding genes confer, for example, resistance to the antibiotic cycloheximide, or provide for prototrophy in an auxotrophic yeast mutant, for example the ura3, leu2, his3 or trp1 gene. It is also possible to employ as markers structural genes which are associated with an autonomously replicating segment providing that the host to be transformed is auxotrophic for the product expressed by the marker.

Of particular importance are marker genes which complement A. niger host lesions, such as the argB gene coding for the ornithine carbamoyl transferase, e.g. derived from A. niger or A. nidulans (EP 184 438), or A. nidulans DNA fragments homologous to the N. crassa pyr4 gene (26).

Preferred embodiments of the present invention are hybrid vectors wherein the structural gene, especially the heterologous structural gene is operatively linked to the promoter and signal sequences of the present invention. Such preferred hybrid vector is e.g. pUC19/pelA-IFN AM119. Another such preferred hybrid vector is e.g. M13(+)KS/pelA-IFN AM119.

In another preferred embodiment of the present invention the structural gene, especially the heterologous structural gene is operatively linked directly to the promoter sequences of the present invention. Such preferred hybrid vector is e.g. M13(+)KS/pelAΔss-IFN AM119.

The DNA molecules of the invention and the derivatives thereof, including fragments can be used for screening DNA gene banks or mRNA for further similar DNAs or mRNAs.

### Process for the Preparation of the Recombinant DNA Molecules

The invention concerns also a process for the preparation of a recombinant DNA molecule coding for a pectin lyase PLA, PLB, PLC, PLE or PLF expression system or a derivative thereof, comprising culturing a host transformed with a DNA molecule containing a DNA sequence coding for the pectin lyase expression system or a derivative thereof and isolating the desired recombinant DNA molecule or the derivative thereof, or preparing it by an in vitro synthesis.

The culturing of the hosts ist carried out in a conventional nutrient medium which may be supplemented with or deprived of chemical compounds allowing negative or positive selection of the transformants, i.e. such hosts containing the desired DNA molecule together with a selection marker, from the nontransformants, i.e. such hosts lacking the desired DNA molecule.

Any transformable hosts useful in the art may be used, e.g. bacteria, such as E. coli, fungi, such as Saccharomyces cerevisiae, or in particular filamentous fungi, such as Aspergillus, e.g. A. nidulans, A. oryzae, A. carbonarius, A. awamori and especially A. niger. A preferred host is A. niger An8, a novel mutant lacking the pyrA gene, as described further below. Transformation of the hosts is carried out by conventional methods.

The DNA sequence coding for the PL expression system is obtained from a filamentous fungi containing such system, in particular from a genomic library thereof or also via the mRNA.

In the following the preparation of the present PL expression systems is described in more detail.

A genomic library can be prepared e.g. by partial digestion of genomic DNA of an A. niger strain, e.g. N756 or N400, with e.g. Sau3Al or Mbol, and cloning the high molecular weight DNA fragments in a suitable host vector, e.g. the E. coli plasmid pUN121 or a lambda vector, e.g. EMBL4.

Any other A. niger strain producing the desired PLs may serve as source for the genomic library and likewise other suitable vectors may be used as recipient for the fragments.

In order to successfully screen the genomic library for DNA sequences coding for PLs a hybridizing DNA probe is necessary. This can be a synthetic DNA probe if the sequence of the desired PL is known or it can be of a known PL gene or parts thereof. The first approach was used to find the PLI gene as described in EP 88 101 397.3. The second approach was used in the present invention. Since the total DNA sequence of the PLI gene became available either DNA probes containing the entire gene or any part thereof having at least about 14 bp can now be used for screening, which includes also the screening for mRNA coding for the PL system.

For screening purposes the DNA probes are 5' radioactively labelled by methods known in the art using γ³²P-ATP and T4 kinase. Host microorganisms carrying nucleic acids of the present invention as an insert, are identified by hybridisation with the labelled DNA probe on filter replicas of the gene library.

Clones showing a hybridisation response to one or more DNA probes are isolated and amplified.

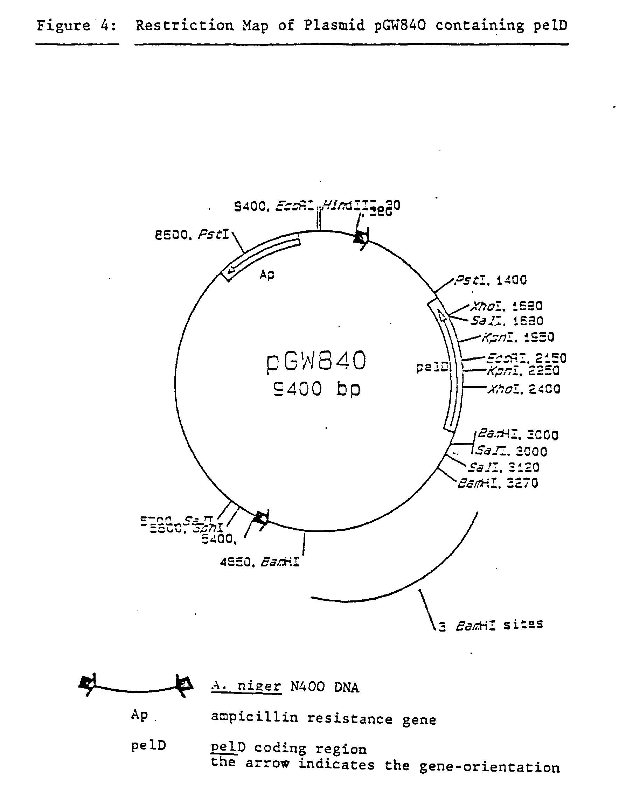

The hybridization conditions used can be more or less stringent, e.g. simply by choosing different temperatures, and in combination with the use of different DNA probes derived from the PLI (or PLD) gene, e.g. by measuring the various hybridization responses to the complete 1.6 kbp BamHl/Pstl fragment, the 649 bp BamHI/XhoI fragment (the N-terminus fragment) and the 244 bp XhoI/PstI fragment (the C-terminus fragment) from pCG3B11 or pGW840 (Fig.4), the clones can be divided into different classes based on their degree of homology (Tables II or IV). Five λ-vectors (λ-PL113, λ-PL122, λ-PL109, λ-PL102 and λ-PL116) were finally identified, restricted and subclones of their pel genes prepared in pBR322 leading to plasmids pGW820, pGW830, pGW850, pGW860 and pGW880 (Figs. 1 to 3, 5 and 6). The five genes of these clones are called pelA, pelB, pelC, pelE and pelF respectively.

The genes can be sequenced. Full sequences of pelA, pelB and pelC are represented by Formulae I, II and III (Figs. 10, 11 and 12).

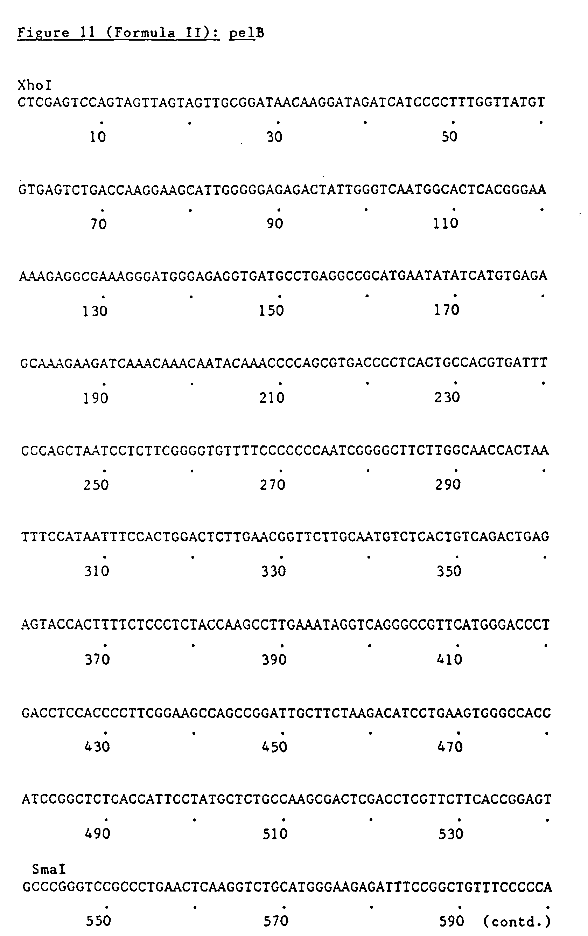

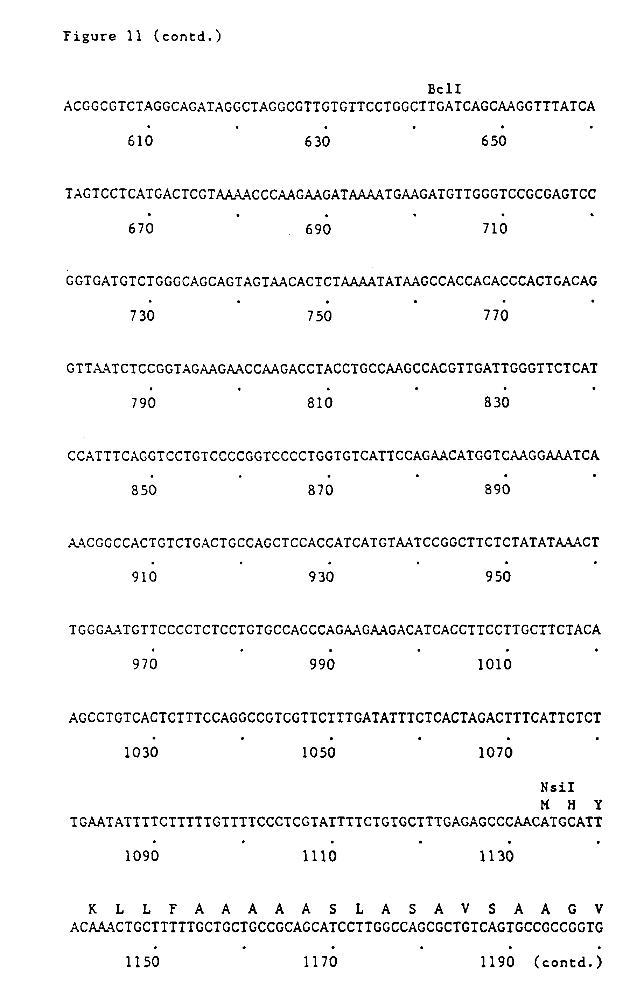

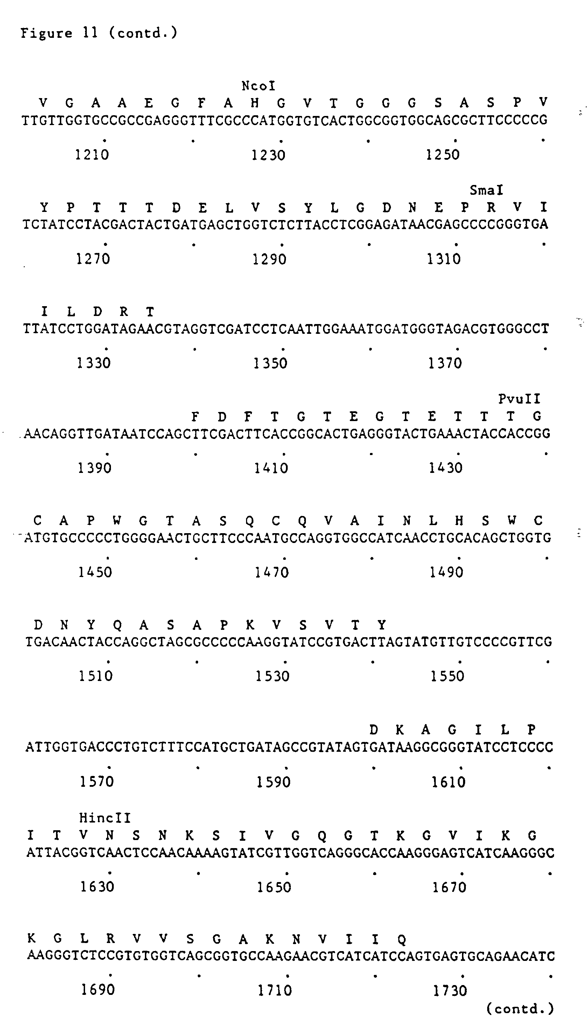

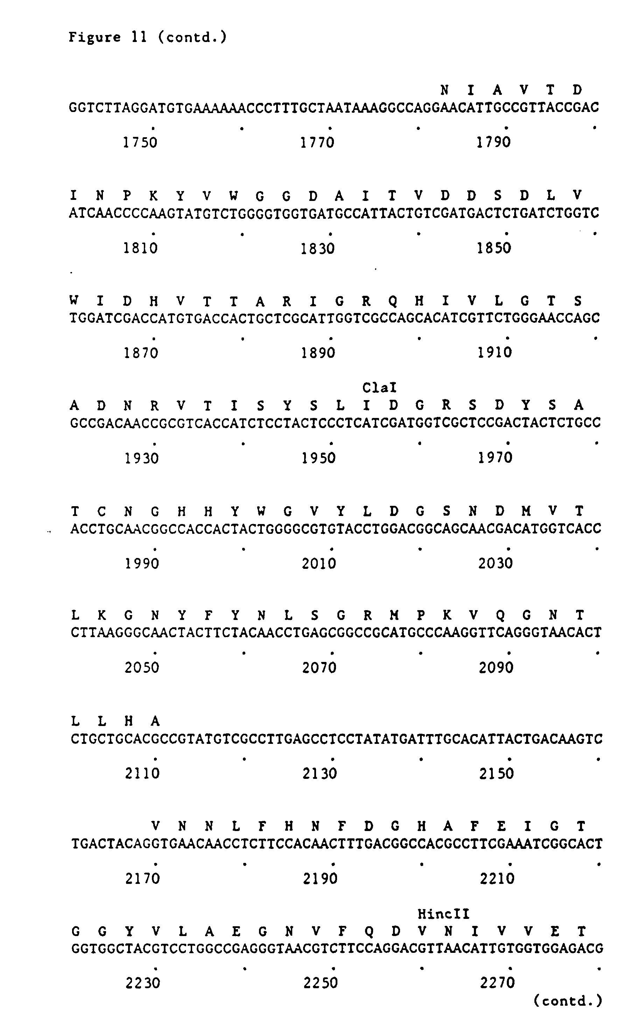

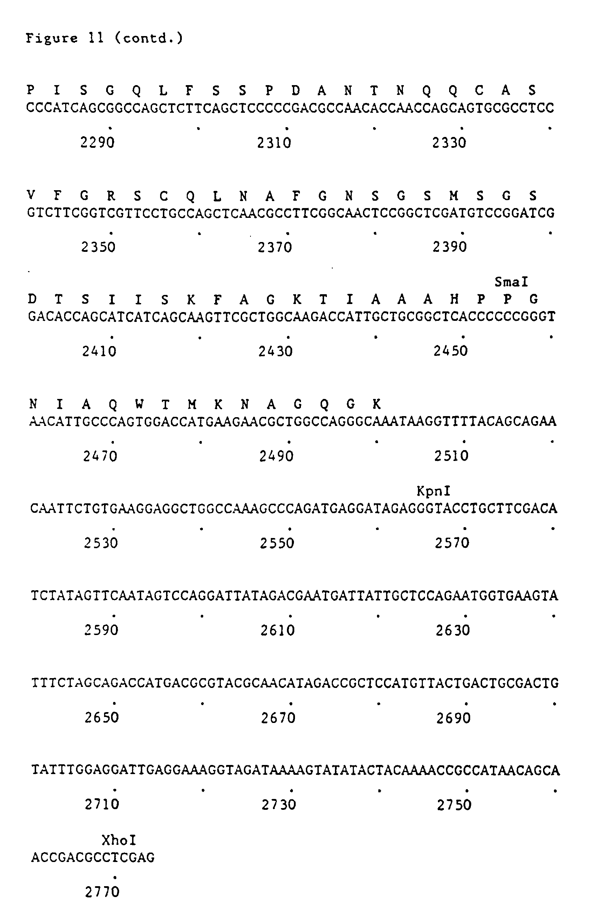

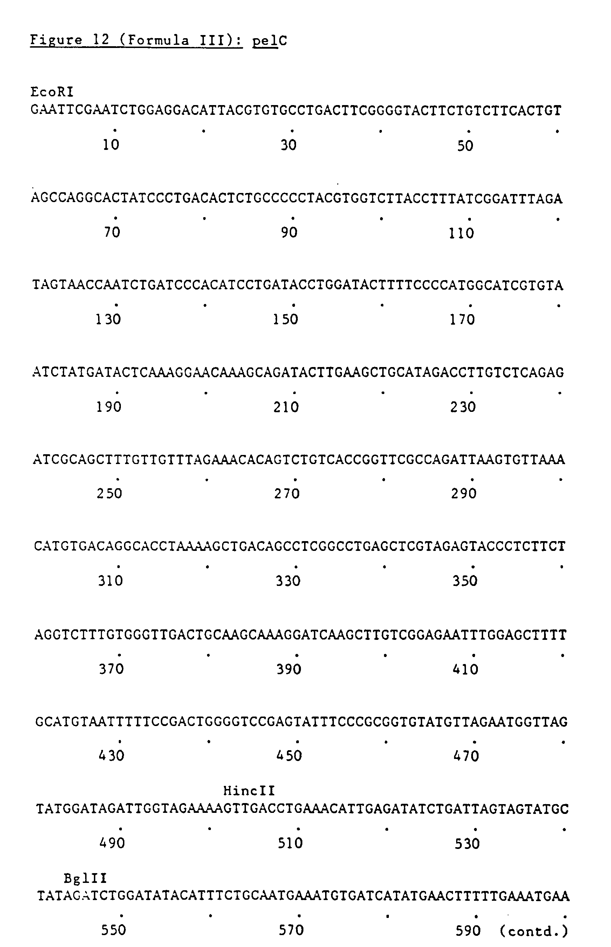

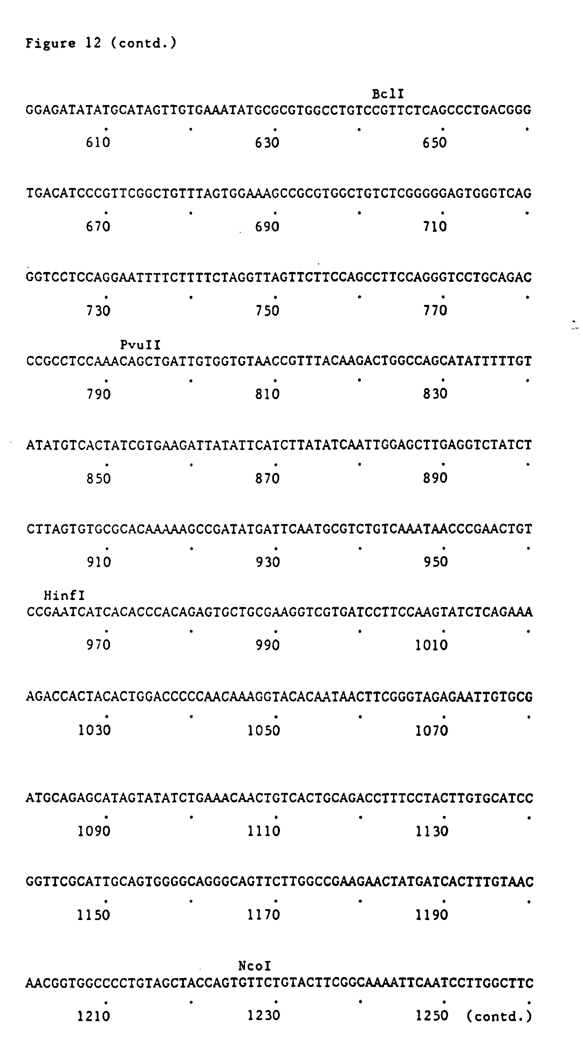

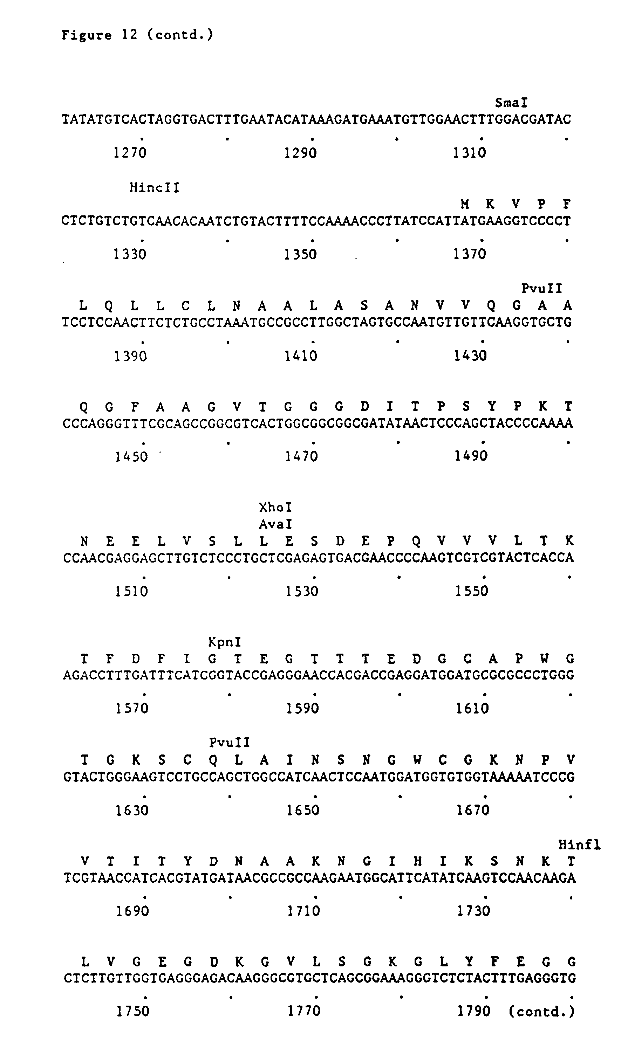

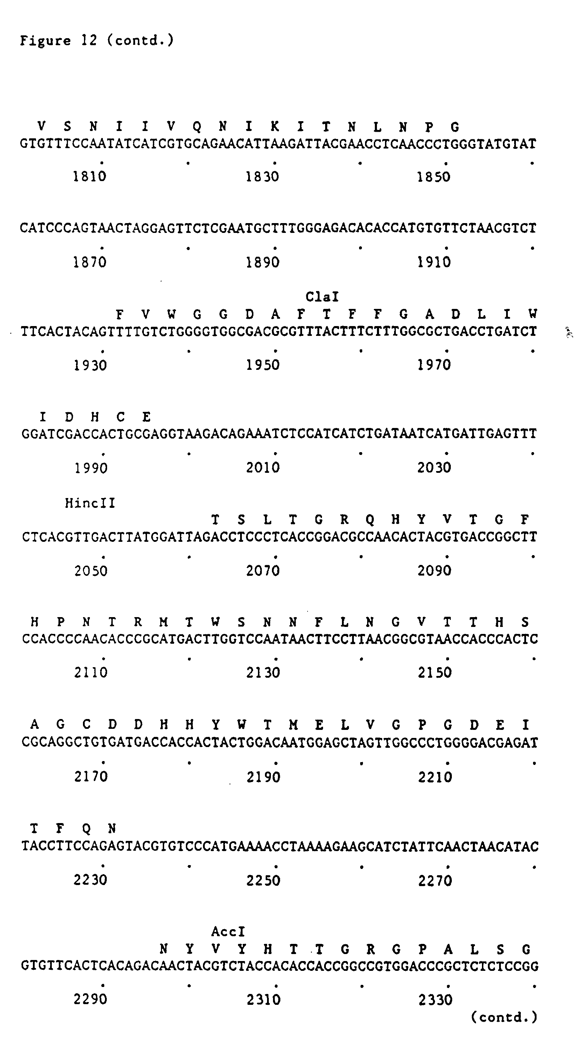

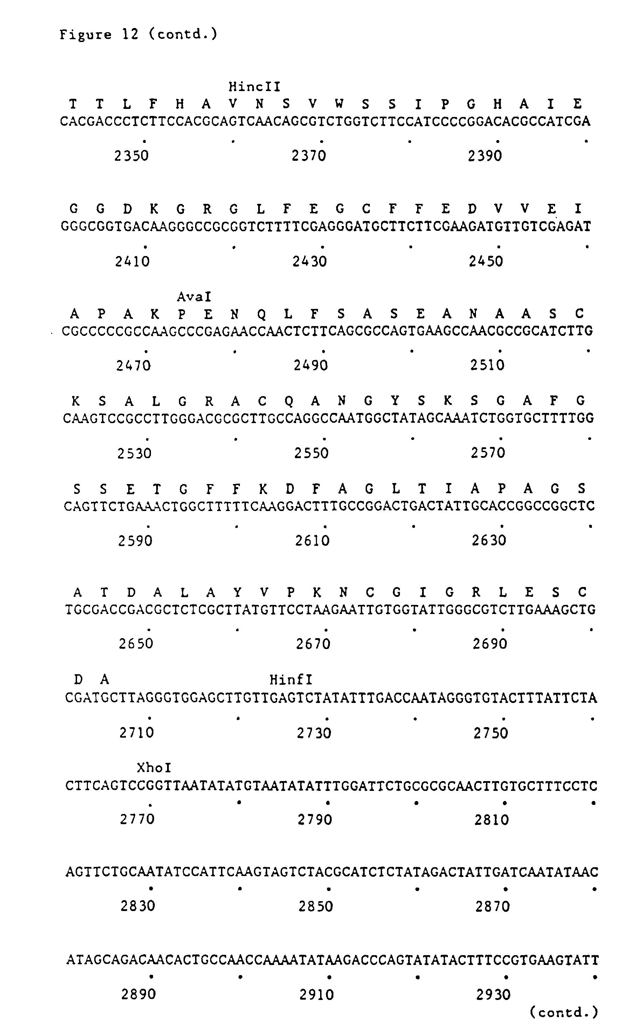

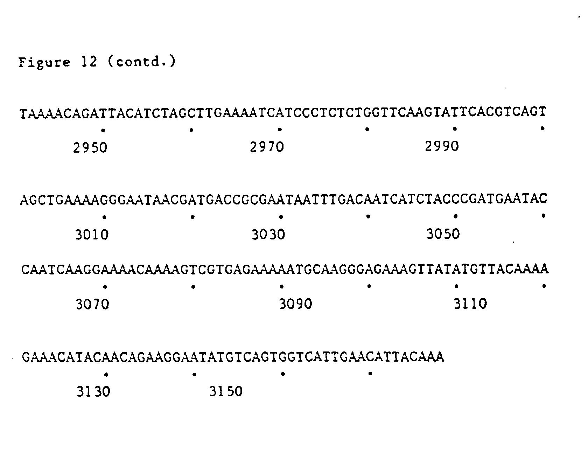

A computer aided search for consensus sequences of exon/intron splice junctions as well as for intron internal sequences (Boel E. et al. (24) and Mount S.M. (25)) leads up to postulate, like in pelD, the presence of four introns in pelA and pelB (Fig. 10 and 11), and of three introus in pelC (Fig. 12).

The plasmids pGW820, pGW830, pGW850, pGW860 and pGW880 are used to prepare other recombinant DNA molecules of the invention. Such other DNA molecules are prepared in conventional manner by applying conventional restriction enzymes, linkers, ligation, amplification and isolation processes.

For example, the plasmid pGW820 is restricted with HindIII. The 3.9 kbp HindIII fragment is subcloned into the HindIII site of pBR322. The 3.3 kbp BamHI-HindIII fragment of the obtained plasmid pGW822 containing the pelA gene is cloned into the BamHI and HindIII site of the vector pUC19 to give a vector called pUC19/pelA. The structural gene of pelA is excised by digestion with Sall and Pstl. Into the remaining fragment containing the pelA promoter, signal and terminator sequences is ligated between the signal and terminator sequence a gene coding for the interferon hybrid BDBB by means of a suitable linker. The obtained plasmid is named pUC19/pelA-IFN AM119 (Fig.11) and contains the promoter and signal sequence of pelA the IFN BDBB gene and the pelA terminator attached to the HindIII-BamHI fragment of pUC19. This plasmid is cotransformed with pCG59D7 into the uridine auxotrophic A. niger mutant An8 (DSM 3917). Transformants are selected in minimal medium containing arginine and Bacto-Agar and analyzed for interferon expression.

In an other embodiment of the invention the signal sequence of pelA may be deleted. The obtained plasmid is named M13(+)KS/pelAΔss-IFN AM119 and contains the promoter sequence of pelA, the IFN BDBB gene and the pelA terminator attached to the HindIII-BamHI fragment of Bluescript M13(+)KS vector. This plasmid is cotransformed with pCG59D7 into the uridine auxotrophic A. niger mutant An8 (DSM 3917). Transformants are selected in minimal medium containing arginine and Bacto-Agar and analyzed for interferon expression.

Mutants containing new restriction sites can be prepared, for example in vitro by site-directed mutagenesis, according to conventional methods [see review article of M.J. Zoller and M. Smith, Methods Enzymol. 100, 468 (1983), D. Botstein and D. Shortle, Science 229, 1193 (1985) or K. Norris et al., Nucl. Acids Res. 11, 5103 (1983)].

For higher expression rates, any eukaryotic terminator sequence can be ligated to the end of the structural gene.

Bacteria are transformed by a conventional method and the transformants identified by their resistance, e.g. against tetracycline.

In particular the described expression vectors are amplified in suitable E. coli host strains, such as HB101, transformed and selected by methods conventional in the art. The amplified plasmid DNA is isolated from the bacteria by conventional methods, in particular as described by Birnboim & Doly (23).

In a similar manner other plasmids with other homologous or heterologous genes can be constructed.

The DNA molecules of the present invention can also be prepared by an in vitro synthesis according to conventional methods. The in vitro synthesis is especially applicable for the preparation of smaller fragments of the PL expression system, e.g. of the DNA sequences coding for the promoter or the signal sequence of the PLs, or mutants thereof.

DNA molecules of the invention comprising a PL promoter and optionally a PL signal sequence, a PL structural gene, any other heterologous structural gene and/or a PL terminator can also be used to transform filamentous fungi, such as Aspergillus, Penicillium or Cephalosporium, e.g. A. nidulans, A. oryzae, A. carbonarius, A. awamori and especially A. niger.

In order to allow selection of the transformed from the non-transformed fungi, the DNA molecules of the invention carry a selection marker or, alternatively, the fungi are cotransformed with a second vector containing such marker. As in other systems such selection marker is an expressible, structural gene, the expressed polypeptide of which (an enzyme) provides resistance against compounds toxic to the transformant or which completes the enzyme system of a mutant lacking such essential polypeptide. Such marker genes are for example the known ga-2, pyrG, pyr4, trpC, amdS or argB genes.

As described in EP 88 101 397.3 a novel marker gene, named pyrA, was isolated from the genomic library of A. niger, which is related to and has similar function as pyrG of A. nidulans and pyr4 of N. crassa, namely producing the enzyme orotidine 5'-phosphate decarboxylase. This enzyme catalyses the decarboxylation of orotidine 5'-phosphate to uridylic acid (uridine 5'-phosphate) and also of fluoro-orotic acid to the toxic fluoro-uridine. An E. coli clone containing the pyrA gene was identified by hybridization with the 1.1 kb Hind III fragment of pDJB2 (24) containing part of the pyr4 gene, however, DNA of any other pyr gene coding for orotidine-5'-phosphate decarboxylase may be used. From a positive clone named E. coli B75183/pCG59D7, the plasmid pCG59D7, comprising the pyrA gene, was isolated and used for cotransformation of - an A. niger pyrA⁻ mutant. Such pyrA⁻ mutant is defective in the orotidine 5'-phosphate decarboxylase gene and therefore is unable to produce the corresponding enzyme. Such mutant was prepared by treating conidiospores of A. niger N756 under mutating UV-irradiation and colonies surviving in the presence of fluoro-orotic acid and uridine are selected. Colonies surviving in the presence of fluoroorotic acid and absence of uridine are eliminated. The remaining uridine-requiring mutants, according to their ability of being transformable, belong to two complementation groups pyrA and pyrB, represented by mutants An8 and An10, respectively. They are treated in the form of protoplasts thereof under transforming condition with the pyrA containing plasmid pCG59D7. Only the An8 colonies were found to be transformed and to contain the pyrA gene as evidenced by the hybridizing ability of digested DNA thereof with DNA of pUN 121.

The invention concerns further hosts transformed with the hybrid vectors of the invention and methods for their preparation. Such transformants are for example bacteria, such as E. coli, or filamentous fungi, such as Aspergillus, Penicillium or Cephalosporium, and in particular A. nidulans, A oryzae, A. carbonarius, A. awamori or preferably A. niger, e.g. A. niger An8. The invention concerns also a method for the preparation of such transformants comprising treatment of a host under transforming conditions with a recombinant DNA molecule, especially a hybrid vector, of the invention, optionally together with a selection marker gene and selecting the transformants.

The invention concerns also the use of the recombinant DNAs or a derivative thereof for the preparation of hybrid vectors which express useful homologous or heterologous polypeptides. Examples of genes encoding such useful polypeptides are given hereinbefore.

The invention concerns further a method for the preparation of polypeptides, characterized in that a hybrid vector of the invention is expressed in a suitable host. When required, the polypeptide is isolated in conventional manner. Depending on the construction of the vector the products are either expressed, or, if a signal sequence is present, are expressed and secreted.

This method comprises the production of useful homologous or heterologous proteins in a suitable host, e.g. Aspergillus species, by cultivating a host transformed with an expression hybrid vector. Examples of genes encoding such proteins are given hereinbefore.

It is now also possible to produce the single polypeptides PLA, PLB, PLC, PLD, PLE or PLF, that means in pure form and uncontaminated by any other PL, whereby various methods can be applied. E.g., one method for the production of a single polypeptide selected from the group consisting of PLA, PLB, PLC, PLD, PLE and PLF is characterized in that a host which is not capable of expressing any pectin lyase PL is transformed with a DNA expression vector expressing the product of PLA, PLB, PLC, PLD, PLE or PLF.

A host not capable of expressing any pectin lyase PL is either a microorganism having no corresponding gene, e.g. a PL⁻ Aspergillus strain, another non-Aspergillus fungi or any other eukaryotic or prokaryotic microorganism, or an Aspergillus strain whose production of PLs is supressed in an appropriately conditioned growth medium. For example, a single PL gene can be expressed under the control of the glucoamylase promoter in A. niger or A. awamori, under the control of the PH05 promoter in S. cerevisiae or under the control of a PL promoter in a PL⁻ A. niger strain.

### Short Description of the Figures

- Figure 1: shows the restriction map of pGW820 containing the pelA gene

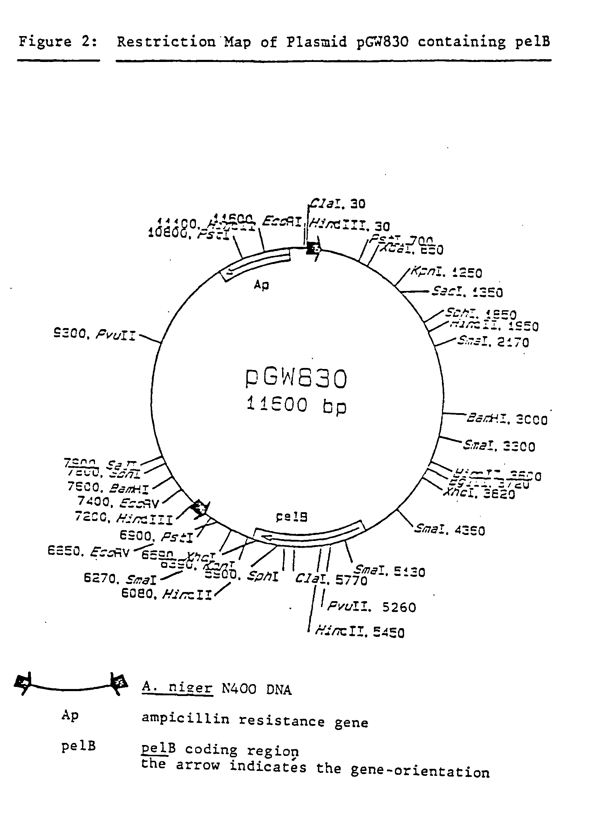

- Figure 2: shows the restriction map of pGW830 containing the pelB gene

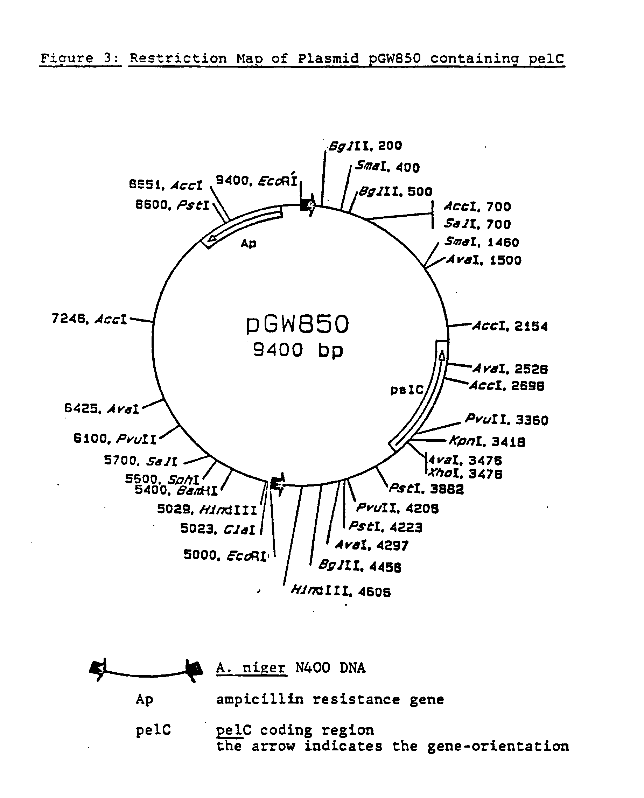

- Figure 3: shows the restriction map of pGW850 containing the pelC gene
- Figure 4: shows the restriction map of pGW840 containing the pelD gene

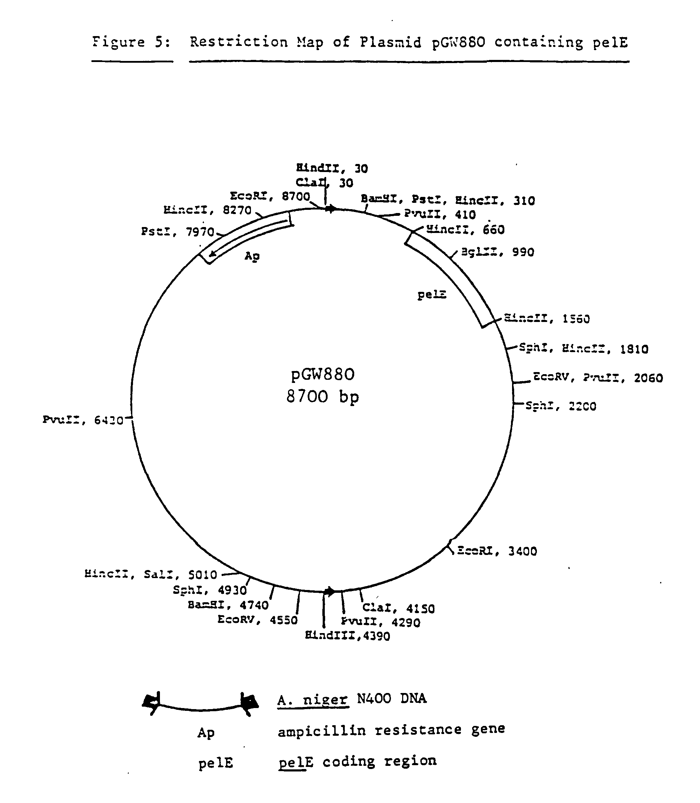

- Figure 5: shows the restriction map of pGW880 containing the pelE gene

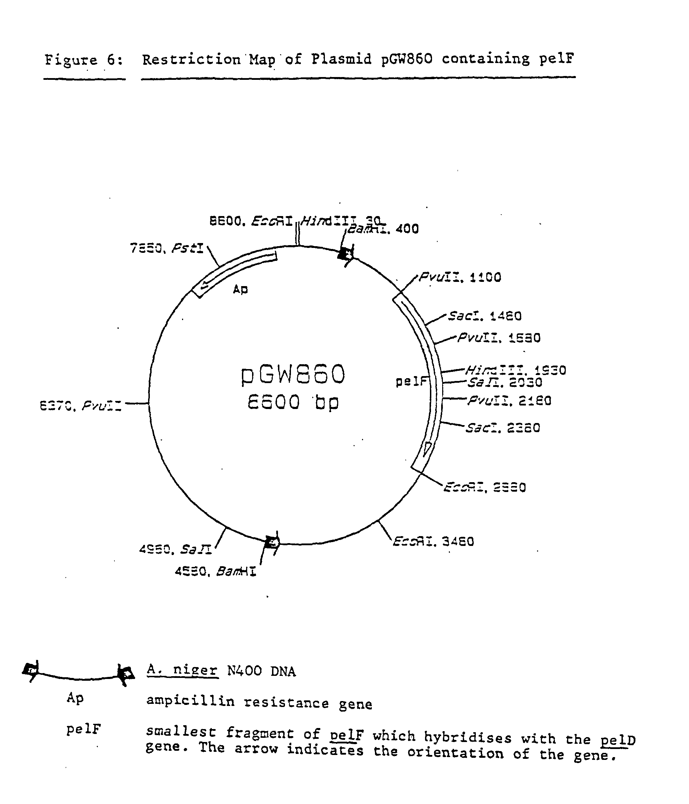

- Figure 6: shows the restriction map of pGW860 containing the pelF gene

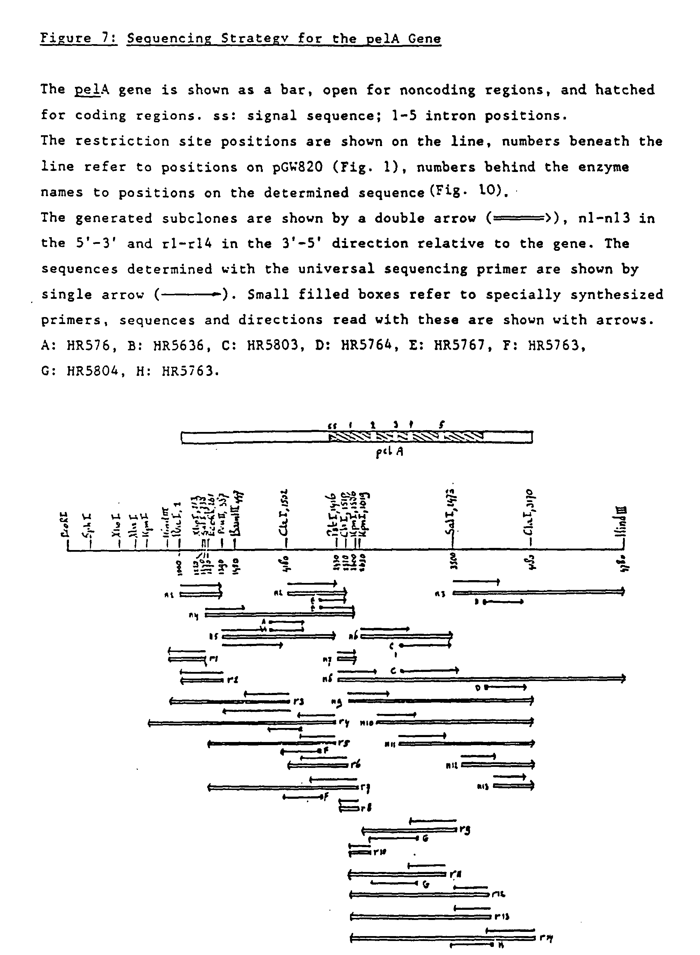

- Figure 7: shows the sequencing strategy for the pelA gene

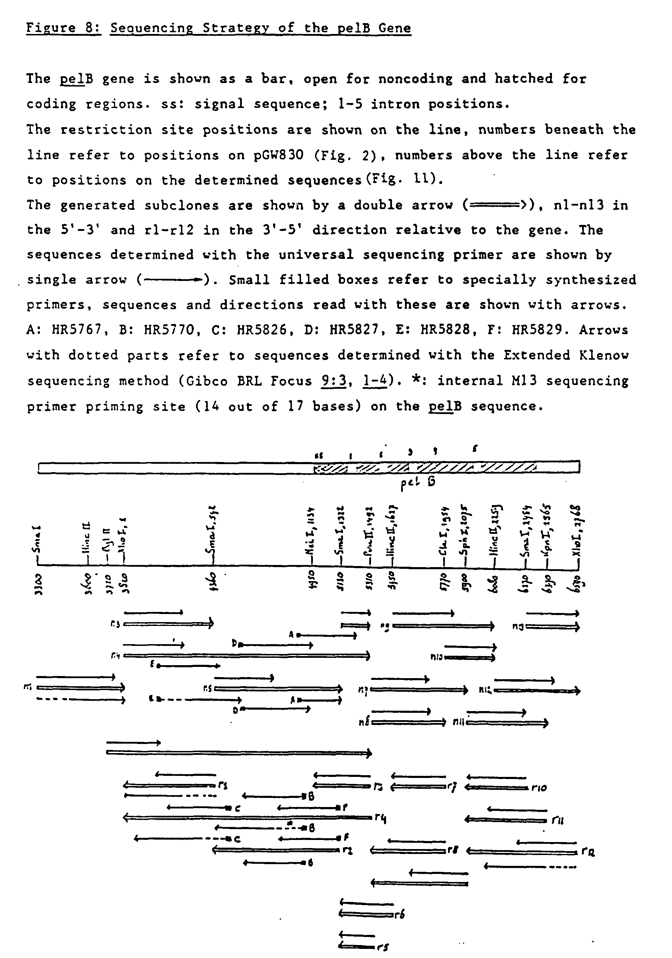

- Figure 8: shows the sequencing strategy for the pelB gene

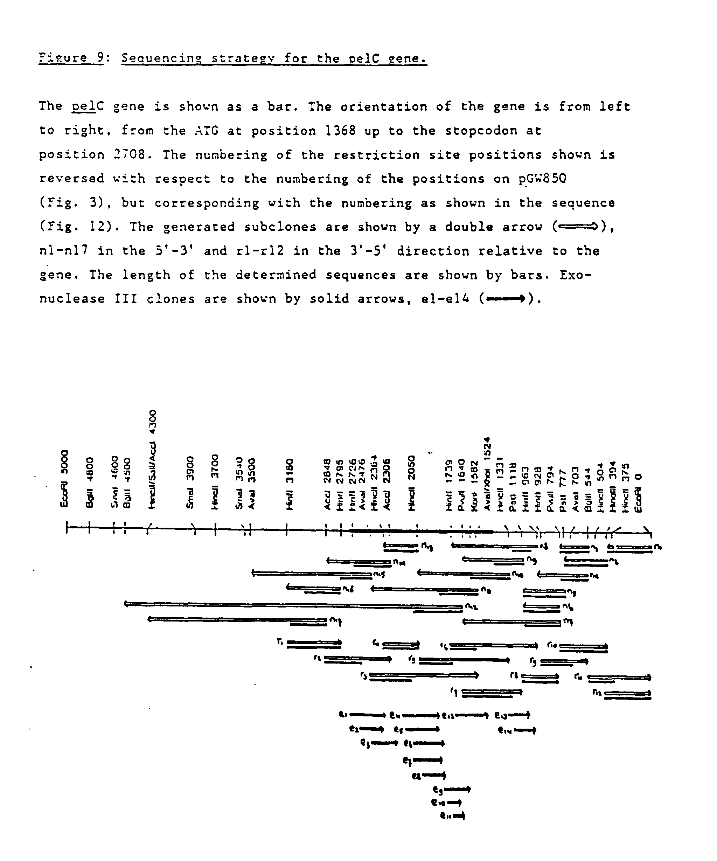

- Figure 9: shows the sequencing strategy for the pelC gene
- Figure 10: shows the sequence of the DNA molecule pelA, having Formula I, containing the gene coding for PLA
- Figure 11: shows the sequence of the DNA molecule pelB, having Formula II, containing the gene coding for PLB
- Figure 12: shows the sequence of the DNA molecule pelC, having Formula III, containing the gene coding for PLC.

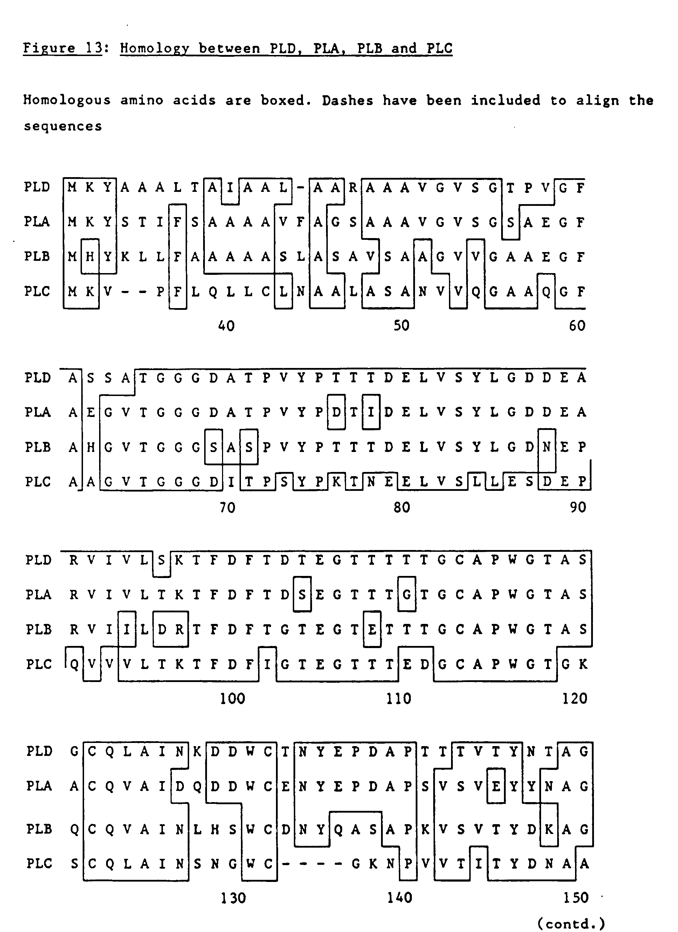

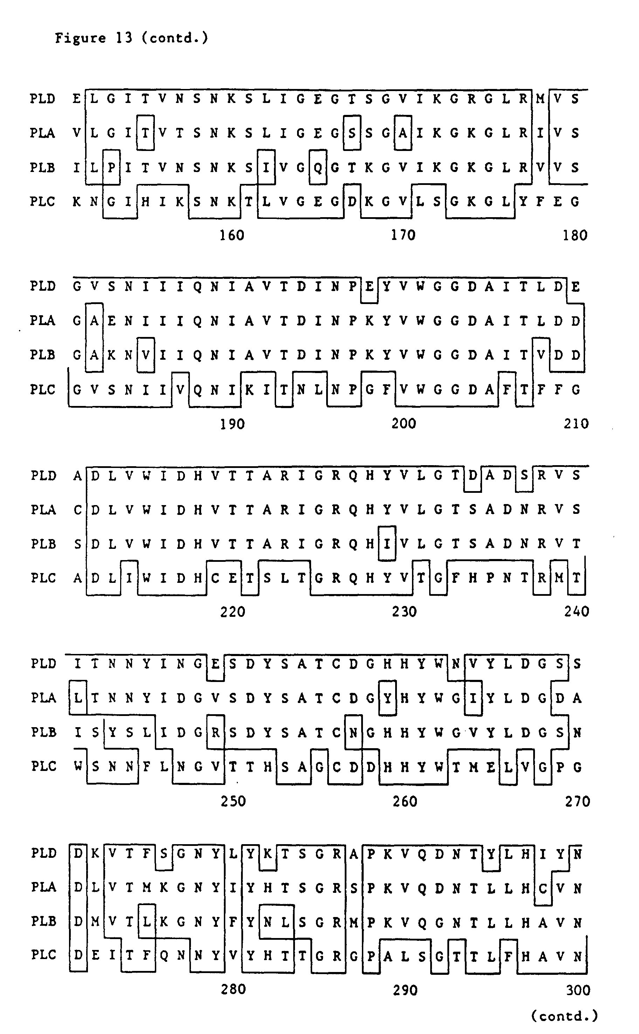

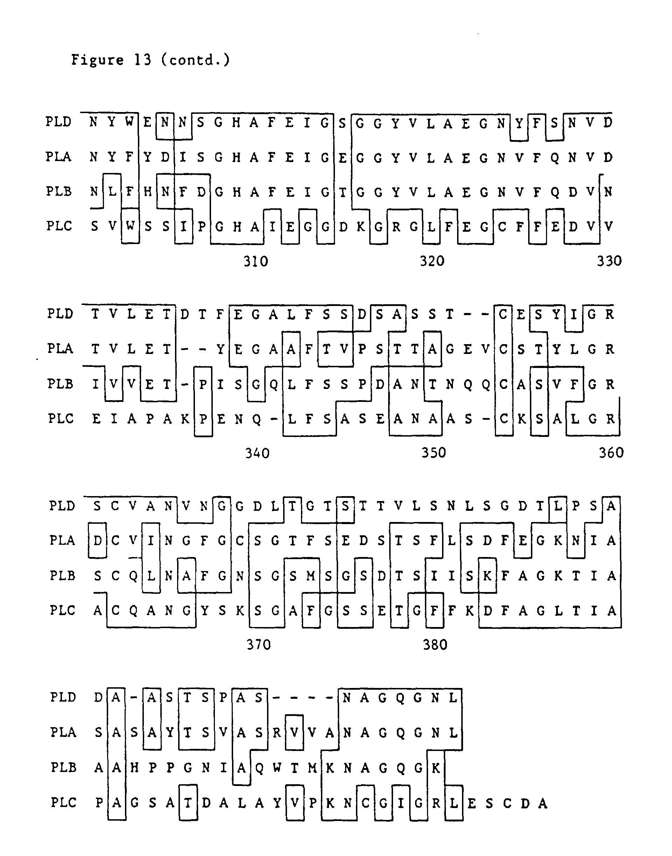

- Figure 13: shows the homology at the amino acid sequence level between PLD, PLA and PLC

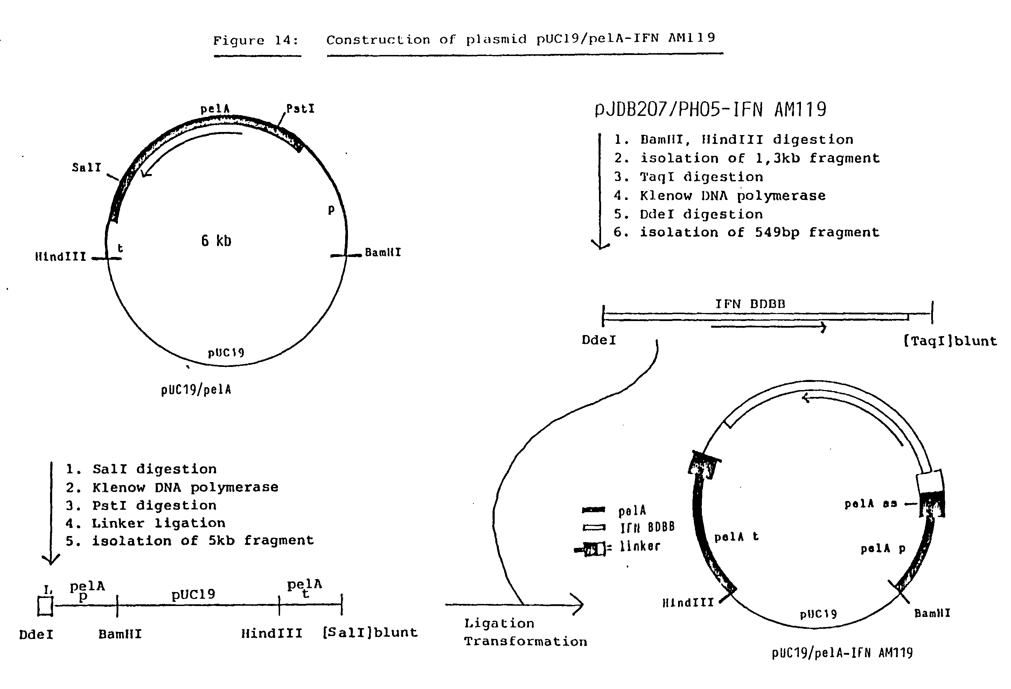

- Figure 14: shows the construction of the vector pUC19/pelA-IFN AM119, containing the gene coding for the hybrid interferon BDBB.

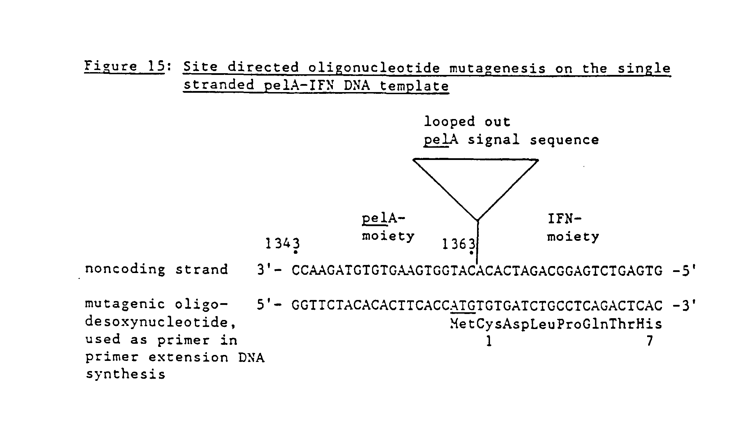

- Figure 15: shows the noncoding strand of part of the pelA-IFN fusion on plasmid pelA-IFN AM119 with looped out signal sequence aligned with the oligonucleotide primer used for deletion mutagenesis of the pelA signal sequence

The following examples serve to illustrate the invention, however are in no way intended to restrict it.

The abbreviations have the following meanings:
- Amp: ampicillin
- ATP: adenosine triphosphate
- BSA: bovine serum albumin
- CIP: calf intestine alkaline phosphatase
- DNA: deoxyribonucleic acid
- DTT: 1,4-dithiothreitol
- EDTA: ethvlenediaminetetraacetic acid disodium salt
- EGTA: bis-(aminoethyl)-glycolether)N,N,N',N'-tetraacetic acid
- kbp: kilobasepairs
- LMP: low melting point
- mOsm: milliosmoles
- PEG: polyethyleneglycol
- RNA: ribonucleic acid
- rpm: rotations per minute
- SDS: sodium dodecyl sulfate
- Tc: tetracycline
- Tris: tris(hydroxymethyl)-aminomethane
- U: units
- V: volt

### Buffers, media, reagents:

- HHA B/g: 10xrestriction-enzyme buffer used for BamHI, BgIII, HindIII, MboI, Pstl and XhoI digests, containing 60 mM Tris-HCI (pH 7.4), 60 mM β-mercaptoethanol, 60 mM MgCl₂, 500 mM NaCl, 0.1 % BSA, 0.1% gelatin
- EcoRlbuffer: 5Xrestriction-enzyme buffer used for EcoRI digests, containing 500 mM Tris-HCI (pH 7.2), 25 mM MgCl₂, 250 mM NaCl, 0.05 % BSA, 0.05 % gelatin
- IPTG: 100 mM isopropyl-β-thio-galactopyranoside (23.8 mg/ml) in H₂O
- LC medium: 1 % trypticase peptone (BBL), 0.5 % yeast extract (BBL), 0.8 % NaCl, 1 ml Tris-HCI pH 7.5 per litre
- minimal medium: 1.05 % K₂HPO₄, 0.45 KH₂PO₄, 0.1 % (NH₄)₂SO₄, 0.05 % for E.coli sodium citrate. 2H₂O, 1 mM MgSO₄, 1 mM thiamine-HCI, 0.2 % glucose
- 2XTY medium: per litre 16 g trypticase peptone (BBL), 10 g yeast extract, 5 g NaCl
- TBE-buffer: 1 litre contains 10.8 g Tris, 5.5 g boric acid, 4 ml 0.5 M EDTA (pH 8.0)
- TE buffer: 10 mM Tris-HCI (pH 8.0), 1 mM EDTA (pH 8.0)
- low TE buffer: 10 mM Tris-HCI (pH 8.0), 0.1 mM EDTA (pH 8.0)
- X-gal: 2 % (5-bromo-4-chloro-3-indolyl-β-galactoside) in dimethylformamide
- SSC: 0.15 M NaCI, 0.015 M sodium citrate
- minimal medium for A. niger: 1 litre contains 1.5 g KH₂PO₄, 0.5 g KCI, 0.5 g MgSO₄.7H₂O, 4.0 g NH₄Cl, 10.0 g glucose, traces of FeSO₄,,MnSO₄, ZnCl₂, adjusted to pH 6.5 with NaOH
- complete medium for A. niger: minimal medium plus 0.2 % trypticase peptone for (BBL) 0.1 % casaminoacids (Difco), 0.1 % yeast extract (BBL), 0.05 % ribonucleic acid sodium salt from yeast (ICN, Cleveland, USA), 2 ml vitamin solution per litre
- vitamin solution: per 100 ml 10 mg thiamine, 100 mg riboflavin, 10 mg panthotenic acid, 2 mg biotin, 10 mg p-aminobenzoic acid, 100 mg nicotinamide, 50 mg pyridoxin-HCI

For plates all media are solidified by addition of 1.5 % agar (BBL), for topagar(ose) 0.7 % agar (BBL) or agarose (Seakem) is used.
- PBS: per litre 0.37 g NaH₂PO₄, 2.7 g Na₂HPO₄, 8.5 g NaCI
- PSB: 10 mM Tris-HCI (pH 7.6), 100 mM NaCl, 10 mM MgCl₂, 0.05 % gelatine
- LDB: 10 mM Tris-HCI (pH 7.6), 100 mM NaCI, 10 mM MgCl₂

The following strains are used:
- A. niger: N400 wildtype
- A. niger N593: cspA, pyrA
- A. niger N756: selected for high production of pectinase complex enzymes
- A. niger AN8: DSM 3917, uridine anxotrophic mutant of A. niger N756
- E.coli NM539: metB, supE, hsdM⁺, hsdR⁻, supF, (P2cox3)
- E.coli MH1: ara D139, ΔlacX74, galU, galK, hsr⁻, hsm⁺, strA,
- E.coli JM103: Δlac-pro, thi, strA, supE, endA, sbcB, hsdR4, F¹, traD36, proAB, laclg, ZΔM15
- E.coli JM109: Δ(lac-proAB), recA1, endA1, gyrA96, thi, hsdR17, supE44, relA1, λ⁻, [F', traD36, proAB, lacI^{q}, ZΔM15]
- E.coli CJ236: (dut-1, ung-1, thi-1, relA-1;pCJ105(Cm^{r}); BIO-RAD Muta-Gene M13 in vitro mutagenesis kit)
- E.coli MV1190: [Δ(lac-proAB), thi, supE, △(srl-recA)306::Tn10(tet^{r}) (F':traD36, proAB, lac 1^{q}Z△M15)]. Strain MV1190 is described in the manual for the BIO-RAD MUTA-GENE M13 in vitro mutagenesis kit

The following vectors are used:

### EMBL4

EMBL4 is a lambda replacement vector with a cloning capacity of 9-23 kbp (Frischauf et al., ref. 2). It contains a multiple cloning region between the lambda arms and the nonessential stuffer region. This allows multiple restriction enzyme digestions to be performed in a manner such that religation of the stuffer to the vector arms is reduced as the foreign DNA of interest is inserted. The vector also makes use of the Spi phenotype to provide a direct selection for recombinants (Zissler et al., ref. 20).

### pCG3B11

This plasmid has been described in the European Patent Application 88 101 397.3, it contains the pelD (PLI) gene of A. niger N756 cloned in pBR322.

### pPL29-5

This plasmid has been described in the European Patent Application 88 101 397.3, it contains the N-terminal EcoRI fragment of the pelD (PLI) gene.

### pPL35-5

This plasmid has been described in the European Patent Application 88 101 397.3, it contains the C-terminal EcoRI fragment of the pelD (PLI) gene.

### pBR322

Plasmid pBR322 carries genes for resistance to the antibiotics ampicillin (amp^{R}) and tetracycline (tet^{R}). The plasmid carries several unique cloning sites, of which some are located in either the amp or tet gene so that insertion of cloned DNA leads to anti biotic-sensitive bacteria.

### pEMBL18 and pEMBL19

These plasmids have been described by Dente et al. (refs. 7, 8).

### pGW613

This plasmid has been described by Goosen et al. (ref. 12).

### M13mp phage

The M13mp18 and M13mp19 vectors (Norrander et al., ref. 21) are derivatives of the single-stranded DNA bacteriophage M13 and are designated to facilitate DNA sequencing by allowing cloning of DNA fragments at a versatile polylinker site and the cloning of the same restriction fragment in both possible orientations. Sequences cloned into these vectors can readily be used as templates for sequencing reactions or the production of single-stranded probes using standard oligodeoxyribonucleotide primer and the Klenow fragment of the E. coli DNA polymerase I. The vector DNA is carrying the E. coli lac-operon promoter and the gentic information of the first 145 amino acids of β-galactosidase. The polylinker sequences containing multiple restriction sites are inserted into the lacZ sequence. The polylinker retains the lacZ reading frame and the vector gives allelic complementation of a LacZα host strain, yielding blue plaqes on plates containing IPTG and X-gal. Recombinant phages containing inserts that destroy the reading frame or otherwise interfere with expresssion of the lacZα peptide are revealed as colorless plaques.

### pCG 59D7

This plasmid is described in EP 88 101 397.3 and can be obtained from Escherichia coli BJ5183/pCG59D7 (DSM 3968). The plasmid comprises the pyrA gene and is used for cotransformation of A. niger pyrA⁻ mutants.
M13(+)KS (Bluescript) (STRATAGENE, San Diego, CA, USA)

dsDNA vector, comprising the origin of replication of M13 phage. In presence of a helper phage, e.g. M13K07 (ref. 29) or R408 (ref. 9), (+)-strand of the vector is replicated, packaged and set free into the medium.

### M13 K07

Helper M13 phage for M13(+)KS. Described in Mead et al. (ref. 29).

### R408

Helper M13 phage for M13(+)KS. Described in Russell et al. (ref. 9).

### Example 1.: Construction of a genomic library of Aspergillus niger.

### Example 1.1.: Isolation of high molecular weight DNA from A. niger.

Conidiospores of Aspergillus niger strain N400 are inoculated in 200 ml minimal medium in a final spore density of 10⁶ spores/ml and shaken in 1 I Erlenmeyers for 24 hrs at 28 C at 300 rpm using a New Brunswick rotary shaker. The mycelium is harvested by filtration using a Büchner funnel with Myracloth, washed with cold sterile saline, frozen in liquid nitrogen and either stored at -60°C or used directly. The method used for isolation of DNA to prepare the genomic library is based on the procedure described by Yelton et al. (ref. 1). The DNA yield is-about 50-100 µg/g mycelium with this method.

For library construction, 10 g mycelium is ground in liquid nitrogen in 1 g portions in a Braun microdismembrator. The ground mycelium is transferred to a 1 I sterile erlenmeyer, containing 200 ml extraction buffer (50 mM EDTA pH 8.5, 0.2 % SDS) and 200 µl diethylpyrocarbonate. The mixture is slowly heated to room temperature and then heated for 20 min to 68° C while occasionally shaking. The suspension is cooled to room temperature and centrifuged for 15 min. at 12,000 x g 1/16 volume of an 8 M potassium acetate solution pH 4.2 is added to the supernatant and the mixture is left on ice for 1 hr. The precipitate is removed by centrifugation (20 min.; 16,000 x g; 4°C). The nucleic acids are precipitated from the supernatant by an incubation with 0.6 volume of isopropanol on ice for 15 min. The pellet is collected by centrifugation (10 min.; 6,000 x g; 4°C), washed with 70 % ethanol and briefly dried. The pellet is suspended in 10 ml TE containing 20 µg/ml RNase A, (Boehringer, Mannheim) and incubated for 15 min. at 37°C. The DNA is treated with nuclease free pronase (1 mg/ml final concentration) (Kochlight, Coinbrook) for 1 hr at 37°C. The pronase stock solution in TE buffer contains 20 mg/ml of enzyme which is incubated for 1 hr at 37°C to digest nucleases.

8.5 g CsCI is dissolved in 9 ml DNA solution, 0.2 ml 10 mg/ml ethidiumbromide is added and this solution is either centrifuged in a Beckman SW41 rotor for 60 hrs at 33,000 rpm, or in a Beckman 50Ti rotor with quickseal tubes for 40 hrs at 45,000 rpm. The DNA band is collected by side-puncturing of the tube. Ethidiumbromide is removed by multiple extraction with water- and NaCI saturated isopropanol. 5 volumes of TE are added, the DNA is extracted with TE saturated phenol, phenol/chloroform/isoamylalcohol 25:24:1 and chloroform/ isoamylalcohol 24:1. The DNA is precipitated by addition of 0.1 volume of 3 M sodium acetate pH 5.2, 2.5 volumes of ethanol and an overnight incubation at -20 ° C. The precipitate is collected by centrifugation (1 hr; 30,000 x g; 4°C), washed with 70 % ethanol, dried and dissolved in 400 µl low TE.

### Example 1.2.: Partial digestion of A. niger DNA with Mbol and isolation of fragments.

To test for the Mbol concentration which gives the largest amount of fragments between 13.6 and 23 kbp, 1 µg portions of A. niger N400 DNA are digested in the appropriate buffer with decreasing amounts of Mbol (0.5-0.001 U) for 1 hr at 37°C in a volume of 10 µl. The reaction is stopped by addition of 1 µl 0.25 M EDTA, and the samples are loaded on a 0.6 % agarose gel in TBE, containing 1 µg/ml ethidiumbromide. Convenient markers are a mixture of lambda DNA and lambda DNA digested with Bglll, which gives bands of 49, 22.8, 13.6, 9.8, 2.3, kbp and a nonvisible fragment of 0.45 kbp. The Mbo I concentration, required to give a high yield of the desired 13.6-23 kbp fragments is 0.02 U/µg DNA. Accordingly, 200 µg of DNA in a total volume of 2 ml are digested, and are divided into 20 portions of equal size immediately after addition of the enzyme. After 1 hr at 37° C the digests are placed on ice and a 1 µg sample is run on a gel to test for proper digestion. As the result is positive, EDTA is added to a final concentration of 25 mM to stop the reaction, the enzyme is heat-inactivated at 65°C for 10 min, samples are pooled and the DNA is precipitated, washed, dried and dissolved in 400 µl TE.

The fragmented DNA is separated overnight on a 0.4 % preparative agarose gel (center well 120 x 1.5 mm). Lambda DNA digested with Bgl II is used as marker to determine the size of the partially digested DNA fragments upon electrophoresis at 4°C and 40 V (3 V/cm). The gel region containing fragments of the correct size is cut out of the gel and the DNA is electroeluted from the gel in a sterile dialysis tubing in 2 ml TBE during 2-3 hrs at 100 V. The current is reversed for 30 s, and the buffer containing the DNA is collected. The fragments are then concentrated by ethanol precipitation and dissolved in 100 µl low TE.

### Example 1.3.: Preparation of vector DNA and cloning of high molecular weight DNA fragments of A. niger into EMBL 4.

The genomic library of A. niger strain N400 is constructed into the lambda vector EMBL4. The vector, which has a cloning capacity of 9-23 kbp, is described by Frischauf et al. (ref. 2) and Karn et al. (ref. 3) and has been purchased from Promega Biotech. Inc. To avoid double inserts originating from different parts of the genome, we have used a minimal fragment length of 13.6 kbp for cloning.

10 µg lambda EMBL4 DNA is digested to completion with 50 units of BamHI in the appropriate buffer in a volume of 100 µl for 2 hrs at 37°C. The enzyme is inactivated for 10 min. at 65°C. The NaCI concentration is raised to 150 mM and 50 units of Sall are added and incubation at 37°C proceeds for another 2 hrs. After addition of EDTA to 25 mM and inactivation of the enzyme (10 min. 65°C) the solution is extracted with equal volumes of phenol (TE saturated), phenol/chloroform/isoamylalcohol 25:24:1, chloroform/isoamylalcohol. To eliminate the small BamHI/Sall polylinker fragments, the DNA is precipitated with 0.6 volume of isopropanol after the addition of 0.1 vol. 3M sodium acetate pH 5.2. After 15 min. on ice and 15 min. centrifugation at 12,000 x g and 4°C, the precipitate is thoroughly washed with 70 % ethanol, dried and dissolved in 40 µl low TE.

### Example 1.4.: Ligation and in vitro packaging of genomic A. niger DNA fragments.

It is essential that the cos sites of the vector prepared according to example 1.3 are annealed prior to the ligation reaction. The vector in 100 mM Tris-HCl pH 7.5 and 10 mM MgCl₂ is heated for 10 min at 65°C and then annealed for 1 hr at 42 ° C. From test ligations a ratio of vector to fragments of approximately 1:1 (by weight) is found to give a maximum of recombinants. Ligation takes place in 50 mM Tris HCI pH 7.5, 10 mM MgCl₂, 10 mM DTT and 1 mM ATP, using 9.5 µg of vector and 10 µg of DNA fragments in a total volume of 100 µl. Then DNA ligase (BRL) is added at a concentration of 0.5 U/µg DNA and the ligation mixture is incubated overnight at 14°C. To test for ligation a sample of the ligated DNA is run on an agarose gel. Also, an amount of 0.5 µg of vector is ligated without the addition of fragments in a 5 µl volume.

The large volume ligation mixture is concentrated by ethanol precipitation and dissolved in 20 µl low TE prior to in vitro packaging. In vitro packaging is done with Promega Packagene extracts according to the instructions of the manufacturer using 10 µl portions to package 1 µg of DNA. Of the high molecular weight lambda c1857 Sam 7, supplied with the extracts, 1 µg is separately packaged to provide a control. After packaging 500 µl of phage solution buffer (PSB) is added and 5 µl of chloroform. The recombinant phage stocks can be stored at 4°C. The library obtained has been constructed from two separate ligation experiments.

### Example 1.5.: Titration and amplification of the A. niger strain N400 genomic library.

Cells of E. coli NM539 are grown on LC medium containing 0.2 % maltose, 10 mM MgSO₄ and 1 mM CaCl₂ to an optical density (600 nm) of 1.0. Then aliquots of 0.2 ml of this culture are added to 0.1 ml of a phage dilution series in PSB. After adsorption of the phages for 20 min at 37 °C, 3 ml 0.6 % LC top-agar of 45°C is added, the mixture is plated on LC agar plates and these are incubated overnight at 37°C. The titration results expressed as the number of plaque forming units (pfu) per ml phage suspension are 12x10⁵ and 4.2x10⁵ pfu/ml for the two phage stocks prepared according to example 1.4. After subtracting the background which is calculated from the control ligations without fragments (17 % and 40 % respectively) the absolute number of recombinants is 6x10⁵ which is over 200 times genome length.

To amplify the library, portions of 80 µl of both phage stocks are used to infect E. coli NM539 cells which are plated in LC top-agarose on LC agar plates and then incubated overnight at 37°C. The phages are eluted from the agarose by gently shaking the plates with 5 ml PSB per plate for 1 hr at room temperature. The PSB is collected, centrifuged (10 min at 6000 xg) to remove bacteria and chloroform is added (0.5 % final concentration). Both phage stocks, which are approximately amplified to the same extent, are then mixed (40 ml stock), titrated (8x10⁹ pfu/ml) and stored at 4°C.

### Example 2. :Screening the genomic library of A. niger N400 for the pectin lyase D gene (pelD) and isolation of the gene.

### Example 2.1.: Screening of the library.

For the isolation of the pelD gene from the lambda genomic library obtained as described in Example 1, 2.5 x 10⁴ phages are mixed with liquefied LC top-agarose and plated out on 5 LC agar plates. The plates are incubated overnight at 37°C and chilled for 2 hrs at 4°C. From each plate 2 replicas are made according to the Benton and Davis (ref. 4) plaque hybridization method. The first filter (Schleicher and Schüll BA85 or Millipore HATF 085) is placed on top of the plate for 1 min, the second replica for 2 min and the position of the replicas is marked using India ink.

After removal of the filters they are placed in a dish containing 100 ml of a denaturing solution (1 M NaCI, 0.5 M NaOH) for 1 min, and for 1 min in 100 ml renaturing solution (0.5 M Tris/HCL pH 7.5, 1.5 M NaCl). Then the filters are transferred to a dish containing 3 x SSC (SSC = 0.15 M NaCl, 0.015 M sodiumcitrate pH 7.0). The filters are gently scrubbed with a gloved hand to remove bacterial debris, transferred to a fresh dish containing 3 x SSC and gently shaken for 20 min at room temperature. The filters are blotted on Whatman 3 MM paper and dried for 10 min at room temperature. The filters are baked on 3 Whatman MM paper in an oven at 80 °C for 2 hrs. Subsequently they are washed for 0.5 hr in 6 x SSC at room temperature and then transferred to 50 ml of the prewarmed (56 ° C) prehybridization mixture which consists of 50 mM Tris/HCl pH 7.5, 10 mM EDTA, 1 M NaCI, 0.5 % SDS, 0.1% sodium pyrophosphate, 10 x Denhardt's (50 x Denhardt's = 1 % BSA, Boehringer fraction V; 1 % polyvinylpyrrolidone -40; 1 % Ficoll 400). To the prehybridization mixture one freshly adds: 0.1 mg/ml denatured salmon sperm DNA (Maniatis, et al. p 327; ref. 5) and 0.01 mg/ml poly rA. Prehybridization is for 4 hrs at 56°C with gentle shaking. The probe used for hybridization is the nick translated 3.5 kbp Eco RI fragment of pPL35-5, which contains the C-terminal region of the pelD gene of the A. niger N756 strain (available from E.coli BJ5183/pCG3B11, DSM 3916, EP 88 101 397.3). The fragment is previously isolated from a low melting agarose gel and 0.3 µg of the fragment is nick-translated (Maniatis, et al. pp 109-112; ref. 5). The nick-translated DNA is denatured for 10 min in a boiling water bath, cooled on ice and added to 50 ml of prewarmed prehybridization mixture. The prehybridized filters are transferred to the hybridization mixture one by one. Hybridization is at 56°C overnight while gently shaking. The filters are washed at 56°C: 2 x 30 min with 0.5 I 4 x SSC, 0.1 % SDS, and 2 x 30 min. with 2 x SSC, 0.1 % SDS. The filters are blotted on 3 MM paper and dried by air for 1 hr. After sticking them to 3 MM paper and proper marking with radiolabeled ink, the filters are covered with Saran wrap and auto-radiographed overnight at -70°C using Konica X-ray films and Kodak X-Omatic cassettes with regular intensifying screens. In this way, 44 positive signals are obtained from the 5 plates screened. Positive plaques are picked with a sterile Pasteur pipette by carefully positioning the plates on the autoradiogram in the right way using the ink markers. The pieces of agar containing the positive plaques are dispensed in 1 ml of PSB. The phages are allowed to diffuse from the agar during 1 hr at room temperature with occasional vortexing. The agar and bacterial cell debris are removed by centrifugation for 5 min, 10 µl chloroform is added and the phage stocks are stored at 4°C. The positive clones are named λ-PL1 to λ-PL44. Since phages are plated in high density, the positive plaques have to be purified twice by plating them at low density (±100 phages/ plate; 0.1 ml of 10³ dilution of the phage stock) and repeating the whole procedure of replica plating, hybridization and picking of positive plaques.

### Example 2.2. :Isolation of lambda DNA.

To isolate DNA from the recombinant clones, phages are first amplified by infecting E. coli NM539 with 0.1 ml of phage stocks from purified clones as described in example 1.5, and plating the cells in LC top-agarose on LC agar plates. This gives plates with confluent lysis of the indicator bacteria. 5 ml PSB is spread over the plates and phages are eluted during 2 hr with gentle shaking. The eluted phages are harvested, cellular debris is removed by centrifugation and chloroform is added to 1 %. The resulting plate lysate is stored at 4 °C. It usually has a titer of about 10¹¹ pfu/ml.

Since small scale isolation procedures from plate lysate stocks and from small scale liquid cultures (Maniatis et al., pp 65-68; ref. 5) do not always result in digestible DNA in our hands, phages are isolated from 250 ml lysates. These are prepared by growing the host, E. coli NM539, to an O.D.₆₀₀ of 0.3 in LC + 0.2 % maltose 10 mM MgSO₄, 1 mM CaCl₂ and then adding approximately 10¹⁰ pfu of the plate lysate. Upon further growth, the bacteria lyse within 4 hrs.

RNase and DNase are added to the lysates to a final concentration of 2 µg/ml, and the lysate is left at room temperature for 1 hr. Cellular debris is removed by centrifugation (20 min., room temperature 10,000xg). 14.8 g NaCI and 25 g PEG6000 are dissolved in the supernatant to obtain the final concentrations of 1 M NaCI and 25 % (w/v) PEG. The phages are left to precipitate for 1 hr on ice or overnight at 4°C, and harvested by centrifugation (20 min.; 10,000 x g; 4°C). The pellets are suspended in 3 ml of lambda dilution buffer (LDB; = 10 mM Tris/HCl pH 7.5, 20 mM MgCl₂, 100 mM NaCl). 2.5 g CsCl is dissolved in 4 ml of phage suspension and the mixture is pipetted in 5 ml Beckman quickseal tubes which are then filled up with the same concentration of CsCI in LDB. The tubes are sealed and centrifuged overnight in a Beckman VTi 65.2 rotor at 50,000 rpm and 4°C. The turbid phage band, visible under a normal lamp, is punctured from the side of the tube. CsCI is removed by dialysis in sterile tubing against 2 I of 10 mM Tris/HCl pH 7.5, 10 mM MgCl₂, 50 mM NaCl at 4°C for 4 hrs. The buffer is changed once. Phages are collected in sterile Greiner tubes, the volume is adjusted to 1 ml with the dialysis buffer, 50 µl of 10 % SDS, 50 µl 0.5 M EDTA pH 8.0 and 25 µl of 20 mg/ml nuclease free pronase are added and the tubes are incubated for 1 hr at 37°C. The volume is raised to 3 ml with TE, and this solution is extracted with equal volumes of TE saturated phenol, phenol/chloroform/isoamylalcohol 25:24:1 and chloroform-/isoamylalcohol 24:1. After addition of 0.1 volume of 3 M sodium acetate pH 5.2 and 2.5 volumes of ethanol, the DNA is precipitated overnight at -20°C and collected by centrifugation (1 hr 20,000 x g at 4°C). The pellet is washed with 70 % ethanol, dried, and dissolved in 200 µl low TE. The yield of phage DNA is about 200 µgs/250 ml lysate.

### Example 2.3. :Restriction analysis of pelD clones.

From the positive phages, 10 were at random selected for further analysis. 1 µg of phage DNA is digested with 10 units of EcoRI or Bglll in a volume of 20 µl for 2 hrs at 37°C in the buffers recommended by the supplier. The samples are separated on a 0.6 % agarose gel using 1 µg lambda cI857 Sam 7 DNA digested with HindIII (lambda x HindIII) as markers and EMBL4 DNA and pCG3B11 DNA digested with EcoRI as controls. The gel is photographed on a UV transilluminator using Polaroid 667 films (4s, diafragma 8). The DNA is transferred to Schleicher and Schüll BA85 nitrocellulose filters according to the method of Southern (1975) as described by Maniatis pp 382-386; (ref. 5). The filters are hybridized with a mixture of 32P labelled (nick-translated) fragments of the pelD gene covering the whole gene. These fragments are the 2.9 kbp EcoRI fragment of pPL29-5 and the 3.5 kbp EcoRI fragment of pPL35-5. Hybridization and washing conditions are identical to those described in example 2.1.

Band lengths are calculated from the photograph and autoradiograms by graphical comparison on semilogarithmic paper with the known marker bands. The restriction maps of the clones are given in Fig. 1. Clones λ-PL4, -14, and -40 contain both the 2.9 and 3.5 kbp EcoRI hybridizing fragments as found in pCG3B11. λ-PL5, -6, -10 and -33 contain the 3.5 kbp EcoRI fragment together with another hybridizing EcoRI fragment. Clones λ-PL26 and -28 contain the 2.9 kbp EcoRI fragment together with another hybridizing fragment, whereas λ-PL9 only contains a small 1.2 kbp hybridizing EcoRI fragment. The complete 5.0 kbp Bglll fragment of pCG3B11, containing the whole structural pelD gene, is only present in clones λ-PL4, -14 and -40. The 3.7 kbp hybridizing band, present in clones λ-PL5, -6, -10, -14, -33 and -40, but not in pCG3B11, is located downstream of the pelD gene in the 5.0 kbp fragment. Some hybridizing Bglll fragments still contain 1.2 kbp of the right arm of the vector. All the clones also contain nonhybridizing fragments that are identical. Since it therefore appears that the restriction patterns of both the structural gene and the gene environments are identical in all clones, it is concluded that they originate from the same gene viz the pelD gene of A. niger strain N400. From the number of clones plated and the assumed genome length of 3x10⁷ bp, for 25,000 clones with an average insert length of 15 kbp at least 12 pelD clones are expected to be found. Since only 2 out of the 10 clones analyzed are identical, the complexity of the library is even higher, than supposed (cf. Example 3).

### Example 2.4.: Subcloning of pelD fragments.

The 5.0 kbp BgIII fragments of λ-PL4 and pCG3B11 containing the pelD genes of A. niger N400 and N756 respectively, are subcloned into the BamHI site of plasmid pBR322 (see cloning vectors). 4 µg of λ-PL4 and 2 µg of pCG3B11 are digested to completion in 20 µl with 10 U of BgIII for 2 hrs at 37°C. The fragments are separated on a 1 % low melting point LMP agarose (BRL) gel in TBE + 1 µg/ml ethidium bromide at 50 V. The 5.0 kbp fragment is cut out from the gel, diluted with 0.4 ml TE, an equal volume of phenol is added and the mixture is heated to 65 ° C for 10 min to melt the agarose. After centrifugation for 5 min at 12000 rpm in an Eppendorf 5414S table centrifuge, the aqueous phase is extracted with phenol/chloroform and chloroform, ethanol precipitated, washed, dried and finally dissolved in 10 µl low TE.

1 µg pBR322 DNA, prepared by the large scale plasmid preparation method (Maniatis, et al. p 86; ref. 5) and purified by CsCI gradient centrifugation, is digested with 5 U BamHI for 2 hrs at 37°C in a volume of 20 µl. 2 µl of 10 x CIP buffer and 0.02 units of CIP are added and the incubation is continued for another 30 min. EDTA is added to a final concentration of 25 mM, and the mixture heated for 10 min. 65°C. The solution is diluted to 200 µl with TE and extracted three times with TE saturated phenol, once with phenol/chloroform and once with chloroform. After ethanol precipitation, the DNA is dissolved in 50 µl low TE.

100 ng of fragment DNA is ligated with 20 ng of vector DNA in a volume of 20 µl, under conditions described in example 1.4. Half of the ligation mixture is used to transform E. coli MH1 competent cells, prepared by the CaCl₂ method (Maniatis, et al. p 250; ref. 5). Cells are plated on LC agar plates containing 50 µg/ml ampicillin and incubated overnight 37°C. Transformants are tested for tetracycline sensitivity by streaking colonies first on LC plates containing 20 µg/ml Tc, and then on LC + amp plates. Transformants are grown overnight in 5 ml of LC + amp at 37 °C, and DNA is isolated from 1.5 ml of the culture; using the alkaline lysis miniprep method (Maniatis, et al. p 368; ref. 5).

The miniprep DNAs are digested with BamHI and Pstl and the fragments analyzed on a 1 % agarose gel. Two plasmids originating from λ-PL4, containing the 5.0 kbp Bglll fragment in opposite orientations, are designated pGW840 and pGW841. Further plasmids originating from pCG3B11 are designated pGW870 and pGW871. The MH1 cells harbouring them are kept on glycerol at -70°C. Plasmid DNA from 0.5 I cultures of these clones is isolated on a large scale (Maniatis, et al. p 86; ref. 5), purified by CsCI centrifugation, phenolized, ethanol precipitated and dissolved in 400 µl low TE. The yield is approximately 500 µg. The plasmids are subjected to restriction mapping. The map for plasmid pGW840 is shown in Fig. 2 and is indistinguishable from pGW870. Also, plasmids pGW841 and pGW871 which contain the fragment in the opposite orientation are indistinguishable, indicating identity of the pelD genes from A. niger strain N400 and N756.

### Example 3.: Screening for, isolation and characterization of genes related to pelD.

### Example 3.1.: Establishing hybridization conditions by analysis of genomic blots of A. niger N400 DNA.

2 µg DNA samples of A. niger strain N400 , isolated as described in Example 1.1, are digested in a volume of 20 µl for 4 hrs at 37°C using BamHI (BRL) or HindIII (BRL) in HHA B/g buffer. The digested samples are electrophoretically separated on 0.6 % agarose gels at 40 V for 18 hrs. The DNA is transferred to nitrocellulose filters and baked as described in Example 2.3. (Pre)-hybridization solutions are identical to those described in Example 2.1.

The temperature used for prehybridization is always the same as the hybridization temperature. The nick translated 1.6 kbp BamHI/Pstl fragment of pGW840 which contains the coding region of the pelD gene, has been used as a probe. Hybridization is carried out at different temperatures (52, 60 and 68°C) for 16 hrs and 40 hrs. The following two washing conditions are used at the three different temperatures: 2 x 30 min 5 x SSC, 0.1 % SDS followed by 2 x 30 min 3 x SSC, 0.1 % SDS as compared to 4 x 30 min 5 x SSC, 0.1 % SDS. For 68°C washing twice with 2 x SSC, 0.1% SDS and twice with 0.2 x SSC, 0.1 % SDS is also included. At 68°C, using 0.2 x SSC washings, only homologous hybridization occurs. Using higher salt concentrations gives rise to the appearance of some other weak signals. At 52°C the background is high and hybridization weak. The best conditions found for "heterologous" hybridization are 60°C for 40 hrs using the washing combination of 5 x SSC and 3 x SSC. This can even be further optimized by using 4 x SSC in combination with 2 x SSC instead of 5 x SSC in combination with 3 x SSC. The signals seen in these genomic blots of A. niger N400 when probed with the 1.6 kbp BamHI/PstI fragment of pGW840 are summarized in Table I.

**Table 1**

| Hybridization signals in genomic DNA of A. niger N400 probed with the 1.6 kb BamHI/PstI from pGW840. | |
|---|---|
| BamHI | HindIII |
| 7.5 kbp strongly homologous; pelD | 21.0 kbp strongly homologous; pelD |
| 4.3 kbp strong | 3.9 kbp strong |
| 4.1 kbp weak | 7.1 kbp weaker |
| 18.0 kbp weak | 4.4 kbp weak |
| 8.4 kbp very weak | 4.6 kbp weak 1.5 kbp very weak |

### Example 3.2. :Screening of the library.

2.5 x 10⁴ phages from the N400 lambda library are plated on 5 plates and 3 nitrocellulose replicas are made from each plate, as described in Example 2.1. The (pre)hybridization buffer is also described in Example 2.1. The first replica from each plate is hybridized at 60 ° C for 40 hrs with the 1.6 kbp BamHI/PstI fragment of pGW840 and washed twice at that temperature for 30 min with 4 x SSC, 0.1 % SDS and twice for 30 min with 2 x SSC, 0.1 % SDS as described in Example 3.1. These conditions are referred to as heterologous conditions. The second replica from each plate is hybridized at 68 ° C with the same fragment washing twice for 30 min with 2 x SSC, 0.1 % SDS and twice for 30 min with 0.2 x SSC, 0.1 % SDS at this temperature. These conditions are referred to as homologous conditions. The third replica from each plate is hybridized homologously with the 0.35 kbp BamHl fragment of pGW840, which is located in the promoter region of pelD directly adjacent to the 1.6 kbp BamHI/PstI fragment. 29 plaques are obtained which hybridize heterologously but not (or very weakly) homologously. These are named λ-PL101 to λ-PL129. 19 plaques are obtained which hybridize strongly homologously and heterologously with the BamHI/PstI probe. These are designated λ-PL130 to λ-PL148 and are considered to be pelD clones. Of these, 2 hybridize only weakly with the 0.35 BamHl probe λ-PL145 and λ-PL146), and therefore probably contain only part of the promotor region. The others hybridize strongly. Only 1 clone is obtained which hybridizes only with the 0.35 kbp BamHl probe (λ-PL149).

All clones are picked and purified twice by plating and hybridizing them heterologously with the 1.6 kbp BamHI/PstI probe. In a second screening experiment 23 pelD clones and 25 other clones have additionally been obtained (λ-PL151 to λ-PL198).

### Example 3.3.: Characterization of lambda clones by plaque hybridization signal with different probes derived from pelD.

In this experiment a classification of the isolated clones is described by using different parts of the coding region of pelD as probe. Included are λ-PL101 to -130, -145 whereas λ-PL4 is used as a positive control. The clones are plated out and only one replica is made which is divided into 6 parts. This is meant to rule out differences in hybridization signal between replicas. Separate parts of the replicas are hybridized both homologously and heterologously with the following 32P labelled probes:
1: the complete 1.6 kbp BamHI/PstI fragment from pGW840
2: the 649 bp BamHI/Xhol fragment from pGW840 (N)terminal coding part of pelD)
3: The 244 bp Xhol/PstI fragment from pGW840 (C-terminal coding part of pelD)
λ-PL4, -130 and -145, as well as λ-PL101 hybridize strongly with these probes under homologous and heterologous conditions, as is expected for pelD clones. The latter clone, λ-PL101, is located at the edge of a filter in Example 3.2 and therefore not scored as pelD in the first screening. The clones just mentioned are classified as Class I (pelD) clones. λ-PL112, -126 and -127 turn out to be negative in this experiment. All other clones can be divided into two other classes: Class II hybridizes heterologously not only with the whole probe but also with the N-terminal probe. Homologous hybridization is only weak with the whole pelD gene as a probe. Class III clones do not hybridize with the N-terminal probe at all, and also not homologously with the whole probe. The C-terminal probe fails to hybridize with both Class II and Class III clones. From these experiments we conclude that there are at least 2 related genes amongst the clones isolated.

To the Class II clones belong: λ-PL104, -105, -109, -113, -114, -115, -119, -122, -124, -125, -128 and -129.

The Class III clones are: λ-PL102, -103, -106, -107, -108, -110, -111, -116, -117, -118, -120, -121 and -123.

### Example 3.4. :Restriction analysis of the isolated lambda clones (Class I, II).

Plate lysate stocks, 250 ml liquid lysates and large scale phage DNA preparations from these lysates are prepared as described in Example 2.2. This is done for 24 clones, 3 of them being pelD clones (λ-PL4, -130, -145). One clone DNA is lost during DNA isolation (λ-PL124). The DNA isolated from these clones is digested in 1 µg samples in a total of 20 µl with 10 units of enzyme. All clones are digested with EcoRI, BamHI, HindIII. EcoRI/Bam,HI, Bam/HindIII, EcoRI/HindIII and EcoRI/BamHI/HindIII. Fragments are separated on a 0.6 % agarose gel, and transferred to nitrocellulose filters as described in Example 2.3. Blots are hybridized heterologously with the 1.6 kbp BamHI/PstI fragment of pGW840 as described in Example 3.1. Two clones fail to hybridize (λ-PL112 and λ-PL129). Band lengths from both the photographs and autoradiograms are calculated. Except for the λ-PL113 clone, all clones contain too many fragments to derive a complete map.

However, it is possible to derive a map of the hybridizing region and, by combining data on other nonhybridizing bands, to assign which clones are derived from the same gene. It is clear that among the remaining 18 Class II and III clones analyzed, 5 genes are present. These are called pelA, pelB, pelC, pelE and pelF. The assignment of clones to these genes is summarized in Table II.

**Table II**

| Assignment of isolated clones to different pel genes (Part) means that the hybridizing band are only partially present in these clones. | | |
|---|---|---|
| Class | Gene | λ-Clones |
| I. | pelD | PL4, PL-130, PL145 (part) |
| I. | pelA | PL113, PL104, PL115, PL125 (part) |
| | pelB | PL119, PL122, PL128, PL105 (part) |
| | pelC | PL109 |
| III. | pelE | PL116, PL107 (part) |
| | pelF | PL102, PL103, PL110, PL120 PL121 (part), PL123 (part) |

**Table III**

| Comparison of hybridizing bands in chromosomal blots and isolated genes. The band length are in kbp. | | | | |
|---|---|---|---|---|
| | EcoRI | BamHI | HindIII | |
| Chromosomal | 2.9 + 3.5 + larger bands | 7.5 | 21.0 | Homologous signal pelD |
| | | 4.3 | 3.9 | Strongest heterologous signals |
| | | 18.0 | 7.1 | Weaker signals |
| | | | 4.4 | Weak signals |
| | | 8.4 | 4.6 | Weak signals |
| | | 4.1 | 1.5 | Weak signals |
| pelD | 2.9 + 3.5 | 7.5 | large | |
| pelA | 7.5 | 4.3 | 3.9 | |
| pelB | 8.7 | large | 7.1 | |
| pelC | 5.0 | large | 4.4 | |
| pelE | 6.2 | 8.4 | 4.6 | |
| pelF | large | 4.1 | 1.5 + 2.8 (very weak) | |

A comparison of the hybridizing fragment lengths of the isolated genes and the hybridizing fragment lengths from genomic DNA blots is shown in Table III. From these data it is concluded that all the bands hybridizing in genomic blots, are present in the clones isolated and that under these hybridization conditions, no other related genes can be isolated. The plaque hybridization results (Example 3.3.) reveal that, not considering partial clones, every gene has a specific hybridization pattern with the probes tested. This is summarized in Table IV.

**Table IV**

| Comparison of signal strength of different genes in homologous and heterologous plaque hybridization with different pelD probes according to the experiment described in Example 3.3. The degree of hybridization is expressed by the number of + signs. The homologous pelD gene hybridizes with at least 10 + sings; ±, very poor hybridization; -, no hybridization. | | | | | | |
|---|---|---|---|---|---|---|
| | heterologous hybridization | | | homologous hybridization | | |
| probe | whole gene | N-term | C-term | whole gene | N-term | C-term |
| pelA | ++++ | ++++ | ± | +++ | ± | - |
| pelB | ++++ | ++++ | ± | + | ± | - |
| pelC | +++ | ++++ | - | - | - | - |
| pelE | +++ | - | - | - | - | - |
| pelF | ++++ | ± | ± | *±* | - | - |

### Example 3.5.: Subcloning of pelD related genes into pBR322.

Subclones of the hybridizing fragments which are obtained from λ clones described in Example 3.3 are made in the vector pBR322 which is digested by EcoRI, BamHI or HindIII and subsequently dephosphorylated. Fragment isolation, from LMP agarose gels, vector preparation, ligation, transformation of E. coli MH1, miniprep DNA isolation and large scale plasmid isolation is all done using standard procedures which have been described in Example 2.4.

Fragments are ligated into the proper vector as described in Example 2.4. Transformed E. coli MH1 cells are plated on LC + 50 µg/ml Amp. Transformants are tested for tetracycline sensitivity. EcoRI clones are all resistent. Miniprep DNAs are then digested with appropriate enzymes to test for the right inserts and to determine the orientation of the fragments. The plasmids which have been selected are summarized in Table V. Cells harbouring them are stored on glycerol at -70 °C.

**Table V**

| Subclones of pel genes in pBR322. | | | | |
|---|---|---|---|---|
| plasmid | gene | fragment | origin | orientation |
| pGW820 | pelA | 7.5 EcoRI | λ-PL113 | 2 |
| pGW821 | | 4.3 BamHI | | 2 |
| pGW822 | | 3.9 HindIII | | 2 |
| pGW823 | | 7.5 EcoRI | | 1 |
| pGW824 | | 4.3 BamHI | | 1 |
| pGW825 | | 3.9 HindIII | | 1 |
| pGW830 | pelB | 7.1 HindIII | λ-PL122 | 1 |
| pGW850 | pelC | 5.0 EcoRI | λ-PL109 | 1 |
| pGW851 | | 5.0 EcoRI | | 2 |
| pGW860 | pelF | 4.1 BamHI | λ-PL102 | 1 |
| pGW880 | pelE | 4.6 Hindlll | λ-PL109 | 1 |
| pGW881 | | 4.6 HindIII | | 2 |

The plasmids pGW820, -830, -850, -860 and -880 have been isolated on a large scale and subjected to restriction mapping. Maps of these plasmids are given in the Figures 1 to 6.

To determine gene location and orientation, Southern blots of plasmid digests are hybridized heterologously with the 1.6 kbp BamHI/PstI fragment of pGW840, and identical blots are hybridized heterologously with a N-terminal fragment of the same plasmid, which is the 649 bp BamHI/Xhol fragment for the mapping of pGW820 an -830, and the 766 bp BamHI/EcoRI fragment for pGW850 and -860.

The plasmids pGW820, pGW830, pGW850, pGW860 and pGW880 were cloned in E.coli HB101 and deposited on February 1, 1988, at the Deutsche Sammlung für Mikroorganismen, Grisebachstr. 8, 3400 Göttingen. They received the DSM-Nos. 4388, 4389, 4390, 4391 and 4392, respectively.

### Example 4.: Sequence determination of the pelD, pelA, pelB and pelC gene.

### Example 4.1.: Partial sequence of the pelD gene from A. niger strain N400 and its identity with pel I from A. niger strain N756.

Suitable restriction fragments are isolated from pGW840 after LMP agarose gel electrophoresis as described in Example 2.4. These are ligated into M13mp18RF and M13mp19RF vectors according to the BRL M13 cloning/dideoxy sequencing instruction manual (pp 26, 27; ref. 6). Transformation of E. coli JM103, plating on minimal X-gal indicator plates and isolation of single stranded DNA from recombinant phages are done according to the instructions in the same manual (pp 29-34). A few clones are sequenced using the Sanger dideoxy chain termination method described at pp 38-74 of the BRL manual.

The sequences determined between nucleotides -457 and +100 are identical to the corresponding sequence of the PLI gene derived from A. niger N756 and present in plasmid PCG3B11 (EP 88 101 397.3). The sequence determined from the BamHI at position -457 up to position +100 comprises 457 nucleotides of the promotor sequence, the 57 nucleotides encoding the signal sequence and 43 nucleotides encoding the first 13 N-terminal amino acids of the mature PLD protein.

From identical restriction maps of the N400 pelD gene in pGW840 and of the N756 pelD in pCG3B11 and the completely identical N-terminal sequence data it is assumed that both genes are identical. Accordingly it is assumed that the PLD protein is identical with the former PLI protein.

### Example 4.2. :Sequence determination of the pelA, pelB and pelC gene.

Fragments of pGW820, pGW830 and pCW850 are subcloned into M13 mp18RF and M13 mp19RF (see Example 4.1.) or into the plasmids pEMBL18 and pEMBL19 (Dente et al. ref. 7, 8). Single stranded DNAs of the plasmid clones are obtained by infection with helper phage R408 (Russell et al. ref. 9).

Exonuclease III deletion clones were prepared of pGW850 by the method of Henikoff (ref. 28). The 3.0 kb Smal-Bglll fragment from pGW850 on which the pelC gene is situated is subcloned in pEMBL19. 50 µg of this clone is digested with Smal and Sacl and after phenol/chloroform extraction and ethanol precipitation digested with exonuclease III. Samples are taken at different time intervals, the exonuclease III inactivated by adding NaCl and EDTA and incubation at 70°C for 10 min. Protruding ssDNA ends are removed by S1 nuclease and the sticky ends made blunt with T4 DNA polymerase. After selfligation and transformation of E. coli JM103 with these deletion clones single stranded DNAs are obtained by infection with helper phage R408. pGW820 is sequenced from the Pvull site at position 1000 until the Clal site at position 4175 (see Fig. 1). In the case of pGW830 the 2774 bp Xhol fragment is sequenced (see Fig. 2). pGW850 is sequenced from the EcoRI site at position 0 until position 3168 (see Fig. 3).

The sequence strategies for these genes are indicated in Fig. 7, 8 and 9. Several oligonucleotides are synthesized using the phosphoramidite method of Caruthers (ref. 10) using an Applied Biosystem (model 380B) oligonucleotide synthesizer. They are used as sequencing primers and their position is indicated in Fig. 7 and 8.

The entire sequence of the pelA gene is given in Fig. 10 in the 5'→3' direction. The 3175 bp sequence starts with the first nucleotide of the Pvull site at position 1000 in pGW820 and ends at the last nucleotide of the Clal site at position 4175 in pGW820.

The pelA sequence comprises 1360 nucleotides of the promoter region, 1355 residues of the structural part and 360 nucleotides of the terminator region.

The amino acid sequence of PLA (see Example 6.3.) is preceded by a signal sequence of 20 amino acids as can be deduced from the nucleotide sequence. 
MET-LYS-TYR-SER-THR-ILE-PHE-SER-ALA-ALA-ALA-ALA-VAL-PHE-ALA-GLY-
 *   *   *                       *       *   *           *
SER-ALA-ALA-ALA
     *   *   *
 The homology with the signal sequence of the pelD gene is indicated by an asterisk. Also, the first 5 residues of the mature protein have the same amino acid sequence in pelA and pelD.

The entire sequence of the pelB gene is given in Fig. 11, also in the 5'→3' direction. The 2774 bp sequence starts with the first nucleotide of the Xhol site in pGW830 and ends at the last nucleotide of the second Xhol site.

The pelB sequence comprises 1133 nucleotides of the promoter region, 1373 residues of the structural part and 268 of the terminator region. The pelB gene codes for a signal sequence of at least 20 amino acids.

The pelC sequence comprises 1367 nucleotides of the promoter region, 1340 residues of the structural part and 461 of the terminator region. The pelC gene codes for a signal sequence of 18 amino acids. 
pelC MET-LYS-VAL-PRO X - X -PHE-LEU-GLN-LEU-LEU-CYS-LEU-ASN-ALA-ALA
pelB *                 *                             *
pelA *   *             *                             *
pelD *   *                                 *         *
 
pelC LEU-ALA-SER-ALA
pelB          *   *
pelA      *       *
pelD  *   *       *
 The homology with the signal sequence of the pelA, pelB and pelD gene is indicated at each position with an asterisk. The X's are included for alignment of the sequences.

Searches for consensus sequences for fungal exon/intron splice junctions and intron internal sequence (Ballance, ref. 11) leads us to postulate the presence of four introns in the pelA, B and C gene. The positions at which the introns are located within the coding regions of these pel genes are conserved in the sense that there are potentially five intron positions but in each gene a different intron lacks. However, for the pelC gene only three introns are postulated. Of these only one is found in a position corresponding to the position of an intron in pelD and pelA (intron 5). The positions of these introns and their respective lengths are summarized in Table VIa.

**Table VIa**

| Positions of introns in the sequences of pelD, pelA, pelB and pelC. Positions refer to the coding sequences in Fig. 10, 11, and 12 and to the sequence of pelD | | | | |
|---|---|---|---|---|
| gene | pelD | pelA | pelB | pelC |
| intron | position length bp | position length bp | position length bp | position length bp |
| 1 | 202-267 66 | absent | 1337-1398 62 | absent |
| 2 | 410-471 62 | 1708-1759 52 | 1543-1598 56 | absent |
| 3 | 598-660 63 | 1886-1933 48 | 1725-1781 57 | absent |
| 4 | absent | absent | absent | 1853-1930 78 |
| 5 | 1446-1502 57 | 2031-2094 64 | absent | 1998-2062 65 |
| 6 | absent | absent | absent | 2232-2294 63 |
| 7 | absent | 2329-2382 54 | 2113-2169 57 | absent |

The lengths of the exons between introns amongst the four pel genes, is the same in these cases. In Figure 13 the aligned derived amino acid sequences of PLA, PLB, PLC, and PLD are shown. In the N-terminal part of the coding regions of pelA, pelB and pelD approx. 80 % homology is observed on the basis of nucleotide sequence homology and over 80 % on the basis of amino acid sequence homology. This percentage is much lower with pelC. In the C-terminal part of pelA, pelB and pelD beyond residue 285 of the mature protein (corresponding to residue 305 in Fig. 13) the homology is lost to a large extent and only returns near to the C-terminus.

Except for the consensus sequences of the splicing signal and of the 5'-and 3'-splicing sites (Table VIb) no homology is found in the introns.

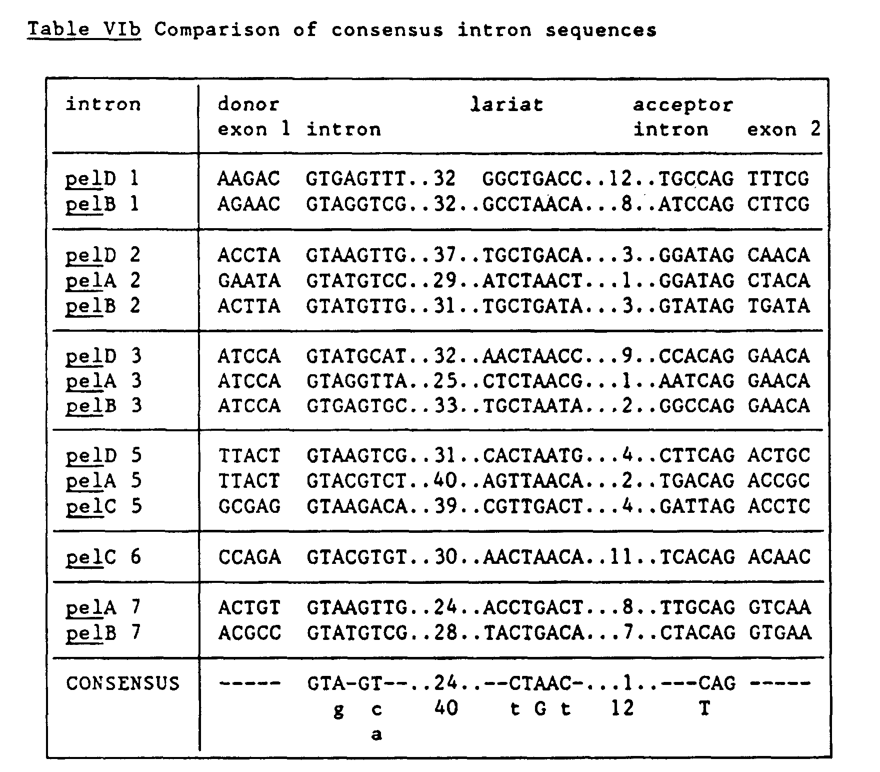

The derived amino acid sequences for the proteins PLA, PLB, PLC and PLD indicate a total of 359 residues for the first two proteins, 360 for PLC and 354 for PLD. With respect to N-glycosylation, one potential glycosylation site is present in all four proteins at position 109 (in the mature protein) (for PLC this corresponds with position 105 in the non-aligned sequence). In PLB a second potential site is present at position 232 whereas in PLD two other potential glycosylation sites are present at residues 255 and 329.

### Example 5: Cotransformation of A. niger using the A. niger pyrA gene as selection marker and the various A. niger pel genes as cotransforming plasmids

### Example 5.1. Propagation and purification of plasmids used to transform A. niger

All plasmids are propagated in E. coli strain MH1 and plasmid DNA is recovered from 500 ml overnight cultures as described by Maniatis et al. pp 90-91; ref. 5). To 2.5 ml DNA solution in TE, containing up to 1 mg DNA, 2.2 g CsCI and 1 ml ethidium bromide (10 mg/ml in water) are added. The solution is taken to quick seal tubes, which are filled and sealed as recommended by the supplier (Beckman). Centrifugation takes place in a VTi 65.2 rotor at 20°C for 16 hrs at 45.000 rpm. Of the two fluorescent bands visible under ultraviolet light, the lower one containing plasmid DNA is isolated by side-puncturing of the tube. The DNA solution (approx. 1 ml) is extracted 5 times with water-saturated butanol to remove ethidium bromide. Then the DNA is precipitated by ethanol, resuspended in TE, extracted once with phenol/chloroform and chloroform, precipitated again and stored at -20 °C.

The plasmid pGW613 which carries the OMP-decarboxylase gene (pyrA) on a 5 kbp A. niger DNA fragment is used to select transformants (Goosen et al.; ref. 12). Plasmids pGW820, pGW830, pGW840, pGW850, pGW860 and pGW880 which are described in Example 3.5 are used as co-transforming plasmids.

### Example 5.2. :Preparation of protoplasts and transformation of the uridine auxotrophic mutant A. niger strain N593.

The A. niger strain N593 (cspA, pyrA) which is a derivative of the parental strain N400 has been obtained by positive selection against the toxic analogue 5-fluoro orotic acid like in yeast (Boeke et al.; ref. 13) in the presence of uridine as described by Goosen et al.; (ref. 12).

Liquid minimal medium supplemented with 0.5 % yeast extract, 0,2 casamino-acids, 10 mM uridine (Janssen Chimica) and 50 mM glucose is inoculated with 10⁶ conidiospores of A. niger N593 per ml and incubated for 20 hrs at 30 ° C in a New Brunswick orbital shaker. Mycelium is harvested by filtration, through Miracloth, washed with iso-osmotic (STS) minimal medium (STC) of the following composition: 1.33 M sorbitol, 10 mM Tris-HCI pH 7.5, 50 mM CaCl₂ adjusted to 1.7 mOsm. An amount of 1 g of mycelium is resuspended in 20 ml STC. Protoplasts are released from the mycelium within 2 hrs by adding 250 mg of filter-sterilized Novozym 234 (Novo Industries, Denmark) and incubating the mixture at 30 ° C in a shaker at 95 rpm. The protoplasts are separated from residual mycelium by filtration using a funnel with a plug of glasswool and purified by centrifugation (10 min, 2500 rpm) by pelleting and resuspending in STC.

For transformation 5 x 10⁶ protoplasts are taken in 200 ul which are then incubated together with 1 µg pGW613 and 10 µg of one of the following plasmids pGW820, pGW830, pGW850, pGW860 or PGW880 (volume less than 20 µl). For pGW840 a ratio of 1:3 is used taking 6 µgs of pGW613. After the addition of plasmid DNA to the protoplasts 50 µl PCT (10 mM Tris-HCI pH 7.5, 50 mM CaCl₂, 25 % PEG6000) is added and the incubation mixture is kept on ice for 20 min.

Then another 2 ml PCT is added and the mixture is incubated for a further 5 min at room temperature. Finally, 4 ml STC are added and mixed. Aliquots of 1 ml of this final transformation solution are mixed with 4 ml liquified iso-osmotic MM top-agar stabilized by 0.95 M sucrose (adjusted to 1.7 mOsm). The protoplast mixture is immediately plated on agar plates containing the same iso-osmotic minimal medium and these are incubated at 30 °C. Appropriate control experiments include protoplasts treated similarly without plasmid DNA and on non-stabilized minimal medium with 2.5 mM uridine.

After 3 days of growth at 30°C well growing transformants appear which sporulate (50-150 transformants/µg pGW613). Besides, a large number of presumably abortive transformants appear. Spores of individual transformants are then taken and plated out separately on 50 mM glucose minimal medium to obtain single colonies which are used to propagate spores for further transformant analysis.

In each case at least ten transformants are cultured on liquid minimal medium, the DNA is extracted and analyzed for the presence of co-transforming plasmid DNA. Fungal DNA is isolated by a slightly modified procedure used to isolate plant RNA (Slater, ref. 14). Mycelium is washed with saline, frozen in liquid nitrogen and 0.5 g is disrupted using a microdismembrator (Braun). The mycelial powder is extracted with freshly prepared extraction buffer. The extraction buffer is prepared as follows: 1 ml tri-isopropylnaphthalene sulphonic acid (TNS) 20 mg/ml is mixed with 1 ml p-aminosalicylic acid (PAS) (120 mg/ml) and 0.5 ml 5 x RNB buffer (5x RNB contains 121.1 g Tris, 73.04 g NaCI and 95.1 EGTA in 1 I, pH 8.5). After the addition of 1.5 ml phenol, the extraction buffer is equilibrated for 10 min at 55°C. The warm buffer is then added to the mycelial powder and the suspension thoroughly mixed for 1 min using a vortex mixer. Then 1 ml chloroform is added and mixed for 1 min.

By centrifugation (10 min, 10.000 x g) the aqueous phase is separated and extracted once more with an equal volume of phenol/chloroform (1:1) and then twice with chloroform.

The aqueous phase contains both RNA and DNA. The DNA is precipitated with 2 vol ethanol at room temperature and collected by centrifugation (10 min, 10.000 x g), washed twice by redissolving in distilled sterile water and precipitating it again with ethanol. RNA is removed by adding RNase A (20 µg/ml) to the final solution. The procedure results in high molecular weight DNA (100-200 kbp) with a yield of about 300 µg DNA/g mycelium which is further purified by CsCl/ethidium bromide density gradient centrifugation essentially according to Maniatis et al. pg 93; (ref. 5).

Digests of the chromosomal DNA (2 µgs) are made by incubating for 2 hrs at 37° C with EcoRI for pelA (pGW820) and pelC (pGW850) transformants, Hind III for pelB (pGW830) transformants, EcoRI or Bgl II for pelD transformants. In all cases initially 20 U of restriction enzyme are used after which the incubation is prolonged for another 2 hrs by adding agains 20 U of the restriction enzymes. The digestions are carried out in the appropriate reaction buffers supplied by BRL.

Then the DNA is fractioned on 0.6 % agarose, blotted on nitrocellulose and hybridized essentially as described by Maniatis et al. (ref. 5), pp 382-386 using the corresponding [α-³² P] labelled probes.

The following cotransformation frequencies are found: pGW820 (pelA) 70 %; pGW830 (pelB), 70 %; pGW840 (pelD) 15 %; pGW850 (pelC) 60 %.

The mass ratio of pGW613 versus the cotransforming plasmid pGW820, pGW830, pGW850 (1:10) and pGW840 (1:3) leads in the majority of the cases observed to multicopy chromosomal integration of the cotransforming plasmids.

### Example 5.3.: Genomic analysis of the pelA transformed A. niger strain A15

A number of the multicopy tranformants described in 5.2 has been analyzed and the analysis of the pelA transformant A15 is given as a detailed example.

DNA of the A. niger strain N593 and of the multicopy transformant pelA strain A15 is isolated and analyzed by hybridization of Southern blots as described in Example 3.1. As a pelA probe the 7.5 kbp EcoRI fragment is used (see Fig. 3a). In the genomic blot an intense band of 7.4 kbp is found in the transformed strain A15 which results from type I and/or type II integration events (cf. Hinnen et al., ref. 14a). Also some minor bands of various lengths are found which represent border fragments of type II integration events of rearrangements.

The optical densities of the 7.4 kbp EcoRI bands in the A. niger pelA transformants A15 and A. niger N593 autoradiograms are scanned using an Ultroscan XL (LKB). The values are corrected for slight differences in the DNA concentration between N593 and the transformant A15 by scanning the densities obtained with a second probe which hybridizes with the pyruvate kinase gene, present as single copy in both strains. From these data it is calculated that in the pelA transformant A15 approx. 19 intact copies of the pelA gene are present.

Using the HindIII insert of pGW830 (Fig. 3b) as a probe, analysis of the Southern blot of the transformed strain A15 indicates that integration of pelA sequences has not occurred in the pelB locus. Similarly, analysis of the transformed pelB strain B13, the pelC strain C16 and the pelD strain D12 with the appropriate corresponding probes give copy numbers of approx. 15, 50 and 10.

### Example 5.4.: Northern blot analysis of induced and non-induced mycelia of the pelA transformed A. niger strain A15 and of A. niger N400.

Minimal medium (250 ml) containing 50 mM glucose or 1 % (w/v) of apple pectin (Brown Ribbon, d.e. 72.8 %, Obipektin AG, Bischofszell) is inoculated at a spore density of 10⁶ spores/ml with spores of transformant A15 or spores of the wild type strain N400 is harvested after 30 hrs of growth at 30°C. Samples of the culture medium (2 ml) are also collected, dialyzed against 2 I 10 mM sodium phosphate buffer pH 7.0 at 4° C and stored at -20° C.

Nucleic acids are isolated from the mycelium as described in Example 5.2. The RNA is precipitated from the aqueous phase after chloroform extraction by adding 1/3 volume of 8 M LiCI. The solution is thoroughly mixed and incubated overnight at 0°C. The RNA is collected by centrifugation (300 rpm for 30 min.) using a MSE Super-Minor centrifuge and then washed once with cold (-20 °C) 2M LiCI and once with cold (-20°C) 96 % ethanol. Finally the RNA is dissolved in distilled water at a concentration of about 1 mg/ml. The preparations essentially free of DNA with a yield of 1-2 mg RNA per g mycelium. Amounts of approx. 10 µg of RNA are run on denaturing formaldehyde 1 % agarose gels according to Maniatis et al. pp 202-203; ref. 5) using Hind III digested lambda DNA or the RNA ladder from BRL as molecular weight markers. The gels are blotted and baked on Nytran (Schleicher and Schuell) as recommended by the manufacturer and hybridized at 68°C for 16 hrs with the 7.4 kbp EcoRI pelA DNA probe. Hybridization and washing procedures are those described for DNA hybridization under homologous conditions according to Example 3.2.).

Both A. niger strains produce a pectin-inducible mRNA with a length of approx. 1.6 kbp. Scanning of the optical densities of the two autoradiograms indicates a 15-20 fold difference in intensity between the A15 transformant and the wild type strain which correlates with the increase in copy number calculated from the Southern blot analysis.

### Example 5.5: Pectin lyase production by the transformed A. niger strain D12

The A. niger pelD transformant D12 obtained according to Example 5.2 is grown in a 2-step cultivation procedure similar to the procedure described by Hermersdörfer et al (27) for the production of polygalacturonase. A mycelium layer is formed on top of a liquid culture by modulating 10⁶ spores per ml of Pre Cultivation Medium (PCM) consisting of sugar beet pulp (4 %) and NH₄NO₃ (1 %) pH 4.5. After 5 days growth period at 30 ° C the mycelium grown in 30 ml of medium is washed with saline and transferred to 50 ml of Main Cultivation Medium (MCM). This culture is grown at 30°C in an orbital shaker at 100 rpm. The composition of MCM is per liter of medium, glucose (5 %), pectin (d.e. 72.8 %; 0.1 %, NH₄NO₃ (0.75 %), KH₂PO₄ (0.5 %), FeSO₄ 7H₂O (0.03 %), MgSO₄ 7H₂O (0.03 %), CaCO₃ (0.03 %), NaNO₃ (0.03 %), pH 4.5. Samples have been taken during 24 hours at different time intervals and are analyzed by SDS polyacrylamide gelelectrophoresis. Expression of pelD is already maximal within 7 hours of cultivation. The protein migrates identical to PLI used as a reference.

### Example 5.6.: Pectin lyase A production by the transformed A. niger strain A15 and by A. niger N400.

The dialyzed culture filtrates (Example 5.4.) are lyophilized, resuspended in 0.2 ml distilled water and subjected to SDS-polyacrylamide gel electrophoresis (10 gels), using standard methods (Laemmli, ref. 15). The separated proteins are transferred to nitrocellulose (Towbin et al. ref. 16). Blots are saturated for 5 hrs at room temperatures with a 1 % BSA solution in 10 mM Tris-HCI buffer pH 7.5 with 0.35 M NaCl. This is followed by an incubation for 16 hrs at room temperature with polyclonal antibody (0.1 %) raised against two pectin lyases purified according to van Houdehoven (1975) in 50 ml of the same buffer containing 150 mM NaCI, 0.5 % BSAm 1 % Triton X100, 0.5 % deoxycholate and 0.1 % SDS. Blots are then rinsed 5 times with 200 ml PBS before incubation with 30 µl goat anti-rabbit IgG-HRP (Sigma) as 2nd antibody per 50 ml and washed again 5 times with PBS. The blots are finally stained using 4-chloro-1-naphthol as a substrate. 40 mgs of the substrate are solved in 0.5 ml 96 % ethanol and mixed with 100 ml 50 mM Tris-HCI pH 7.5 and 30 µl 30 % hydrogenperoxide and then added to the blots.

### Example 5.7.: Screening of pelA transformed strains by halo-formation on pectin-containing solid media.

Conidia of pelA transformed strains are inoculated at a spore density of approx 40 colonies per Petridish on sterile paper filters (Schleicher and Schuell) which are put on top of 1 % agar-solidified minimal medium containing 0.01 % Triton-X100 and apple pectin (1 % d.e. 72.8 % Obipektin) as carbon source. The incubation period is 72 hrs at 30°C resulting in individual colonies (2-4 mm). The paper filter is transferred to another sterile Petridish and the medium containing dish is stained with at least 5 ml of a ruthenium red (0.1 % w/v) solution in distilled water, incubated for 2 hrs and then washed several times with distilled water to remove the unadsorbed dye-stuff, following essentially a procedure described by Ried and Collmer (ref. 17) for polygalacturonate to characterize bacterial pectate lyase enzyme activities.

Transformants overproducing PL A protein are detected by an increase in halo-diameter around the individual colonies as compared to the parental strain N400.

### Example 6: Isolation, purification and characterization of pectin lyase A (PLA) from the A. niger pelA transformant A15.

### Example 6.1: Culture conditions to prepare pectin lyase A

The A. niger pelA transformant A15 described in Example 5 is grown on complete medium in Petridishes for 3 days at 28° C to produce conidia. The conidia are harvested from these plates by suspending the spores in 5 ml sterile saline containing 0.005 % Tween 80. The suspension is heavily agitated on a Griffin shaker for 20 min. Minimal medium (ref. 21) containing 1 % (w/v) of apple pectin (Brown Ribbon, d.e. 72.8 %; Obipektin AG, Bischofzell) is inoculated at a spore density of 10⁶ spores/ml using 250 ml of medium in 1 I siliconized Erlenmeyer flasks. The mycelium is grown for 40 hrs at 30 °C using a Gallenkamp orbital shaker at 200 rpm. After cultivation the mycelium is removed by filtration over Miracloth using a Büchner funnel. The culture filtrate (ref. 21) is diluted with distilled water (ref. 21), the pH of the filtrate (pH 3.7) is taken to pH 6.0 using 1 N NaOH and then filtered again using Whatman 1 filter paper.

### Example 6.2.: Purification of PLA

The diluted culture filtrate (4 I) is applied to a DEAE-Sepharose Fast Flow (Pharmacia) column (10 cm x 2.6 cm), pre-equilibrated with 20 mM sodium phosphate buffer pH 6.0. The column is eluted with the same buffer until the absorbance at 280 nm reaches a value <0.1 0.D. Pectin lyase activity is then eluted from the column by applying a linear gradient composed out of 20 mM sodium phosphate buffer (150 ml) and 20 mM sodiumphosphate buffer containing 1 M NaCI (150 ml).

Fractions are assayed for the presence of active pectin lyase according to the procedure described by van Houdenhoven (ref. 18 p 11) using Brown Ribbon pectin (d.e. 72.8 %) as a substrate. The activity appears around a concentration of 0.43 M NaCI in the salt gradient. Active fractions are then pooled and dialyzed twice against 1 I of 20 mM piperazine HCI buffer pH 5.5 (sample size: 45.5 ml).

In the next step the dialyzed sample is divided into 3 portions of equal size which are subjected to anionic exchange chromatography in three separate runs using a standard Pharmacia MONO Q column in combination with the Pharmacia FPLC system. The column is pre-equilibrated with 20 mM piperazine-HCI buffer pH 5.5. After loading of the enzyme sample the column is eluted with the same buffer at a flow rate of 1 ml/min until base-line absorbance is reached again. Then a linear salt gradient is applied. Within 22 ml of the elution buffer applied to the column, a final concentration of 0.6 M NaCI is reached.

The active fraction (4.4 ml) are collected, diluted to 25 ml using equilibration buffer and the same chromatography procedure is repeated.

Two fractions containing the active enzyme (total volume 3 ml) are then dialyzed against a 25 mM piperazine buffer pH 5.0 and injected in 1 ml portions onto a MONO P column (Pharmacia), pre-equilibrated with the same buffer. After the injections a pH gradient is formed using a 5 % solution of polybuffer TM 74 (in distilled water set at pH 3.0 using 4 N HCI). The active enzyme (3.2 ml) appears around pH 3.2. The preparation is extensively dialyzed against 50 mM sodium phosphate buffer pH 6.0 and stored at -20°C. The purification results are summarized in Table VIIa and are compared with the data of a similar purification for pectin lyase produced by A. niger strain N400 (Table VIIb).

**Table VIIa and VIIb**

| Purification scheme of pectin lyase A from the A. niger pelA N593 transformant and pectin lyase from A. niger strain N400. | | | |
|---|---|---|---|
| VIIa | | | |
| Step | Volume ml | Total activity (I.U.) | Specific pectin lyase activity (I.U./mg of protein) |
| DEAE Sepharose | 45.5 | 39.8 | 4.9 |
| MONO Q chromatography (2 x) | 2.3 | 19.9 | 18.4 |
| MONO P chromatography VIIb wild type | 3.2 | 17.4 | 28.6 |
| DEAE Sepharose | 43.0 | 10.1 | 0.9 |
| MONO Q chromatography (2 x) | 1.1 | 2.5 | 6.3 |
| MONO P chromatography | 1.4 | 1.7 | 10.0 |

Protein concentrations have been determined using the BCA protein assay reagent (Pierce) according to the instructions of the manufacturer.

Compared to the wild type total activity the total activity of the A. niger N592 transformed with pGW820 has increased after purification about 10 times and the specific activity about 3 times.

### Example 6.3.: Amino Acid sequence determination of the N-terminal part of pectin lyase A.

500 µg of pectin lyase (PLA), purified according to Example 6.2. are dialyzed three times against 1 I Millipore filtered distilled water and lyophilized. Amino acid sequences are determined with an Applied Biosystems model 470A protein sequencer, on-line connected with a 120 A PTH analyzer, according to the method described by Hunkapiller (ref. 17).

The following N-terminal amino acid sequence is determined for the enzyme:

VAL-GLY-VAL-SER-GLY-SER-ALA-GLU-GLY-PHE-ALA-GLU-?-VAl-THR-GLY-GLY-GLY-
ASP-ALA.

The amino acid sequence thus determined corresponds exactly with the one based on the nucleotide sequence of the pelA gene (see Example 4.2.).

### Example 6.4.: Properties of pectin lyase A

Both the A. niger strain N400 and the pelA transformant A15 are cultivated as described in Example 6.1 and the enzyme is purified from the culture filtrate according to the procedure in Example 6.2. The two enzyme preparations thus obtained have been compared with pectin lyase II which is purified according to van Houdenhoven (ref. 18).

SDS polyacrylamide gelelectrophoresis, using 10 % gels, results in a single band of identical molecular weight in all three cases applying in each case 2-5 µg of protein.

Isoelectric focussing has been performed using standard procedures (see e.g. instruction leaflet of Pharmacia, Isoelectric Focussing, 1982). A FSBE-3000 apparatus and the corresponding power supply ECPS 3000/150 (Pharmacia) have been used. The obtained PLA is homogeneous upon isoelectric focussing and has a lower isoelectric point (0.2 pH units) than PLII reported to be 3.75 according to van Houdenhoven (ref. 18). The PLII purified from the N400 culture filtrate is heterogeneous upon isoelectric focussing. The major band corresponds in position with the PLA protein. Two minor bands are shifted slightly towards the cathode. Thus in the A. niger multicopy transformant A15 the minor enzyme forms seen in N400 are lacking.

A direct comparison of the kinetic parameters of PLA and of PLII, which in the latter case were established by van Houdenhoven (ref. 18), using 95 % esterified apple pectin. as substrate indicates identical Kₘ and Vₘₐₓ values for both enzymes.

### Example 7 :Isolation, purification and characterization of pectin lyase D (PLD) from the A. niger pelD transformant D12

### Example 7.1 :Culture conditions to prepare pectin lyase D

The A. niger pelD transformant D12 obtained according to Example 5.2 is initially grown according to Example 5.5, in 300 ml aliquots. After a 5 day growth period in PCM at 30°C, the mycelial mat is then transferred to 20 mM sodium phosphate buffer (pH 6.0), containing 0.1 M sodium chloride. By shaking the mycelium in 150 ml on an orbital shaker at 200 rpm for 2 hr, most of the pectin lyase is released into the medium.

### Example 7.2: Purification of PLD

After removal of the mycelium by filtration, the enzyme solution is applied to a DEAE-Sepharose Fast Flow (Pharmacia) column (25 cm x 1.25 cm) preequilibrated with 20 mM sodium phosphate buffer (pH 6.0), containing 0.2 M sodium chloride. The column is eluted with the same buffer until the absorbance at 280 nm reaches a value <0.1. Pectin lyase activity is eluted from the column by applying a linear sodium chloride gradient (0:2 to 0.7 M) in 20 mM sodium phosphate buffer (pH 6.0) (800 ml total volume). The fractions are screened for the presence of pectin lyase D by SDS-polyacrylamide gel electrophoresis and Western blotting (Towbin et al., ref. 16) as described in Example 5.6. Fractions containing pectin lyase D are pooled and dialyzed against 20 mM sodium phosphate buffer (pH 6.0) containing 0.15 M sodium chloride, and subjected to anionic exchange chromatography using a standard Pharmacia MONO Q column in combination with the FPLC system. After loading, the column is wased with the same buffer before applying a 50 ml linear sodium chloride gradient in 20 mM sodium phosphate buffer (pH 6.0). Active fractions are collected and aliquots of 1 ml were applied to a 100 ml Superose-12 gel permeation column, equilibrated in 20 mM sodium phosphate (pH 6.0) containing 0.2 M sodium chloride and connected to the FPLC system. Active enzyme fractions (1 ml each) obtained from the GPC column are analyzed by SDS polyacrylamide gel electrophoresis to check the purity of the pectin lyase obtained.

**Table VIII**

| Purification scheme of pectin lyase D from A. niger pelD transformant D12 | | | |
|---|---|---|---|
| Step | volume (ml) | Activity (I.U.) | Specific pectin lyase activity (I.U./mg protein) |
| culture filtrate | 1950 | 109 | 0.096 |
| DEAE-Sepharose FF | 103 | 32.2 | 5.65 |
| MONO Q and GPC | 14.5 | 10.4 | 7.5 |

### Example 7.3: Amino acid sequence determination of the N-terminal part of pectin lyase D

500 µg of pectin lyase D, purified according to Example 6.2. are dialysed three times against 1 I Millipore filtered distilled water and lyophilized. Amino acid sequences are determined with an Applied Biosystems model 470A protein sequencer, on-line connected with 120A PTH analyser, according to the method described by Hunkapillar (ref. 19).

The following N-terminal amino acid sequence is determined for the enzyme:

VAL-GLY-VAL-SER-GLY-THR-PRO-VAL-GLY-PHE-ALA-SER-SER-ALA-THR-GLY-GLY-GLY-
ASP-ALA-THR

The amino acid sequence determined corresponds exactly with the one based on the nucleotide sequence of the pelD gene.

### Example 8: Isolation, purification and characterization of pectin lyase B (PLB) from the A. niger transformant B13.

### Example 8.1.: Culture conditions to prepare pectin lyase B.

The A. niger pelB transformant B13, obtained according to Example 5.2 is analyzed for its multicopy character according to Example 5.3. Nothern blot analysis, according to the procedure described in Example 5.4, shows that a pectin-inducible mRNA with a length of approximately 1.6 kb is produced. Growth for 35 hr at 30°C in minimal medium containing 1 % (w/v) citrus pectin (d.e. 72.8%) and 1 % of wheat bran is used to produce the enzyme as described in Example 5.4. The enzyme is also produced using a medium composed of 4 % (w/v) sugar beet pulp and 1 % NH₄HO₃.

### Example 8.2. :Purification of PLB

The culture filtrate is diluted two-fold with distilled water and pH is taken to pH 6.0 using 1 N NaOH and then filtered again using Whatman 1 filter paper. The diluted filtrate (2 I) is then applied to a DEAE-Sepharose Fast Flow (Pharmarcia) column (9 cm x 3.2 cm) which is preequilibrated with 50 mM sodium acetate buffer pH 5.0. Part of the pectin lyase activity is present in the eluate as can be assayed by the procedure described by van Houdenhoven (ref. 18, p. 11) using Brown Ribbon pectin (d.e. 72.8 %) or highly esterified pectin (d.e. 94 %). The majority of the pectin lyase activity can be eluted by applying a salt gradient. This activity appears in the salt gradient and is found to be identical to PLA on the basis of elution behaviour and apparent molecular weight on SDS-polyacrylamide gel electrophoresis as described in Example 6.

The eluate of the DEAE Fast Flow column is dialyzed against distilled water and applied to a MONO S column (Pharmarcia), which has been previously equilibrated with a 20 mM sodium acetate buffer pH 4.5. The enzyme is eluted from the column by applying a 0 - 1.0 M sodium chloride salt gradient in the same buffer. The enzyme is eluted almost immediately from the column. Active fractions are pooled, and then diluted approximately four-fold with distilled water. Rechromatography of the enzyme solution results in fractions which on the basis of SDS-polyacrylamide gel electrophoresis contain the pure PLB protein.

### Example 8.3. Properties of pectin lyase B

The properties of the enzyme purified from the pelB transformant B13 according to Example 8.2. are determined and compared with pectin lyase A and D.

SDS polyacrylamide gel electrophoresis using 10 % gels, results in a single band with an apparent molecular mass of 39.7 kDa. Under the same conditions the apparent molecular mass of PLA and PLD are 49.1 kDa and 52.3 kDa respectively.

Isoelectric focussing is performed as described in Example 6.4. The isoelectric point of PLB is 6.0 using a pH gradient between pH 3 and 7. The sample is approximately applied at a position corresponding to a pl of 5.0.

The purified enzyme PLB is also reactive with polyclonal antibodies prepared against PLI and PLII as tested by Western blot analysis. PLD, A and B can easily be discriminated this way by their apparent molecular mass values.

### Example 8.4: Kinetic properties of PLB

The enzyme purified according to Example 8.2 is characterized kinetically. The enzyme catalyses a pectin lyase reaction and is active over a wide pH range (pH 5-9.5) as tested in Mc Ilvaine buffer (µ = 0.5) of different pH values (van Houdenhoven, ref. 18). The pH optimum is broad (pH 7.5-8.5). At pH 6.0, the activity is approximately 55 %; at pH 9.5 this is still 85 % of the activity at pH 8.0. Both highly esterified pectin (d.e. 94 %) as well as pectins with lower degree of esterification like Brown Ribbon apple pectin (d.e. 72.8 %; Obipektin AG Bischoffzell) can be used as substrate. The highest activity is obtained with highly esterified pectin, however. The enzyme does not react with polygalacturonate.

The pectin lyase reaction catalyzed by PLB can be inhibited by adding EDTA to the reaction mixture (3 mM final concentration is used); the enzyme is reactivated by the addition of excess of Ca²⁺-ions. Thus, pelB codes for a Ca²⁺-depenent pectin lyase. At 25°C the enzyme has an approximate turn-over number of 6500 and a Km value of 2.5 mM when using highly esterified pectin as a substrate. These values are determined according to the procedure of van Houdenhoven (ref. 18) using a Mc Ilvaine buffer of pH 8.0 (µ = 0.5).

### Example 9: Expression and secretion of foreign genes under the control of the pelA promoter

The 3.9 kb HindIII fragment of plasmid pGW820 is subcloned into the HindIII site of pBR322. The plasmid DNA of ampicillin-resistant transformants is analysed by HindIII and Sall restriction digests. One clone which contains the pelA insert in clockwise orientation is referred to as pGW822. The inducible promoter of pelA is used to express foreign genes in A. niger. The complete pelA gene is present on plasmid pGW822. The promoter and the pelA signal sequence are on a 1 kb BamHI-PstI fragment. The PstI cleavage site at nucleotide position 1420 (Fig. 10) coincides with the 3' end of the signal sequence and can be used for the in frame fusion to the coding sequence of a foreign protein. The transcription termination signals of pelA are on a 1.3 kb SalI-HindIII fragment.

### Example 9.1 :Subcloning of the pelA gene in vetor pUC19

The 3.3 kb BamHI-HindIII fragment of plasmid pGW822 contains the pelA gene. The fragment is cloned in the vector pUC19 (Pharmacia) cut with BamHI and HindIII. The purified fragments are ligated. An aliquot of the ligation mixture is used to transform Ca²⁺ treated, competent JM109 cells. Successful cloning of the 3.3 kb BamHI-HindIII fragment into pUC19 is selected for on ampicillin plates in the presence of X-Gal and IPTG (T. Maniatis et al. in "Molecular Cloning, A Laboratory Manual", Cold Spring Harbor Laboratory, 1982, p. 52). White, ampicillin-resistant transformants are picked. Plasmid DNA of these colonies is analysed by BamHI/HindIII double digestion. One transformant with a correct insert is referred to as pUC19/pelA. An analogous construction with pUC18 results in pUC18/pelA.

### Example 9.2: Expression of human hybrid interferon BDBB under the control of the pelA promoter

### 9.2.1. :Construction of plasmid pUC19/pelA-IFN AM119 (see Fig.14)

Plasmid pUC19/pelA is digested with Sall. The sticky ends of the linear fragment are filled in a reaction with Klenow DNA polymerase I (BRL) in the presence of 0.1 mM each of dCTP, dGTP, dATP, dTTP, 60 mM Tris·Hcl pH 7.5, 10 mM MgCl₂ for 30 min at room temperature. The reaction is stopped by the addition of EDTA to a final concentration of 12.5 mM. The DNA is ethanol precipitated. The linear DNA fragment is digested with Pstl. After phenol/chloroform extraction the DNA is precipitated with ethanol.

An oligodesoxynucleotide linker of the formula

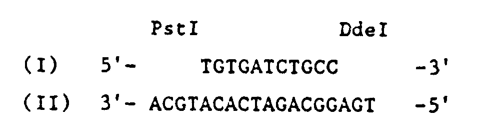

is ligated to the Pstl site of the linearized plasmid. The linker fills the 3' recessed end of the pstl site which coincides with the end of the pelA signal sequence and establishes the inframe fusion to the coding sequence of interferon BDBB. (I) represents the 5' terminal nucleotide sequence of the interferon BDBB gene up to the Ddel restriction site.

200 pmoles each of oligonucleotides (I) and (II) are phosphorylated and annealed. The Pstl cut pUC19/pelA plasmid and a 100-fold molar excess of the double-stranded linker DNA are ligated in 60 mM Tris·Hcl pH 7.5, 10 mM MgCl₂, 1 mM ATP, 5 mM DTT and 400 units of T4 DNA ligase for 16 hours at 15°C. The DNA ligase is inactivated for 10 min at 85°C. The excess linkers are removed by precipitation in the presence of 10 mM EDTA, 300 mM sodium acetate pH 6.0 and 0.54 volumes of isopropanol.

The large, 5 kb fragment is isolated on a preparative 0.6 % agarose gel. The fragment comprises the pelA promoter and signal sequence (BamHI-[PstI]/linker) and the pelA terminator ([Sall]blunt-HindIII) in vector pUC19.

Plasmid pJDB207/PH05-IFN AM119 (EP 205404) contains the gene for hybrid α-interferon BDBB under the control of the regulated promoter of the yeast acid phosphatase (PH05). The plasmid DNA is digested with BamHI and HindIII, The 1.3 kb BamHI-HindIII fragment is isolated, which contains the PH05 promoter, the coding sequence of IFN BDBB and the PH05 transcription termination sequences. The fragment is purified by DE52 chromatography and ethanol precipitation and is further digested with Taql. The sticky ends of the Taql restriction fragments are filled in a reaction with Klenow DNA Polymerase I (BRL) in the presence of 0.1 mM each of dCTP and dGTP, 60 mM Tris·HCl pH 7.5, 10 mM MgCl₂ for 30 min at room temperature. The reaction is stopped by the addition of EDTA to a final concentration of 12.5 mM. The DNA fragments are ethanol precipitated and further digested with Ddel. The DNA fragments are separated on a preparative 0.8 % agarose gel. The 549 bp Ddel-[Taql]blunt fragment is electroeluted from the gel, purified by DE52 ion exchange chromatography and ethanol precipitation. The DNA fragment is resuspended in H₂O at a concentration of about 0.1 pmoles/µl.

0.2 pmoles of the 549 bp Ddel-[Taql]blunt fragment and 0.1 pmoles of the 5 kb vector fragment are ligated in 10 µl of 60 mM Tris·HCl pH 7.5, 10 mM MgCl₂, 3.5 mM ATP, 5 mM DTT and 200 units of T4 DNA ligase (Biolabs) for 16 hours at 15°C. An 1 µl aliquot of the ligation mixture is used to transform Ca²⁺ treated, competent HB101 cells.

Plasmid DNA is prepared from 12 ampicillin-resistant transformed colonies and analysed by EcoRI, Pvull, Clal and EcoRI/HindIII digestion. A clone with the expected restriction pattern is selected. The correct in frame fusion of the pelA signal sequence and the mature coding sequence of interferon BDBB is verified by DNA sequencing. The plasmid DNA of the selected clone is referred to as pUC19/pelA-IFN AM119.

An analogous construction with pUC18/pelA results in plasmid pUC18/pelA-IFN AM119.

### Example 9.2.2 :Cotransformation of Aspergillus niger mutant An8 with pCG59D7 and pUC19/pelA-IFN AM119

The uridine anxotrophic mutant An8 (≙ DSM 3917, disclosed in EP 88 101 397.3) is transformed with plasmid pCG59D7 to yield uridine prototrophs. Together with the transforming plasmid the non-selective plasmid pUC19/pelA-IFN AM119 is added to give random cointegrants upon cotransformation.

Conidial spores of A. niger An8 are grown for 4 days at 28°C in complete medium until fully sporulated. 2·10⁸ conidiospora are used to inoculate 200 ml minimal medium supplemented with 1 g/l arginine and uridine.

After 20 hours growth at 28°C and 180 rpm. the mycelium is harvested by filtration through Miracloth, washed twice with 10 ml 0.8 M KCI, 50 mM CaCl₂ and resuspended in 20 ml 0.8 M KCI, 50 mM CaCl₂, 0.5 mg/ml Novozym 234 (Novo Industries). The mixture is incubated in a shaking waterbath (30°C, 50 rpm.) until maximum protoplast release can be detected microscopically (90-120 min). The protoplast suspension is filtrated through a glass wool plug in a funnel to remove mycelial debris. The protoplasts are pelleted by mild centrifugation (10 min, 2000 r.p.m.) at room temperature and washed twice with 10 ml 0.8 M KCI, 50 mM CaCl₂. The protoplasts are finally resuspended in 200-500 µl 0.8 M KCI, 50 mM CaCl₂ to give a concentration of 1 x 10⁸/ml.

For transformation a 200 µl aliquot of the protoplast dispersion is incubated with 5 µg of pCG59D7 and 10 µg pUC19/pelA-IFN AM119 DNA, 50 µl PCT (10 mM Tris·HCl pH 7.5, 50 mM CaCl₂, 25 % PEG 6000). The incubation mixture is kept on ice for 20 min, another 2 ml of PCT are added and the mixture incubated for further 5 min at room temperature. 4 ml 0.8 M KCI, 50 mM CaCl₂ are added and 1 ml aliquots of the final transformation solution are mixed with lignified minimal agar medium (Minimal medium + 1 g/l arginine + 10 g/l Bacto-Agar (Difco)), stabilised with 0.8 M KCI. The mixtures are immediately poured on agar plates of the same medium and incubated at 28 °C.

After 2-3 days of growth at 28°C, stable transformants appear as vigorously growing and sporulating colonies on a background growth of many hundred small presumably abortive transformants.

### Example 9.2.3. :Expression of the Hybrid-Interferon BDBB gene under the control of the pelA promoter

37 transformants. of the cotransformation experiment (Example 9.2.2.) are picked and analysed for interferon expression. Interferon activity is determined according to the procedure of Armstrong. (J.A. Armstrong, Appl. Microbiol. 21, 732 (1971)) using human CCL-23 cells and vesicular stomatitis virus (VSV) as the challenge virus.

Conidial spores from transformants are individually precultured into 50 ml of a preculture medium (Pectin Slow Set L (Unipectin, SA, Redon, France) 3 g/l, NH₄Cl 2 g/l, KH₂PO₄ 0.5 g/l, NaCI 0.5 g/l, Mg₂SO₄ x 7 H₂O 0.5 g/l, Ca₂SO₄ x 2 H₂O 0.5 g/l, pH 7.0). The preculture is incubated for 72 hours at 250 rpm and 28°C. 10 % of the preculture is used to inoculate 50 ml of main culture medium (Soybean fluor 20 g/l, pectin Slow Set 5 g/l). The culture is grown up for 72-96 hours at 250 rpm and 28 °C.

At various times (every 20 hours) samples are taken, the cells are pelleted by centrifugation and broken by ultrasonic desintegration. Supernatant and cell extracts are both tested for interferon activity as described (supra). The bulk of the interferon activity is found secreted into the medium.

### Example 10: Construction of plasmid M13(+)KS/pelAΔss-IFN AM119

The 2.8 kb expression cassette of plasmid M13(+)KS/pelAΔss-IFN AM119 comprises the inducible pelA promoter, the coding sequence of hybrid interferon BDBB and the pelA transcriptional terminator on the Bluescript M13(+)KS vector. Hybrid interferon BDBB will be expressed and located in the host cell. Plasmid M13(+)KS/pelAΔss-IFN AM119 is derived from pUC18/pelA-IFN AM119 (see Example 9.2.1). The pelA signal sequence (ss) is looped out by site-directed mutagenesis (see Fig. 15). Hybrid interferon expressed from the construct without signal sequence is expected to be located in the cytosol.

### Example 10.1: Subcloning of the pelA-IFN AM119 cassette into the Bluescript M13(+)KS vector

Plasmid pUC18/pelA-IFN AM119 (see Example 9.2.1.) is digested with BamHI and HindIII. The 2.8 kb BamHI-HindIII fragment contains the pelA promoter, signal sequence, the IFN BDBB coding sequence and the pelA terminator. The BamHI-HindIII fragment is isolated and ligated to the Bluescript M13(+)KS (Stratagene) vector, cut with BamHI and HindIII. An aliquot of the ligation mixture is used to transform Ca²⁺ treated, competent JM109 cells. Successfull cloning of the 2.8 kb BamHI-HindIII fragment into M13(+)KS is selected for on ampicillin plates in the presence of X-Gal and IPTG. 12 white, ampicillin resistant colonies are picked. Plasmid DNA of these colonies is analysed by BamHI/BgIII double digestion. One transformant with the correct insert is referred to as M13(+)KS/pelA-IFN AM119.

### Example 10.2: Recovery of single stranded DNA from cells containing the Bluescript M13(+)KS plasmid

Plasmid M13(+)KS/pelA-IFN AM119 is used to transform competent E. Coli strain CJ236 (dut-1,ung-1, thi-1, relA-1;pCJ105(Cm^{r}); BIO-RAD Muta-Gene M13 in vitro mutagenesis kit). This strain allows incorporation of uracil into the DNA. CJ236 is grown in 10 ml of LB medium containing 100 mg/l of ampicillin. At an OD₆₀₀ of 0.5 the cells are superinfected with phage M13K07 (Mead et al. ref. 29) at a multiplicity of infection of 50. After one hour at 37 °C kanamycin (50 mg/l) is added and the culture is incubated at 37°C on a shaker for 5 to 6 hours. The non-coding strand with respect to the pelA-IFN AM119 insert of plasmid M13(+)KS/pelA-IFN AM119 is synthesized, packaged and released to the medium. Single stranded DNA is prepared from the culture supernatant according to Kunkel et al. (ref. 30).

### Example 10.3: Site-directed oligonucleotide mutagenesis on the single stranded DNA template

Site-directed deletion mutagenesis is performed on the non-coding single stranded pelA-IFN AM119 template to loop out the pelA signal sequence (Fig. 15).

The mutagenic primer comprises part of the pelA promoter sequence including the ATG (nucleotide position 1343 to 1363 in Fig. 10) and 21 nucleotides coding for amino acids 1 to 7 of mature IFN BDBB.

For the mutagenesis 200 pmoles of the mutagenic primer are phosphorylated in 20 µl of 50 mM Tris.HCI pH 7.5, 10 mM MgCl₂, 5 mM DTT and 0.5 mM ATP using 8 units of T₄ polynucleotide kinase (Boehringer). After one hour at 37 °C the reaction is stopped by heating to 65° for 10 min.

0.2 pmoles of single stranded template is incubated with 10 pmoles of phosphorylated mutagenic oligodeoxyribonucleotide primer and 10 pmoles of universal M13 sequencing primer in 30 µl of 20 mM Tris.HCl pH 7.5, 10 mM MgCl₂, 50 mM NaCI, 1 mM DTT at 80°C for 5 min. The solution is allowed to cool slowly to room temperature over a period of 30 min. To the annealed mixture 10 µl of enzyme-dNTP (dATP,dGTP,dCTP,dTTP) solution are added containing 1 µl of buffer [0.2 M Tris.HCI pH 7.5, 0.1 M MgCl₂], 5 ul of 2 mM dNTP mixture, 0.5 µl of 20 mM ATP, 1 µl of 0.2 M DTT, 0.5 µl of T₄ DNA ligase (Biolabs, 400 U/µl) and 1.2 µl of Klenow DNA polymerase (BRL, 5 U/µl). The mixture is incubated at 15°C for 16 hours. The reaction is stopped by incubating at 65°C for 10 min.

1 µl and 3 µl of the ligation mixture are used to transform 0.2 ml of competent cells of the repair-minus strain E. coli MV1190 [△(lac-proAB), thi, supE, △(sr1-recA)306::Tn10(tet^{r}) (F':traD36, proAB, lac I^{q}Z△M15)]. Strain MV1190 is described in the manual for the BIO-RAD MUTA-GENE M13 in vitro mutagenesis kit. 12 ampicillin resistant colonies are picked. Plasmid DNA is prepared and analysed by Scal digestion. Successful loop-out of the pelA signal sequence removes a Scal site. Correct plasmids are linearized at the Scal site in the Bluescript vector. One plasmid is further analysed. The correct junction between the pelA promoter and the coding sequence for hybrid IFN BDBB with the ATG included is confirmed by DNA sequencing. One correct construct is referred to as M13(+)KS/pelAΔss-IFN AM119.

### Example 10.4: Cotransformation of A. niger and expression of hybrid interferon

Plasmids M13(+)KS/pelAΔss-IFN AM119 and pCG59D7 are used to cotransform A. niger mutant An8 according to Example 8.2.2. Transformants are cultivated as described in Example 8.2.3. At various times (every 20 hours) samples are taken, the cells are pelleted by centrifugation and broken by ultrasonic desintegration. The cell extracts are tested for interferon activity (supra). The bulk of the interferon activity is found intracellularly.

### Deposition of Microorganisms

The following microorganisms were deposited under the Budapest Treaty with Deutsche Sammlung von Mikroorganismen, Grisebachstr. 8, D-3400 Göttingen:

| Microorganisms | Dep. Nr. | Date of Dep. |
|---|---|---|
| Escherichia coli HB 101/pGW 820 | DSM 4388 | February 1, 1988 |
| Escherichia coli HB 101/pGW 830 | DSM 4389 | February 1, 1988 |
| Escherichia coli HB 101/pGW 850 | DSM 4390 | February 1, 1988 |
| Escherichia coli HB 101/pGW 860 | DSM 4391 | February 1, 1988 |
| Escherichia coli HB 101/pGW 880 | DSM 4392 | February 1, 1988 |

### References

1. Yelton, M.M., Hamer, J.E. and Timberlake, W.E.(1984 Proc. Natl. Acad. Sci. USA 81, 1470-1474.
2. Frischauf, A.M., Lehrach, H., Poustra, A. and Murray, N. (1983) J.Mol. Biol. 170, 827-842.
3. Karn, J., Brenner, S., Barneff, L. and Cesareni, G. (1980) Proc. Natl. Acad. Sci. USA 77, 5172-5176.
4. Benton, W.D. and Davis, R.W. (1977) Science 196, 180-182.
5. Maniatis, T., Fritsch, E.F. and Sambrook, J. (1982) in: Molecular cloning, a laboratory manual, Cold Spring Harbor Harbor, New York.
6. BRL. M13 cloning/dideoxy sequencing instruction manual.
7. Dente, L., Cesareni, G. and Cortese, R. (1983) Nucl. Acids Res. 11, 1645-1655.
8. Dente, L., Sollazzo, M., Baldari, C., G. Cesareni and R. Cortese. in: DNA Cloning vol. 1. a practical approach ed. D.M. Glover, IRL Press, Oxford 1985.
9. Russell, M., Kidd, S. and Kelley, M.R. (1986) Gene 45, 333-338.
10. Caruther, M.H. Chemical and Enzymatic Synthesis of Gene Fragments: a laboratory manual, Verlag Chemie (1982).
11. Ballance, D.J. (1986) Yeast 2, 229-236.
12. Goosen, T., Bloemheuvel, G., Gysler, Ch., de Bier, D,A., van den Broek, H.W.J. and Swart, K. (1987) Current Genetics 11, 499-503.
13. Boeke, J.D., Lacroute, F. and Fink, G.R. (1984) Mol. Gen. Genet. 197, 345-346.
14. Slater, R.J. (1984) in: Methods in Molecular Biology vol. 2 Ed. J.M. Walker, The Humana Press Inc.
14a.Hinnen A., Hicks J.B. & Fink G.R. (1978) Proc. Natl. Acad. Sci.USA 75, 1929-1933.
15. Laemmli, U.K. (1970) Nature 227, 681-682.
16. Towbin, H., Staehelin, T. and Gordon, J. (1979) Proc. Natl. Acad. Sci. USA 76, 4350-4354.
17. Ried, J.L. and Collmer, A. (1985) Applied and Environm. Microbiol. 50, 615-622.
18. van Houdenhoven, F.E.A. (1975) Ph.D. Thesis Agricultural University Wageningen, The Netherlands.
19. Hunkapiller, M.W. (1985) PTH Amino Acid Analysis, User Bulletin no. 14 Applied Biosystems.
20. Zissler, J. et al. (1971) in: The Bacteriophage Lambda, Cold Spring Harbor Labs, New York, A.D. Hersley editor.
21. Norrander, J., Kempe, T. and Messing, J. (1983) Gene 26, 101-106.
22. F.E.A. von Houdenhoven. Ph.D. Thesius Agricultural University, Wageningen in Communications Agricultural University Wageningen, The Netherlands 75-13 (1975).
23. Birnboim H.C. & Doly J. (1979). Nucleic Acids Res 7, 1513-1523.
24. Boel E., Hansen M.T., Hjort I., Hoegh I., Fiil N.P. (1984). EMBO J. 3, 1581-1585.
25. Mount S.M. (1982). Nucleic Acids Res.10. 459-472.
26. Ballance D.J. and Turner G. (1985), Gene 36, 321-331.
27. Hermersdörfer H, Leuchtenberger A, Wardsack Ch and Ruttloff H (1907) Influence of culture conditions on mycelial structure and polygalacturonase synthesis of A. niger. J. Basic Microbiol. 27, 309-315.
28. Henikoff, S. (1984) Gerne 28, 351-359.
29. Mead et al., (1986) Protein Engeneering 1, 67.
30. Kunkel et al., (1985) Proc. Natl. Acad. Sci. USA 82, 485.

## Claims (Claims for the following Contracting State(s): AT BE CH DE FR GB GR IT LI LU NL SE)

1. A recombinant DNA molecule comprising a DNA sequence coding for pectin lyase pelA having the amino acid sequence set forth in fig. 10; pectin lyase pelB having the amino acid sequence set forth in fig. 11; pectin lyase pelC obtainable by expression of pelC contained in pGW850 shown in fig. 3, deposited under accession no. DSM 4390 ; pelE obtainable by expression of pelE contained in pGW880 shown in fig. 5, deposited under accession no. DSM 4392 or pelF obtainable by expression of pelF contained in pGW860 shown in fig. 6, deposited under accession no. DSM 4391 or a derivative thereof, wherein a derivative designates a larger derivative including flanking sequences or DNA sequences which are degenerated in accordance with the genetic code.

2. A recombinant DNA molecule according to claim 1 comprising the promoter which is located in the DNA region before the structural gene and comprises up to 2000 nucleotides; the signal sequence which is located in the DNA region between the end of the promoter and the beginning of the sequence coding for the mature protein; the structural gene; or the terminator, which starts with the stop codon, of anyone of the pectin lyase genes, or any combination of these fragments.

3. A recombinant DNA molecule according to claim 1, comprising a DNA sequence of the formula I shown in figure 10 or a derivative thereof as defined in claim 1.

4. A recombinant DNA molecule according to claim 1, comprising a DNA sequence of the formula II shown in figure 11 or a derivative thereof as defined in claim 1.

5. A recombinant DNA molecule according to claim 1, which is pGW820 deposited under accession no. DSM 4388.

6. A recombinant DNA molecule according to claim 1, which is pGW830 deposited under accession no. DSM 4389.

7. A recombinant DNA molecule according to claim 1, which is pGW850 deposited under accession no. DSM 4390.

8. A recombinant DNA molecule according to claim 1, which is pGW880 deposited under accession no. DSM 4392.

9. A recombinant DNA molecule according to claim 1, which is pGW860 deposited under accession no. DSM 4391.

10. A recombinant DNA molecule according to claim 1, which comprises a fragment extending between two restriction sites of plasmid pGW820 deposited under accession no. DSM 4388, pGW830 deposited under accession no. DSM 4389, pGW850 deposited under accession no. DSM 4390, pGW880 deposited under accession no. DSM 4392 or pGW860 deposited under accession no. DSM 4391 and retaining a pectin lyase promoter, signal, structural or terminator functions or a combination of such fragments.

11. A recombinant DNA molecule according to claim 1, comprising the promoter sequence of pelA.

12. A recombinant DNA molecule according to claim 1, comprising the promoter sequence of pelA, pelB. pelC. pelE or pelF.

13. A recombinant DNA molecule according to claim 1, comprising the signal sequence of pelA, pelB, pelC, pelE or pelF.

14. A recombinant DNA molecule according to claim 1, comprising the PLI structural gene of pelA, pelB, pelC, pelE or pelF.

15. A recombinant DNA molecule according to claim 1, comprising the PLI structural gene of pelA, pelB, pelC, pelE or pelF without the introns.

16. A recombinant DNA molecule according to claim 1, comprising the terminator of pelA, pelB, pelC, pelE or pelF.

17. A recombinant DNA molecule according to claim 1, which is a recombinant hybrid vector comprising a structural gene heterologous to Aspergillus niger.

18. A recombinant DNA molecule according to claim 1, which is a hybrid vector comprising the heterologous structural gene coding for the hybrid interferon BDBB.

19. A recombinant DNA molecule according to claim 1, which is the recombinant hybrid vector pUC19/pelA-IFN AM119 shown in figure 14.

20. A recombinant DNA molecule according to claim 1, which is the recombinant hybrid vector M13(+)KS/pelAΔss-IFN AM119, consisting of the inducible pelA promoter, the coding sequence of hybrid interferon BDBB and the pelA transcriptional terminator on the Bluescript M13(+)KS vector.

21. A recombinant DNA molecule according to claim 1, which is the hybrid vector M13(+)KS/pelA-IFN AM119 obtained by digesting pUC19/pelA-IFN AM119 as shown in figure 14 with BamHI and HindIII and ligating the isolated fragment to a Bluescript M13(+)KS vector, cut with BamHI and HindIII.

22. Process for the preparation of a recombinant DNA molecule according to claim 1, comprising culturing a host transformed with a DNA molecule containing a DNA sequence coding for the pectin lyase PLA, PLB, PLC, PLE or PLF expression system or a derivative thereof, and isolating the desired recombinant DNA molecule or the derivative thereof, or preparing it by in vitro synthesis.

23. A transformed host containing a recombinant DNA molecule according to claim 1.

24. A transformed host according to claim 24 which is Escherichia coli HB 101/pGW820 deposited under accession no. DSM 4388.

25. A transformed host according to claim 24 which is Escherichia coli HB 101/pGW830 deposited under accession no. DSM 4389.

26. A transformed host according to claim 24 which is Escherichia coli HB 101/pGW850 deposited under accession no. DSM 4390.

27. A transformed host according to claim 24 which is Escherichia coli HB 101/pGW860 deposited under accession no. DSM 4391.

28. A transformed host according to claim 24 which is Escherichia coli HB 101/pGW880 deposited under accession no. DSM 4392.

29. A transformed host according to claim 24 which is Aspergillus niger An8 deposited under accession no. DSM 3917 transformed with a recombinant DNA molecule according to claim 1 and the selection marker plasmid pCG59D7 deposited under accession no. DSM 3968.

30. A transformed host according to claim 24 which is Aspergillus niger An8 deposited under accession no. DSM 3917 transformed with pUC19/pelA-IFN AM119 shown in figure 14 and the selection marker plasmid pCG59D7 deposited under accession no. DSM 3968.

31. A transformed host according to claim 24 which is Aspergillus niger An8 deposited under accession no. DSM 3917 transformed with M13(+)KS/pelA△ss-IFN AM119 according to claim 21 and the selection marker plasmid pCG59D7 deposited under accession no. DSM 3968.

32. A transformed host according to claim 24 which is Aspergillus niger An8 deposited under accession no. DSM 3917 transformed with M13(+)KS/pelA-IFN AM119 according to claim 22 and the selection marker plasmid pCG59D7 deposited under accession no. DSM 3968.

33. Method for the preparation of a transformed host according to claim 24, comprising treatment of such host under transforming conditions with a recombinant DNA molecule according to claim 1, optionally together with a selection marker gene and selecting the transformants.

34. Method according to claim 34, wherein Aspergillus niger An8 is cotransformed with a recombinant DNA molecule according to claim 1 and the selection marker plasmid pCG59D7 deposited under accession no. DSM 3968.

35. Method for the preparation of a polypeptide, **characterized in that** a hybrid vector according to claim 1 is expressed in a suitable host.

36. Method for the preparation of a polypeptide, **characterized in that** a hybrid vector according to claim 18 is expressed in a suitable host.

37. Method according to claim 36 for the overproduction of the pectin lyases PLA, PLB, PLC, PLE or PLF in Aspergillus species comprising cultivating an Aspergillus host transformed with an expression hybrid vector for the expression of said pectin lyases.

38. Method according to claim 37 for the production of the hybrid interferon BDBB in Aspergillus species comprising cultivating an Aspergillus host transformed with an expression hybrid vector for the expression of said hybrid interferon.

39. Pectin lyases PLA, PLB, PLC, PLE or PLF in pure form, obtainable by transforming a host which is not capable of expressing any pectin lyase with a recombinant DNA molecule according to claim 1 and isolating said pectin lyases.

## Claims (Claims for the following Contracting State(s): ES)

1. A process for the preparation of a recombinant DNA molecule comprising a DNA sequence coding for pectin lyase pelA having the amino acid sequence set forth in fig. 10; pectin lyase pelB having the amino acid sequence set forth in fig. 11; pectin lyase pelC obtainable by expression of pelC contained in pGW850 shown in fig. 3, deposited under accession no. DSM 4390; pelE obtainable by expression of pelE contained in pGW880 shown in fig. 5, deposited under accession no. DSM4392 or pelF obtainable by expression of pelF contained in pGW860 shown in fig. 6, deposited under accession no. DSM4391 or a derivative thereof, wherein a derivative designates a larger derivative including flanking sequences or DNA sequences which are degenerated in accordance with the genetic code.

2. A process according to claim 1 for the preparation of a recombinant DNA molecule according to claim 1 comprising the promoter which is located in the DNA region before the structural gene and comprises up to 2000 nucleotides; the signal sequence which is located in the DNA region between the end of the promoter and the beginning of the sequence coding for the mature protein; the structural gene; or the terminator, which starts with the stop codon, of anyone of the pectin lyase genes, or any combination of these fragments.

3. A process according to claim 1 for the preparation of a recombinant DNA molecule comprising a DNA sequence of the formula I shown in figure 10 or a derivative thereof as defined in claim 1.

4. A process according to claim 1 for the preparation of a recombinant DNA molecule comprising a DNA sequence of the formula II shown in figure 11 or a derivative thereof as defined in claim 1.

5. A process according to claim 1 for the preparation of a recombinant DNA molecule which is pGW820 deposited under accession no. DSM4388.

6. A process according to claim 1 for the preparation of a recombinant DNA molecule which is pGW4389 deposited under accession no. DSM4389.

7. A process according to claim 1 for the preparation of a recombinant DNA molecule which is pGW 850 deposited under accession no. DSM4390.

8. A process according to claim 1 for the preparation of a recombinant DNA molecule which is pGW880 deposited under accession no. DSM 4392.

9. A process according to claim 1 for the preparation of a recombinant DNA molecule which is pGW860 deposited under accession no. DSM4391

10. A process for the preparation of a recombinant DNA molecule which comprises a fragment extending between two restriction sites of plasmid pGW820 deposited under accession no. DSM4388, pGW830 deposited under accession no. DSM4389, pGW850 deposited under accession no. DSM4390, pGW880 deposited under accession no. DSM4392 of pGW860 deposited under accession no. DSM4391 and retaining a pectin lyase promoter, signal, structural or terminator functions or a combination of such fragments.

11. A process for the preparation of a recombinant DNA molecule, comprising the promoter sequence of pelA.

12. A process for the preparation of a recombinant DNA molecule, comprising the promoter sequence of pelA, pelB, pelC, pelE or pelF.

13. A process for the preparation of a recombinant DNA molecule, comprising the signal sequence of pelA, pelB, pelC, pelE or pelF.

14. A process for the preparation of a recombinant DNA molecule, comprising the PLI structural gene of pelA, pelB, pelC, pelE or pelF.

15. A process for the preparation of a recombinant DNA molecule, comprising the PLI structural gene of pelA, pelB, pelC, pelE or pelF without the introns.

16. A process for the preparation of a recombinant DNA molecule, comprising the terminator of pelA, pelB, pelC, pelE or pelF.

17. A process for the preparation of a recombinant DNA molecule, which is a recombinant hybrid vector comprising a structural gene heterologous to Aspergillus niger.

18. A process for the preparation of a recombinant DNA molecule, which is a hybrid vector comprising the heterologous structural gene coding for the hybrid interferon BDBB.

19. A process for the preparation of a recombinant DNA molecule, which is the recombinant hybrid vector pUC19/pelA-IFN AM119 shown in fig. 14.

20. A process for the preparation of a recombinant DNA molecule, which is the recombinant hybrid vector M13(+)KS/pelAΔss-IFN AM119, consisting of the inducible pelA promoter, the coding sequence of hybrid interferon BDBB and the pelA transcriptional terminator on the Bluescript M13(+)KS vector.

21. A process for the preparation of a recombinant DNA molecule, which is the hybrid vector M13(+)KS/pelA-IFN AM119 obtained by digesting pUC19/pelA-IFN AM119 as shown in figure 14 with BamHI and Hindlll and ligating the isolated fragment to a Bluescript M13(+)KS vector, cut with BamHI and HindIII.

22. A process for the preparation of a recombinant DNA molecule according to claim 1, comprising culturing a host transformed with a DNA molecule containing a DNA sequence coding for the pectin lyase PLA, PLB, PLC, PLE or PLF expression system or derivative thereof, and isolating the desired recombinant DNA molecule or the derivative thereof, or preparing it by in vitro synthesis.

23. A process according to claim 22 for the preparation of a transformed host containing a recombinant DNA molecule according to claim 1.

24. A process according to claim 22 for the preparation of a transformed host which is Escherichia coli HB 101/pGW820 deposited under accession no. DSM4388.

25. A process according to claim 22 for the preparation of a transformed host which is Escherichia coli HB 101/pGW830 deposited under accession no. DSM4389.

26. A process according to claim 22 for the preparation of a transformed host which is Escherichia coli HB 101/pGW850 deposited under accession no. DSM4390.

27. A process according to claim 22 for the preparation of a transformed host which is Escherichia coli HB 101/pGW860 deposited under accession no. DSM4391.

28. A process according to claim 22 for the preparation of a transformed host which is Escherichia coli HB 101/pGW880 deposited under accession no. DSM4392.

29. A process according to claim 22 for the preparation of a transformed host which is Aspergillus niger An8 deposited under accession no. DSM3917 transformed with a recombinant DNA molecule according to claim 1 and the selection marker plasmid pCG59D7 deposited under accession no. DSM3968.

30. A process according to claim 22 for the preparation of a transformed host, which is Aspergillus niger An8 deposited under accession no. DSM3917 transformed with pUC19/pelA-IFN AM119 shown in figure 14 and the selection marker plasmid pCG59D7 deposited under accession no. DSM3968.

31. A process according to claim 22 for the preparation of a transformed host, which is Aspergillus niger An 8 deposited under accession no. DSM3917 transformed with M13(+)KS/pelAAss-IFN AM119 according to claim 21 and the selection marker plasmid pCG59D7 deposited under accession no. DSM3968.

32. A process according to claim 22 for the preparation of a transformed host, which is Aspergillus niger An8 deposited under accession no. DSM3917 transformed with M13(+)KS/pelA-IFN AM119 according to claim 22 and the selection marker plasmid pCG59D7 deposited under accession no. DSM3968.

33. Method for the preparation of transformed host according to claim 22, comprising treatment of such host under transforming conditions with a recombinant DNA molecule according to claim1, optionally together with a selection marker gene and selecting the transformants.

34. Method according to claim 33, wherein Aspergillus niger An8 is co transformed with a recombinant DNA molecule according to claim 1 and the selection marker plasmid pCG59D7 deposited under accession no. DSM3968.

35. Method for the preparation of a polypeptide, **characterized in that** a hybrid vector according to claim 1 is expressed in a suitable host.

36. Method for the preparation of a polypeptide, **characterized in that** a hybrid vector according to claim 18 is expressed in a suitable host.

37. Method according to claim 36 for the overproduction of the pectin lyases PLA, PLB, PLC, PLE or PLF in Aspergillus species comprising cultivating an Aspergillus host transformed with an expression hybrid vector for the expression of said pectin lyases.

38. Method according to claim 37 for the production of the hybrid interferon BDBB in Aspergillus species comprising cultivating an Aspergillus host transformed with an expression of said hybrid interferon.

39. Process for the production of pectin lyases PLA, PLB, PLC, PLE or PLF in pure form, obtainable by transforming a host which is not capable of expressing any pectin lyase with a recombinant DNA molecule according to claim 1 and isolating said pectin lyases.

## Patentansprüche (Patentansprüche für folgende(n) Vertragsstaat(en): AT, BE, CH, DE, FR, GB, GR, IT, LI, LU, NL, SE)

1. Rekombinantes DNA-Molekül, umfassend eine DNA-Sequenz, die Pectinlyase pelA mit der in Fig. 10 dargestellten Aminosäuresequenz, Pectinlyase pelB mit der in Fig. 11 dargestellten Aminosäuresequenz, Pectinlyase pelC, erhältlich durch Expression von pelC, enthalten in pGW850, das in Fig. 3 gezeigt ist und unter der Hinterlegungsnummer DSM 4390 hinterlegt ist, pelE, erhältlich durch Expression von pelE, enthalten in pGW880, das in Fig. 5 gezeigt ist und unter der Hinterlegungsnummer DSM 4392 hinterlegt ist, oder pelF, erhältlich durch Expression von pelF, enthalten in pGW860, das in Fig. 6 gezeigt ist und unter der Hinterlegungsnummer DSM 4391 hinterlegt ist, codiert, oder ein Derivat davon, wobei ein Derivat ein größeres Derivat einschließlich flankierender Sequenzen oder DNA-Sequenzen, die gemäß dem genetischen Code degeneriert sind, bezeichnet.

2. Rekombinantes DNA-Molekül nach Anspruch 1, umfassend den Promoter, der in der DNA-Region vor dem Strukturgen lokalisiert ist und bis zu 2000 Nucleotide umfaest, die Signalsequenz, die in der DNA-Region zwischen dem Ende des Promoters und dem Beginn der Sequenz, die das reife Protein codiert, lokalisiert ist, das Strukturgen oder den Terminator, der mit dem Stoppcodon beginnt, eines der Pectinlyasegene, oder eine beliebige Kombination dieser Fragmente.

3. Rekombinantes DNA-Molekül nach Anspruch 1 umfassend eine DNA-Sequenz der in Figur 10 gezeigten Formel I oder ein Derivat davon wie in Anspruch 1 definiert.

4. Rekombinantes DNA-Molekül nach Anspruch 1, umfassend eine DNA-Sequenz der in Figur 11 gezeigten Formel II oder ein Derivat davon wie in Anspruch 1 definiert.

5. Rekombinantes DNA-Molekül nach Anspruch 1, das pGW820, hinterlegt unter der Hinterlegungsnummer DSM 4388, ist.

6. Rekombinantes DNA-Molekül nach Anspruch 1, das pGW4389, hinterlegt unter der Hinterlegungsnummer DSM 4389, ist.

7. Rekombinantes DNA-Molekül nach Anspruch 1, das pGW850, hinterlegt unter der Hinterlegungsnummer DSM 4390, ist.

8. Rekombinantes DNA-Molekül nach Anspruch 1, das pGW880, hinterlegt unter der Hinterlegungsnummer DSM 4392, ist.

9. Rekombinantes DNA-Molekül nach Anspruch 1, das pGW860, hinterlegt unter der Hinterlegungsnummer DSM 4391, ist.

10. Rekombinantes DNA-Molekül nach Anspruch 1, das ein Fragment umfasst, das sich zwischen zwei Restriktionsspaltstellen von Plasmid pGW820, hinterlegt unter der Hinterlegungsnummer DSM 4388, pGW830, hinterlegt unter der Hinterlegungsnummer DSM 4389, pGW850, hinterlegt unter der Hinterlegungsnummer DSM 4390, pGW880, hinterlegt unter der Hinterlegungsnummer DSM 4392, pGW860, hinterlegt unter der Hinterlegungsnummer DSM 4391, erstreckt und eine Pektinlyasepromoter, -signal, -struktur oder terminatorfunktion beibehält, oder eine Kombination solcher Fragmente umfasst.

11. Rekombinantes DNA-Molekül nach Anspruch 1, umfassend die Promotersequenz von pelA.

12. Rekombinantes DNA-Molekül nach Anspruch 1, umfassend die Promotersequenz von pelA, pelB, pelC, pelE oder pelF.

13. Rekombinantes DNA-Molekül nach Anspruch 1, umfassend die Signalsequenz von pelA, pelB, pelC, pelE oder pelF.

14. Rekombinantes DNA-Molekül nach Anspruch 1, umfassend das PLI Strukturgen von pelA, pelB, pelC, pelE oder pelF.

15. Rekombinantes DNA-Molekül nach Anspruch 1, umfassend das PLI Strukturgen von pelA, pelB, PelC, pelE oder pelF ohne Introns.

16. Rekombinantes DNA-Molekül nach Anspruch 1, umfassend den Terminator von pelA, pelB, pelC, pelE oder pelF.

17. Rekombinantes DNA-Molekül nach Anspruch 1, das ein rekombinanter Hybridvektor ist, der ein Strukturgen umfasst, das zu Aspergillus niger heterolog ist.

18. Rekombinantes DNA-Molekül nach Anspruch 1, das ein Hybridvektor ist, der das heterologe Strukturgen, das das Hybridinterferon BDBB codiert, umfasst.

19. Rekombinantes DNA-Molekül nach Anspruch 1, das der rekombinante Hybridvektor pUC19/pelA-IFN AM119, gezeigt in Figur 14, ist.

20. Rekombinantes DNA-Molekül nach Anspruch 1, das der rekombinante Hybridvektor M13(+)KS/pelA△ss-IFN AM119 ist, der aus dem induzierbaren pelA-Promoter, der codierenden Sequenz des Hybridinterferons BDBB und dem pelA-Transkriptionsterminator auf dem Bluescript M13(+)KS-Vektor besteht.

21. Rekombinantes DNA-Molekül nach Anspruch 1, das der Hybridvektor M13(+)KS/pelA-IFN AM119 ist, der durch Spalten von pUC19/pelA-IFN AM119, wie in Fig. 14 gezeigt, mit BamHI und HindIII und Ligieren des isolierten Fragments an einen mit BamHI und HindIII gespaltenen Bluescript M13(+)KS-Vektor erhalten wurde.

22. Verfahren zur Herstellung eines rekombinanten DNA-Moleküls nach Anspruch 1, umfassend das Züchten eines Wirts, der transformiert wurde mit einem DNA-Molekül, das eine DNA-Sequenz enthält, die das Pectinlyase PLA-, PLB-, PLC-, PLE- oder PLF-Expressionssystem codiert, oder einem Derivat davon, und das Isolieren des gewünschten rekombinanten DNA-Moleküls oder des Derivats davon, oder dessen Herstellung durch in vitro-Synthese.

23. Transformierter Wirt, der ein rekombinantes DNA-Molekül nach Anspruch 1 enthält.

24. Transformierter Wirt nach Anspruch 23, der Escherichia coli HB101/pGW820, hinterlegt unter der Hinterlegungsnummer DSM 4388, ist.

25. Transformierter Wirt nach Anspruch 24, der Escherichia coli HB101/pGW830, hinterlegt unter der Hinterlegungsnummer DSM 4389, ist.

26. Transformierter Wirt nach Anspruch 24, der Escherichia coli HB101/pGW850, hinterlegt unter der Hinterlegungsnummer DSM 4390, ist.

27. Transformierter Wirt nach Anspruch 24, der Escherichia coli HB101/pGW860, hinterlegt unter der Hinterlegungsnummer DSM 4391, ist.

28. Transformierter Wirt nach Anspruch 24, der Escherichia coli HB101/pGW880, hinterlegt unter der Hinterlegunganummer DSM 4392, ist.

29. Transformierter Wirt nach Anspruch 24, der Aapergillus niger An8, hinterlegt unter der Hinterlegungsnummer DSM 3917, transformiert mit einem rekombinanten DNA-Molekül nach Anspruch 1 und dem Selektionsmarkerplasmid pCG59D7, hinterlegt unter der Hinterlegungsnummer DSM 3968, ist.

30. Transformierter Wirt nach Anspruch 24, der Aspergillus niger AnB, hinterlegt unter der Hinterlegungsnummer DSM 3917, transformiert mit pUC19/pelA-IFN AM119, gezeigt in Figur 14, und dem Selektionsmarkerplasmid pCG59D7, hinterlegt unter der Hinterlegungsnummer DSM 3968, ist.

31. Transformierter Wirt nach Anspruch 24, der Aspergillus niger An8, hinterlegt unter der Hinterlegungsnummer DSM 3917, transformiert mit M13(+)KS/pelAΔss-IFN AM119 nach Anspruch 21 und dem Selektionsmarkerplasmid pCG59D7, hinterlegt unter der Hinterlegungsnummer DSM 3968, ist.

32. Transformierter Wirt nach Anspruch 24, der Aspergillus niger An8, hinterlegt unter der Hinterlegungsnummer DSM 3917, transformiert mit M13(+)KS/pelA-IFN AM119 nach Anspruch 22 und dem Selektionsmarkerplasmid pCG59D7, hinterlegt unter der Hinterlegungsnummer DSM 3968, ist.

33. Verfahren zur Herstellung eines transformierten Wirts nach Anspruch 24, umfassend die Behandlung eines solchen Wirts unter transformierenden Bedingungen mit einem rekombinanten DNA-Molekül nach Anspruch 1 gegebenenfalls zusammen mit einem Selektionsmarkergen und Selektion der Transformanten.

34. Verfahren nach Anspruch 33, wobei Aspergillus niger An8 mit einem rekombinanten DNA-Molekül nach Anspruch 1 und dem Selektionsmarkerplasmid pCG59D7, hinterlegt unter der Hinterlegungsnummer DSM 3968, cotransformiert wird.

35. Verfahren zur Herstellung eines Polypeptids, **dadurch gekennzeichnet, dass** ein Hybridvektor nach Anspruch 1 in einem geeigneten Wirt exprimiert wird.

36. Verfahren zur Herstellung eines Polypeptids, **dadurch gekennzeichnet, dass** ein Hybridvektor nach Anspruch 18 in einem geeigneten Wirt exprimiert wird.

37. Verfahren nach Anspruch 36 zur Überproduktion der Pectinlyasen PLA, PLB, PLC, PLE oder PLF in Aspergillusarten, umfassend die Züchtung eines mit einem Expressionshybridvektor zur Expression der Pektinlyasen transformierten Aspergilluswirts.

38. Verfahren nach Anspruch 37 zur Produktion von Hybridinterferon BDBB in einer Aspergillusart umfassend die Züchtung eines mit einem Expressionsvektor des Hybridinterferons transformierten Aspergilluswirts.

39. Pectinlyasen PLA, PLB, PLC, PLE oder PLF in reiner Form, erhältlich durch Transformation eines Wirts, der zur Expression einer Pektinlyase nicht fähig ist, mit einem rekombinanten DNA-Molekül nach Anspruch 1 und Isolieren der Pectinlyasen.

## Patentansprüche (Patentansprüche für folgende(n) Vertragsstaat(en): ES)

1. Verfahren zur Herstellung eines rekombinanten DNA-Moleküls, umfassend eine DNA-Sequenz, die Pectinlyase pelA mit der in Fig. 10 dargestellten Aminosäuresequenz, Pectinlyase pelB mit der in Fig. 11 dargestellten Aminosäuresequenz, Pectinlyase pelc, erhältlich durch Expression von pelc, enthalten in pGW850, das in Fig. 3 gezeigt ist und unter der Hinterlegungsnummer DSM 4390 hinterlegt ist, pelE, erhältlich durch Expression von pelE, enthalten in pGW880, das in Fig. 5 gezeigt ist und unter der Hinterlegungsnummer DSM 4392 hinterlegt ist, oder pelF, erhältlich durch Expression von pelF, enthalten in pGW860, das in Fig. 6 gezeigt ist und unter der Hinterlegungsnummer DSM 4391 hinterlegt ist, codiert, oder ein Derivat davon, wobei ein Derivat ein größeres Derivat einschließlich flankierender Sequenzen oder DNA-Sequenzen, die gemäß dem genetischen code degeneriert sind, bezeichnet.

2. Verfahren nach Anspruch 1 zur Herstellung eines rekombinanten DNA-Moleküls nach Anspruch 1, umfassend den Promoter, der in der DNA-Region vor dem Strukturgen lokalisiert ist und bis zu 2000 Nucleotide umfasst, die Signalsequenz, die in der DNA-Region zwischen dem Ende des Promoters und dem Beginn der Sequenz, die das reife Protein codiert, lokalisiert ist, das strukturgen oder den Terminator, der mit dem Stoppcodon beginnt, eines der Pectinlyasegene, oder eine beliebige Kombination dieser Fragmente.

3. Verfahren nach Anspruch 1 zur Herstellung eines rekoabinanten DNA-Moleküls, umfassend eine DNA-Sequenz der in Figur 10 gezeigten Formel I oder ein Derivat davon wie in Anspruch 1 definiert.

4. Verfahren nach Anspruch 1 zur Herstellung eines rekombinanten DNA-Moleküls, umfassend eine DNA-Sequenz der in Figur 11 gezeigten Formel II oder ein Derivat davon wie in Anspruch 1 definiert.

5. Verfahren nach Anspruch 1 zur Herstellung eines rekombinanten DNA-Moleküls, das pGW820, hinterlegt unter der Hinterlegungsnummer DSM 4388, ist.

6. Verfahren nach Anspruch 1 zur Herstellung eines rekombinanten DNA-Moleküls, das pGW4389, hinterlegt unter der Hinterlegungsnummer DSM 4389, ist.

7. Verfahren nach Anspruch 1 zur Herstellung eines rekombinanten DNA-Moleküls, das pGW850, hinterlegt unter der Hinterlegungsnummer DSM 4390, ist.

8. Verfahren nach Anspruch 1 zur Herstellung eines rekombinanten DNA-Moleküls, das pGE880, hinterlegt unter der Hinterlegungsnummer DSM 4392, ist.

9. Verfahren nach Anspruch 1 zur Herstellung eines rekombinanten DNA-Moleküls, das pGW860, hinterlegt unter der Hinterlegungsnummer DSM 4391, ist.

10. Verfahren zur Herstellung eines rekombinanten DNA-Moleküls, das ein Fragment umfasst, das sich zwischen zwei Restriktionsspaltstellen von Plasmid pGW820, hinterlegt unter der Hinterlegungsnummer DSM 4388, pGW830, hinterlegt unter der Hinterlegungsnummer DSM 4389, pGW850, hinterlegt unter der Hinterlegungsnummer DSM 4390, pGW880, hinterlegt unter der Hinterlegungsnummer DSM 4392, pGW860, hinterlegt unter der Hinterlegungsnummer DSM 4391, erstreckt und eine Pektinlyasepromoter, -signal, -struktur oder - terminatorfunktion beibehält, oder eine Kombination solcher Fragmente umfasst.

11. Verfahren zur Herstellung eines rekombinanten DNA-Moleküls, umfassend die Promotersequenz von pelA.

12. Verfahren zur Herstellung eines rekombinanten DNA-Moleküls, umfassend die Promotersequenz von pelA, pelB, pelC, pelE oder pelF.

13. Verfahren zur Herstellung eines rekombinanten DNA-Moleküls, umfassend die Signalsequenz von pelA, pelB, pelC, pelE oder pelF.

14. Verfahren zur Herstellung eines rekombinanten DNA-Moleküls, umfassend das PLI strukturgen von pelA, pelB, pelC, pelE oder pelF.

15. Verfahren zur Herstellung eines rekombinanten DNA-Moleküls, umfassend das PLI Strukturgen von pelA, pelB, pelC, pelE oder pelF ohne Introns.

16. Verfahren zur Herstellung eines rekombinanten DNA-Moleküls, umfassend den Terminator von pelA, pelB, pelC, pelE oder pelF.

17. Verfahren zur Herstellung eines rekombinanten DNA-Moleküls, das ein rekombinanter Hybridvektor ist, der ein Strukturgen umfasst, das zu Aspergillus niger heterolog ist.

18. Verfahren zur Herstellung eines rekombinanten DNA-Moleküls, das ein Hybridvektor ist, der das heterologe Strukturgen, das das Hybridinterferon BDBB codiert, umfasst.

19. Verfahren zur Herstellung eines rekombinanten DNA-Moleküls, das der rekombinante Hybridvektor pUC19/palA-IFN AK119, gezeigt in Figur 14, ist.

20. Verfahren zur Herstellung eines rekombinanten DNA-Moleküls, das der rekombinante Hybridvektor M13(+)KS/pelAΔss-IFN AM119 ist, der aus dem induzierbaren pelA-Promoter, der codierenden Sequenz des Hybridinterferons BDBB und dem pelA-Transkriptionsterminator auf dem Bluescript M13(+)KS-Vektor besteht.

21. Verfahren zur Herstellung eines rekombinanten DNA-Moleküls, das der Hybridvektor M13(+)KS/pelA-IFN AM119 ist, der durch spalten von pUC19/pelA-IFN AM119, wie in Fig. 14 gezeigt, mit BamHI und HindIII und Ligieren der isolierten Fragmente an einen mit BamHI und HindIII gespaltenen Bluescript M13(+)KS-Vektor erhalten wurde.

22. Verfahren zur Herstellung eines rekombinanten DNA-Moleküls nach Anspruch 1, umfassend das Züchten eines Wirts, der transformiert wurde mit einem DNA-Molekül, das eine DNA-Sequenz enthält, die das Pectinlyase PLA-, PLB-, PLC-, PLE- oder PLF-Expressionssystem codiert, oder einem Derivat davon, und das Isolieren des gewünschten rekombinanten DNA-Moleküls oder des Derivats davon, oder dessen Herstellung durch in vitro-Synthese.

23. Verfahren nach Anspruch 22 zur Herstellung eines transformierten Wirts, der ein rekombinantes DNA-Molekül nach Anspruch 1 enthält.

24. Verfahren nach Anspruch 22 zur Herstellung eines transformierten Wirts, der Escherichia coli HB101/pGW820, hinterlegt unter der Hinterlegungsnummer DSM 4388, ist.

25. verfahren nach Anspruch 22 zur Herstellung eines transformierten Wirts, der Escherichia coli HB101/pGW830, hinterlegt unter der Hinterlegungsnummer DSM 4389, ist.

26. Verfahren nach Anspruch 22 zur Herstellung eines transformierten Wirts, der Escherichia coli HB101/pGW850, hinterlegt unter der Hinterlegungsnummer DSM 4390, ist.

27. Verfahren nach Anspruch 22 zur Herstellung eines transformierten Wirts, der Escherichia coli HB101/pGW860, hinterlegt unter der Hinterlegungsnummer DSM 4391, ist.

28. Verfahren nach Anspruch 22 zur Herstellung eines transformierten Wirts, der Escherichia coli HB101/pGW880, hinterlegt unter der Hinterlegungsnummer DSM 4392, ist.

29. Verfahren nach Anspruch 22 zur Herstellung eines transformierten Wirts, der Aspergillus niger An8, hinterlegt unter der Hinterlegungsnummer DSM 3917, transformiert mit einem rekombinanten DNA-Molekül nach Anspruch 1 und dem Selektionsmarkerplasmid pCG59D7, hinterlegt unter der Hinterlegungsnummer DSM 3968, ist.

30. Verfahren nach Anspruch 22 zur Herstellung eines transformierten Wirts, der Aspergillus niger An8, hinterlegt unter der Hinterlegungsnummer DSM 3917, transformiert mit pUC19/palA-IFN AM119, gezeigt in Figur 14, und dem Selektionsmarkerplasmid pCG59D7, hinterlegt unter der Hinterlegungsnummer DSM 3968, ist.

31. Verfahren nach Anspruch 22 zur Herstellung eines transformierten Wirts, der Aspergillus niger An8, hinterlegt unter der Hinterlegungsnummer DSM 3917, transformiert mit M13(+)KS/pelAΔss-IFN AM119 nach Anspruch 21 und dem Selektionsmarkerplasmid pCG59D7, hinterlegt unter der Hinterlegungsnummer DSM 3968, ist.

32. Verfahren nach Anspruch 22 zur Herstellung eines transformierten Wirts, der Aspergillus niger An8, hinterlegt unter der Hinterlegungsnummer DSM 3917, transformiert mit M13(+)KS/pelA-IFN AM119 nach Anspruch 22, und dem Selektionsmarkerplasmid pCG59D7, hinterlegt unter der Hinterlegungsnummer DSM 3968, ist.

33. Verfahren zur Herstellung eines transformierten Wirts nach Anspruch 22, umfassend die Behandlung eines solchen Wirts unter transformierenden Bedingungen mit einem rekombinanten DNA-Molekül nach Anspruch 1 gegebenenfalls zusammen mit einem Selektionsmarkergen und Selektion der Transformanten.

34. Verfahren nach Anspruch 33, wobei Aspergillus niger An8 mit einem rekombinanten DNA-Molekül nach Anspruch 1 und dem Selektionsmarkerplasmid pCG59D7, hinterlegt unter der Hinterlegungsnummer DSM 3968, cotransformiert wird.

35. Verfahren zur Herstellung eines Polypeptids, **dadurch gekennzeichnet, dass** ein Hybridvektor nach Anspruch 1 in einem geeigneten Wirt exprimiert wird.

36. Verfahren zur Herstellung eines Polypeptids, **dadurch gekennzeichnet, dass** ein Hybridvektor nach Anspruch 18 in einem geeigneten Wirt exprimiert wird.

37. Verfahren nach Anspruch 36 zur Überproduktion der Pectinlyasen PLA, PLB, PLC, PLE oder PLF in Aspergillusarten, umfassend die Züchtung eines mit einem Expressionshybridvektor zur Expression der Pektinlyasen transformierten Aspergilluswirts.

38. Verfahren nach Anspruch 37 zur Produktion von Hybridinterferon BOBB in einer Aspergillusart umfassend die Züchtung eines mit einem Expressionsvektor des Hybridinterferons transformierten Aspergilluswirts.

39. Verfahren zur Produktion der Pectinlyasen PLA, PLB, PLC, PLE oder PLF in reiner Form, erhältlich durch Transformation eines Wirts, der zur Expression einer Pektinlyase nicht fähig ist, mit einem rekombinanten DNA-Molekül nach Anspruch 1 und Isolieren der Pectinlyasen.

## Revendications (Revendications pour l'(les) Etat(s) contractant(s) suivant(s): AT, BE, CH, DE, FR, GB, GR, IT, LI, LU, NL, SE)

1. Une molécule d'ADN recombinant comprenant une séquence d'ADN codant pour la lyase de pectine pelA ayant la séquence d'acides aminés représentée sur la figure 10 ; la lyase de pectine pelB ayant la séquence d'acides aminés représentée sur la figure 11 ; la lyase de pectine pelC pouvant être obtenue par l'expression de pelC contenue dans pGW850 représenté sur la figure 3, déposé sous le numéro d'accession DSM 4390 ; la pelE pouvant être obtenue par l'expression de pelE contenue dans pGW880 représenté sur la figure 5, déposé sous le numéro d'accession DSM 4392 ou pelF pouvant être obtenue par l'expression de pelF contenue dans pGW860 représenté sur la figure 6, déposé sous le numéro d'accession DSM 4391 ou un dérivé de celle-ci, dans laquelle un dérivé désigne un plus grand dérivé comprenant des séquences adjacentes ou des séquences d'ADN qui sont dégénérées conformément au code génétique.

2. Une molécule d'ADN recombinant selon la revendication 1 comprenant le promoteur qui est situé dans la région de l'ADN avant le gène structural et comprend jusqu'à 2000 nucléotides ; la séquence signal qui est située dans la région de l'ADN entre la fin du promoteur et le début de la séquence codant pour la protéine mature ; le gène structural ; ou le terminateur, qui commence avec le codon d'arrêt, de l'un quelconque des gènes de lyase de pectine, ou toute combinaison de ces fragments.

3. Une molécule d'ADN recombinant selon la revendication 1, comprenant une séquence d'ADN de la formule I représentée sur la figure 10 ou un dérivé de celle-ci comme défini dans la revendication 1.

4. Une molécule d'ADN recombinant selon la revendication 1, comprenant une séquence d'ADN de la formule II représentée sur la figure 11 ou un dérivé de celle-ci comme défini dans la revendication 1.

5. Une molécule d'ADN recombinant selon la revendication 1, qui est pGW820 déposé sous le numéro d'accession DSM 4388.

6. Une molécule d'ADN recombinant selon la revendication 1, qui est pGW830 déposé sous le numéro d'accession DSM 4389.

7. Une molécule d'ADN recombinant selon la revendication 1, qui est pGW850 déposé sous le numéro d'accession DSM 4390.

8. Une molécule d'ADN recombinant selon la revendication 1, qui est pGW880 déposé sous le numéro d'accession DSM 4392.

9. Une molécule d'ADN recombinant selon la revendication 1, qui est pGW860 déposé sous le numéro d'accession DSM 4391.

10. Une molécule d'ADN recombinant selon la revendication 1, qui comprend un fragment s'étendant entre deux sites de restriction du plasmide pGW820 déposé sous le numéro d'accession DSM 4388, pGW830 déposé sous le numéro d'accession DSM 4389, pGW850 déposé sous le numéro d'accession DSM 4390, pGW880 déposé sous le numéro d'accession DSM 4392, ou pGW860 déposé sous le numéro d'accession DSM 4391 et gardant un promoteur de lyase de pectine, des fonctions de signal, structurale ou terminatrice ou une combinaison de ces fragments.

11. Une molécule d'ADN recombinant selon la revendication 1, comprenant la séquence promotrice de pelA.

12. Une molécule d'ADN recombinant selon la revendication 1, comprenant la séquence promotrice de pelA, pelB, pelC, pelE ou pelF.

13. Une molécule d'ADN recombinant selon la revendication 1, comprenant la séquence signal de pelA, pelB, pelC, pelE ou pelF.

14. Une molécule d'ADN recombinant selon la revendication 1, comprenant le gène structural PLI de pelA, pelB, pelC, pelE ou pelF.

15. Une molécule d'ADN recombinant selon la revendication 1, comprenant le gène structural PLI de pelA, pelB, pelC, pelE ou pelF, sans les introns

16. Une molécule d'ADN recombinant selon la revendication 1, comprenant le terminateur de pelA, pelB, pelC, pelE ou pelF.

17. Une molécule d'ADN recombinant selon la revendication 1, qui est un vecteur hybride recombinant comprenant un gène structural hétérologue à l'Aspergillus niger.

18. Une molécule d'ADN recombinant selon la revendication 1, qui est un vecteur hybride comprenant le gène structural hétérologue codant pour l'interféron hybride BDBB.

19. Une molécule d'ADN recombinant selon la revendication 1, qui est le vecteur hybride recombinant pUC19/pelA-IFN AM119 représenté sur la figure 14.

20. Une molécule d'ADN recombinant selon la revendication 1, qui est le vecteur hybride recombinant M13(+)KS/pelAΔss-IFN AM119, composé du promoteur inductible de pelA, de la séquence codante de l'interféron hybride BDBB et du terminateur de transcription de pelA sur le vecteur Bluescript M13(+)KS.

21. Une molécule d'ADN recombinant selon la revendication 1, qui est le vecteur hybride M13(+)KS/pelA-IFN AM119 obtenu en digérant le pUC19/pelA-IFN AM119 comme représenté sur la figure 14 avec BamHI et HindIII et en soudant le fragment isolé à un vecteur Bluescript M13(+)KS, coupé avec BamHI et HindIII.

22. Un procédé pour la préparation d'une molécule d'ADN recombinant selon la revendication 1, comprenant la culture d'un hôte transformé avec une molécule d'ADN contenant une séquence d'ADN codant pour le système d'expression de la lyase de pectine PLA, PLB, PLC, PLE ou PLF ou un dérivé de celui-ci, et en isolant la molécule d'ADN recombinant désirée ou le dérivé de celle-ci, ou en la préparant par synthèse in vitro.

23. Un hôte transformé contenant une molécule d'ADN recombinant selon la revendication 1.

24. Un hôte transformé selon la revendication 23, qui est l'Escherichia coli HB 101/pGW820 déposé sous le numéro d'accession DSM 4388.

25. Un hôte transformé selon la revendication 23, qui est l'Escherichia coli HB 101/pGW830 déposé sous le numéro d'accession DSM 4389.

26. Un hôte transformé selon la revendication 23, qui est l'Escherichia coli HB 101/pGW850 déposé sous le numéro d'accession DSM 4390.

27. Un hôte transformé selon la revendication 23, qui est l'Escherichia coli HB 101/pGW860 déposé sous le numéro d'accession DSM 4391.

28. Un hôte transformé selon la revendication 23, qui est l'Escherichia coli HB 101/pGW880 déposé sous le numéro d'accession DSM 4392.

29. Un hôte transformé selon la revendication 23 qui est l'Aspergillus niger An8 déposé sous le numéro d'accession DSM 3917 transformé avec une molécule d'ADN recombinant selon la revendication 1 et le plasmide marqueur de sélection pCG59D7 déposé sous le numéro d'accession DSM 3968.

30. Un hôte transformé selon la revendication 23 qui est l'Aspergillus niger An8 déposé sous le numéro d'accession DSM 3917 transformé avec le pUC19/pelA-IFN AM119 représenté sur la figure 14 et le plasmide marqueur de sélection pCG59D7 déposé sous le numéro d'accession DSM 3968.

31. Un hôte transformé selon la revendication 23 qui est l'Aspergillus niger An8 déposé sous le numéro d'accession DSM 3917 transformé avec le M13(+)KS/pelAΔss-IFN AM119 selon la revendication 21 et le plasmide marqueur de sélection pCG59D7 déposé sous le numéro d'accession DSM 3968.

32. Un hôte transformé selon la revendication 23 qui est l'Aspergillus niger An8 déposé sous le numéro d'accession DSM 3917 transformé avec le M13(+)KS/pelA-IFN AM119 selon la revendication 22 et le plasmide marqueur de sélection pCG59D7 déposé sous le numéro d'accession DSM 3968.

33. Un procédé pour la préparation d'un hôte transformé selon la revendication 24, comprenant le traitement de cet hôte sous des conditions transformantes avec une molécule d'ADN recombinant selon la revendication 1, de façon optionnelle ensemble avec un gène marqueur de sélection et en sélectionnant les transformants.

34. Un procédé selon la revendication 33 dans lequel l'Aspergillus niger An8 est cotransformé avec une molécule d'ADN recombinant selon la revendication 1 et le plasmide marqueur de sélection pCG59D7 déposé sous le numéro d'accession DSM 3968.

35. Un procédé pour la préparation d'un polypeptide, **caractérisé en ce qu'**un vecteur hybride selon la revendication 1 est exprimé dans un hôte adapté.

36. Un procédé pour la préparation d'un polypeptide, **caractérisé en ce qu'**un vecteur hybride selon la revendication 18 est exprimé dans un hôte adapté.

37. Un procédé selon la revendication 36 pour la surproduction de lyases de pectine PLA, PLB, PLC, PLE ou PLF dans l'espèce Aspergillus comprenant la culture d'un hôte Aspergillus transformé avec un vecteur hybride d'expression pour l'expression desdites lyases de pectine.

38. Un procédé selon la revendication 37 pour la production de l'interféron hybride BDBB dans l'espèce Aspergillus comprenant la culture d'un hôte Aspergillus transformé avec un vecteur hybride d'expression pour l'expression dudit interféron hybride.

39. Des lyases de pectine PLA, PLB, PLC, PLE ou PLF en forme pure, pouvant être obtenues en transformant un hôte qui n'est capable d'exprimer aucune lyase de pectine avec une molécule d'ADN recombinant selon la revendication 1 et en isolant lesdites lyases de pectine.

## Revendications (Revendications pour l'(les) Etat(s) contractant(s) suivant(s): ES)

1. Un procédé pour la préparation d'une molécule d'ADN recombinant comprenant une séquence d'ADN codant pour la lyase de pectine pelA ayant la séquence d'acides aminés représentée sur la figure 10 ; la lyase de pectine pelB ayant la séquence d'acides aminés représentée sur la figure 11 ; la lyase de pectine pelC pouvant être obtenue par l'expression de pelC contenue dans pGW850 représenté sur la figure 3, déposé sous le numéro d'accession DSM 4390 ; la pelE pouvant être obtenue par l'expression de pelE contenue dans pGW880 représenté sur la figure 5, déposé sous le numéro d'accession DSM 4392 ou pelF pouvant être obtenue par l'expression de pelF contenue dans pGW860 représenté sur la figure 6, déposé sous le numéro d'accession DSM 4391 ou un dérivé de celle-ci, dans laquelle un dérivé désigne un plus grand dérivé comprenant des séquences adjacentes ou des séquences d'ADN qui sont dégénérées conformément au code génétique.

2. Un procédé selon la revendication 1 pour la préparation dune molécule d'ADN recombinant comprenant le promoteur qui est situé dans la région de l'ADN avant le gène structural et comprend jusqu'à 2000 nucléotides ; la séquence signal qui est située dans la région de l'ADN entre la fin du promoteur et le début de la séquence codant pour la protéine mature ; le gène structural ; ou le terminateur, qui commence avec le codon d'arrêt, de l'un quelconque des gènes de lyase de pectine, ou toute combinaison de ces fragments.

3. Un procédé selon la revendication 1 pour la préparation d'une molécule d'ADN recombinant, comprenant une séquence d'ADN de la formule I représentée sur la figure 10 ou un dérivé de celle-ci comme défini dans la revendication 1.

4. Un procédé selon la revendication 1 pour la préparation d'une molécule d'ADN recombinant comprenant une séquence d'ADN de la formule II représentée sur la figure 11 ou un dérivé de celle-ci comme défini dans la revendication 1.

5. Un procédé selon la revendication 1 pour la préparation d'une molécule d'ADN recombinant qui est pGW820 déposé sous le numéro d'accession DSM 4388.

6. Un procédé selon la revendication 1 pour la préparation d'une molécule d'ADN recombinant qui est pGW4389 déposé sous le numéro d'accession DSM 4389.

7. Un procédé selon la revendication 1 pour la préparation d'une molécule d'ADN recombinant qui est pGW850 déposé sous le numéro d'accession DSM 4390.

8. Un procédé selon la revendication 1 pour la préparation d'une molécule d'ADN recombinant qui est pGW880 déposé sous le numéro d'accession DSM 4392.

9. Un procédé selon la revendication 1 pour la préparation d'une molécule d'ADN recombinant qui est pGW860 déposé sous le numéro d'accession DSM 4391.

10. Un procédé pour la préparation dune molécule d'ADN qui comprend un fragment s'étendant entre deux sites de restriction du plasmide pGW820 déposé sous le numéro d'accession DSM 4388, pGW830 déposé sous le numéro d'accession DSM 4389, pGW850 déposé sous le numéro d'accession DSM 4390, pGW880 déposé sous le numéro d'accession DSM 4392, ou pGW860 déposé sous le numéro d'accession DSM 4391 et gardant un promoteur de lyase de pectine, des fonctions de signal, structurale ou terminatrice ou une combinaison de ces fragments.

11. Un procédé pour la préparation d'une molécule d'ADN recombinant comprenant la séquence promotrice de pelA.

12. Un procédé pour la préparation d'une molécule d'ADN recombinant comprenant la séquence promotrice de pelA, pelB, pelC, pelE ou pelF.

13. Un procédé pour la préparation d'une molécule d'ADN recombinant comprenant la séquence signal de pelA, pelB, pelC, pelE ou pelF.

14. Un procédé pour la préparation d'une molécule d'ADN recombinant comprenant le gène structural PLI de pelA, pelB, pelC, pelE ou pelF.

15. Un procédé pour la préparation d'une molécule d'ADN recombinant comprenant le gène structural PLI de pelA, pelB, pelC, pelE ou pelF, sans les introns.

16. Un procédé pour la préparation dune molécule d'ADN recombinant comprenant le terminateur de pelA, pelB, pelC, pelE ou pelF.

17. Un procédé pour la préparation d'une molécule d'ADN recombinant qui est un vecteur hybride recombinant comprenant un gène structural hétérologue à l'Aspergillus niger.

18. Un procédé pour la préparation d'une molécule d'ADN recombinant qui est un vecteur hybride comprenant le gène structural hétérologue codant pour l'interféron hybride BDBB.

19. Un procédé pour la préparation d'une molécule d'ADN recombinant qui est le vecteur hybride recombinant pUC19/pelA-IFN AM119 représenté sur la figure 14.

20. Un procédé pour la préparation d'une molécule d'ADN recombinant qui est le vecteur hybride recombinant M13(+)KS/pelAΔss-IFN AM119, composé du promoteur inductible de pelA, de la séquence codante de l'interféron hybride BDBB et du terminateur de transcription de pelA sur le vecteur Bluescript M13(+)KS.

21. Un procédé pour la préparation d'une molécule d'ADN recombinant qui est le vecteur hybride M13(+)KS/pelA-IFN AM119 obtenu en digérant le pUC19/pelA-IFN AM119 comme représenté sur la figure 14 avec BamHI et HindIII et en soudant le fragment isolé à un vecteur Bluescript M13(+)ES, coupé avec BamHI et HindIII.

22. Un procédé pour la préparation dune molécule d'ADN recombinant selon la revendication 1, comprenant la culture d'un hôte transformé avec une molécule d'ADN contenant une séquence d'ADN codant pour le système d'expression de la lyase de pectine PLA, PLB, PLC, PLE ou PLF ou un dérivé de celui-ci, et en isolant la molécule d'ADN recombinant désirée ou le dérivé de celle-ci, ou en la préparant par synthèse in vitro.

23. Un procédé selon la revendication 22 pour la préparation d'un hôte transformé contenant une molécule d'ADN recombinant selon la revendication 1.

24. Un procédé selon la revendication 22 pour la préparation d'un hôte transformé qui est l'Escherichia coli HB 101/pGW820 déposé sous le numéro d'accession DSM 4388.

25. Un procédé selon la revendication 22 pour la préparation d'un hôte transformé qui est l'Escherichia coli HB 101/pGW830 déposé sous le numéro d'accession DSM 4389.

26. Un procédé selon la revendication 22 pour la préparation d'un hôte transformé qui est l'Escherichia coli HB 101/pGW850 déposé sous le numéro d'accession DSM 4390.

27. Un procédé selon la revendication 22 pour la préparation d'un hôte transformé qui est l'Escherichia coli HB 101/pGW860 déposé sous le numéro d'accession DSM 4391.

28. Un procédé selon la revendication 22 pour la préparation d'un hôte transformé qui est l'Escherichia coli HB 101/pGW880 déposé sous le numéro d'accession DSM 4392.

29. Un procédé selon la revendication 22 pour la préparation d'un hôte transformé qui est l'Aspergillus niger An8 déposé sous le numéro d'accession DSM 3917 transformé avec une molécule d'ADN recombinant selon la revendication 1 et le plasmide marqueur de sélection pCG59D7 déposé sous le numéro d'accession DSM 3968.

30. Un procédé selon la revendication 22 pour la préparation d'un hôte transformé qui est l'Aspergillus niger An8 déposé sous le numéro d'accession DSM 3917 transformé avec le pUC19/pelA-IFN AM119 représenté sur la figure 14 et le plasmide marqueur de sélection pCG59D7 déposé sous le numéro d'accession DSM 3968.

31. Un procédé selon la revendication 22 pour la préparation d'un hôte transformé qui est l'Aspergillus niger An8 déposé sous le numéro d'accession DSM 3917 transformé avec le M13(+)KS/pelAΔss-IFN AM119 selon la revendication 21 et le plasmide marqueur de sélection pCG59D7 déposé sous le numéro d'accession DSM 3968.

32. Un procédé selon la revendication 22 pour la préparation d'un hôte transformé qui est l'Aspergillus niger An8 déposé sous le numéro d'accession DSM 3917 transformé avec le M13(+)KS/pelA-IFN AM119 selon la revendication 22 et le plasmide marqueur de sélection pCG59D7 déposé sous le numéro d'accession DSM 3968.

33. Un procédé pour la préparation d'un hôte transformé selon la revendication 22, comprenant le traitement de cet hôte sous des conditions transformantes avec une molécule d'ADN recombinant selon la revendication 1, de façon optionnelle ensemble avec un gène marqueur de sélection et en sélectionnant les transformants.

34. Un procédé selon la revendication 33 dans lequel l'Aspergillus niger An8 est cotransformé avec une molécule d'ADN recombinant selon la revendication 1 et le plasmide marqueur de sélection pCG59D7 déposé sous le numéro d'accession DSM 3968.

35. Un procédé pour la préparation d'un polypeptide, **caractérisé en ce qu'**un vecteur hybride selon la revendication 1 est exprimé dans un hôte adapté.

36. Un procédé pour la préparation d'un polypeptide, **caractérisé en ce qu'**un vecteur hybride selon la revendication 18 est exprimé dans un hôte adapté.

37. Un procédé selon la revendication 36 pour la surproduction de lyases de pectine PLA, PLB, PLC, PLE ou PLF dans l'espèce Aspergillus comprenant la culture d'un hôte Aspergillus transformé avec un vecteur hybride d'expression pour l'expression desdites lyases de pectine.

38. Un procédé selon la revendication 37 pour la production de l'interféron hybride BDBB dans l'espèce Aspergillus comprenant la culture d'un hôte Aspergillus transformé avec une expression dudit interféron hybride.

39. Un procédé pour la production de lyases de pectine PLA, PLB, PLC, PLE ou PLF en forme pure, pouvant être obtenues en transformant un hôte qui n'est capable d'exprimer aucune lyase de pectine avec une molécule d'ADN recombinant selon la revendication 1 et en isolant lesdites lyases de pectine.
